**(19)** Europäisches Patentamt

European Patent Office

Office européen des brevets

**(11)** **EP 1 258 529 A2**

**(12)** **EUROPÄISCHE PATENTANMELDUNG**

**(43)** Veröffentlichungstag:
**20.11.2002 Patentblatt 2002/47**

**(51)** Int Cl.$^7$: **C12N 15/12**, C07K 14/47,
C12N 5/10, G01N 33/68,
A61K 38/17, A61K 48/00

**(21)** Anmeldenummer: **02013267.6**

**(22)** Anmeldetag: **31.03.1998**

**(84)** Benannte Vertragsstaaten:
**AT BE CH DE DK ES FI FR GB GR IE IT LI LU MC
NL PT SE**

**(30)** Priorität: **01.04.1997 DE 19713393**

**(62)** Dokumentnummer(n) der früheren Anmeldung(en)
nach Art. 76 EPÜ:
**98919192.9 / 0 973 901**

**(71)** Anmelder: **Apotech S.A.**
**1204 Geneva (CH)**

**(72)** Erfinder:
- **Tschopp, Jürg**
  **1066 Epalinges (CH)**
- **Thome, Margot**
  **1083 Mézières (CH)**
- **Burns, Kimberly**
  **1007 Lausanne (CH)**
- **Irmler, Martin**
  **50823 Koeln (DE)**
- **Hofmann, Kay**
  **50823 Koeln (DE)**
- **Schröter, Michael**
  **4104 Oberswil (DE)**

- **Schneider, Pascal**
  **1066 Epalinges (CH)**
- **Bodmer, Jean-Luc**
  **Del Mar, California 92014 (US)**
- **Rochat-Steiner, Véronique**
  **1322 Croy (CH)**
- **Rimoldi, Donata**
  **1091 Grandvaux (CH)**
- **Hahne, Michael**
  **28039 Madrid (ES)**
- **French, Lars, E.**
  **1206 Genève (CH)**

**(74)** Vertreter:
**Graf von Stosch, Andreas, Dr. rer. nat. et al
Bosch - Graf v. Stosch - Jehle
Patentanwälte
Theatinerstrasse 8
80333 München (DE)**

Bemerkungen:
Diese Anmeldung ist am 17 - 06 - 2002 als
Teilanmeldung zu der unter INID-Kode 62
erwähnten Anmeldung eingereicht worden.

**(54)** **FLIP-Gen und FLIP-Protein**

**(57)** Offenbart werden DNA-Sequenzen mit mindestens einer Todeseffektordomäne, Expressionsvektoren mit DNA-Sequenzen mit mindestens einer Todeseffektordomäne, mit diesen Expressionsvektoren transformierte Wirtszellen, Verfahren zur Expression oder Isolation von Genprodukten mit mindestens einer Todeseffektordomäne und gereinigte Genprodukte oder Abschnitte dieser Genprodukte mit mindestens einer Todeseffektordomäne.

EP 1 258 529 A2

**Beschreibung**

DNA-Sequenzen mit mindestens einer Todeseffektordomäne, Expressionsvektoren mit DMA-Sequenzen mit mindestens einer Todeseffektordomäne, mit diesen Expressionsvektoren transformierte Wirtszellen, Verfahren zur Expression oder Isolation von Genprodukten mit mindestens einer Todeseffektordomäne und gereinigte Genprodukte oder Abschnitte dieser Genprodukte mit mindestens einer Todeseffektordomäne.

Die Erfindung betrifft DNA-Sequenzen, die für Proteine oder Proteinabschnitte codieren, die mindestens eine Todeseffektordomäne aufweisen und die Zellapoptose inhibieren, darüber hinaus Expressionsvektoren mit den obengenannten DNA-Sequenzen mit mindestens einer Todeseffektordomäne, Wirtszellen, die mit diesen Expressionsvektoren transformiert sind, Verfahren zur Expression oder Isolation von Genprodukten mit mindestens einer Todeseffektordomäne und gereinigte Genprodukte oder Abschnitte dieser Genprodukte mit mindestens einer Todeseffektordomäne, wobei die Erfindung mit Hilfe der verschiedenen Erfindungsgegenstände in die Regulation des programmierten Zelltods eingreift.

Der programmierte Zelltod ist unter dem Namen Apoptose bekannt und tritt in der Embryogenese, in der Metamorphose, bei der Gewebeerneuerung, bei der Eliminierung krankhafter Zellen und beim Absterben der immunologischen Thymozyten und T-Zellen auf. Während der T-Zellreifung im Thymus lernen die T-Zellen, körperfremde von körpereigenen Antigenen zu unterscheiden. T-Zellen, die körpereigene Antigene erkennen, werden im Verlauf des Reifungsprozesses getötet. Der Untergang dieser Zellen stellt einen apoptotischen Prozeß dar.

Im Immunsystem wird die Apoptose auch als Verteidigungsstrategie zur Eliminierung von möglicherweise schädlichen Agentien eingesetzt. Insbesondere tritt die Apoptose bei solchen Parasiten auf, die intrazellulär ihren Vermehrungszyklus bestreiten (z.B. Viren, gewisse intrazellulär parasitierende Bakterien, z.B. Mycobakterium tuberculosis oder Mycobakterium leprae oder Bordetella pertussis oder auch Lysteriaarten, und auch Protozoen, wie z.B. Trypanosomen oder Toxoplasmen). Die apoptotischen Vorgänge bei virusinfizierten Zellen werden durch T-Lymphozyten gesteuert. Hierfür stehen verschiedene Wege zur Verfügung. So etwa kann der T-Lymphozyt durch die Ausschüttung von proapoptotischen Proteinen (z.B. Perforin oder Granzymen) oder auch durch die Exprimierung von CD95-(APO-1/Fas)-Ligand die Apoptose auslösen (Tschopp & Hofmann, Trends in Microbiology 4, 91-94, 1996). Außerdem ist es in diesem Fall auch möglich, daß die Apoptose bei infizierten Zellen durch einen zellautonomen Mechanismus gesteuert wird. Die Zellen reagieren auf die Anwesenheit von intrazellulär-parasitierenden Viren und antworten auf diese Gefahr mit einem Zellselbstmord.

Eine zentrale Rolle bei der Apoptose spielt der Fas-Rezeptor. Über den Fas-Rezeptor tritt ein extrazelluläres Signal an die Zelle heran und führt - nach Durchlaufen einer Signalkaskade - zur Apoptose der Zelle. Beispielsweise wird das Fas-Protein (CD95) auf aktivierten T-Zellen, auf B-Zellen und auf Neutrophilen exprimiert. Es ist ein 45 kD-Protein (Itoh et al., Cell 66:232, 1991; Watanabe-Fukunaga et al., Nature 356:314, 1992). Eine starke Expression von Fas-mRNA kann auch im Thymus, in der Leber, im Herzen, in der Lunge und/oder in den Ovarien von Mäusen (Watanbe-Fukunaga et al., Journal of Immunology, 148:1274, 1992) beobachtet werden. Eine Quervernetzung spezifischer monoklonaler Antikörper gegen den Fas-Rezeptor führt zur Induktion des Zelltods (Apoptose) bei zahlreichen Zellinien (Yonehara et al., Journal of Experimental Medicine, 169:1747, 1989; Traut et al., Science, 245:301, 1989).

Es liegen darüber hinaus auch Hinweise vor, daß neben dem Fas-Rezeptor andere Rezeptoren die zum Zelltod führende Signalkaskade nach Bindung eines extrazellulären Liganden initiieren können. So sind auch die membranständigen Rezeptoren TNFR-1 und TRAMP (wsl/DR-3/Apo-3) durch die Bindung eines extrazellulären Liganden an der Auslösung der Apoptose beteiligt. (Nagata, Cell 88, 355-365, 1977; Bodmer et al., Immunity 6, 79-88, 1997; Kitson et al., Nature 384, 372-375, 1996; Yu et al., Science 274, 990-992, 1996). Die genannten Rezeptoren werden auch als Todesrezeptoren ("Death Receptors") bezeichnet. Sie gehören alle zur Familie der Tumornekrosefaktor-Rezeptoren (TNF-R).

Diese Rezeptoren vermitteln das Todessignal (das apoptotische Signal) durch ein zytoplasmatisches Sequenzmotiv, das auch Todesdomäne (DD) genannt wird. Diese Todesdomäne interagiert mit den Adaptormolekülen FADD und/oder TRADD (Nagata, Cell 88, 355-365, 1997), d.h. die Todesdomäne bewirkt eine Anbindung von Adaptormolekülen. Von dem Adaptormolekül FADD ist bekannt, daß es mit der sog. ICE-ähnlichen Protease FLICE (auch genannt caspase-8, Mch5, oder MACH) assoziiert (Muzio et al., Cell 85, 817-827, 1996; Boldin, Goncharov, Goltsev, Y.V. & Wallach, Cell 85, 803-815, 1996). Diese Assoziierung wird über die sog. Todeseffektordomänen (DEDs) bewirkt, die sowohl am C-Terminus des Adaptormoleküls, z.B. FADD, als auch am N-Terminus der Protease FLICE vorhanden sind. Der Komplex aus Rezeptor, Adaptormolekül und Protease wird auch DISC ("Death Inducing Signaling Complex") genannt (Kischkel et al., EMBO J. 14, 5579-5588, 1995). Das im DISC-Komplex gebundene FLICE-Protein reguliert schließlich die weiteren proteolytischen Aktivitäten anderer Proteine aus der Gruppe der ICE-Proteasen. Diese proteolytische Kaskade führt schließlich zur apoptotischen Reaktion der Zelle (Muzio et al., Cell 85, 817-827, 1996; Boldin, Goncharov, Goltsev, Y.V. & Wallach, Cell 85, 803-815, 1996).

Die Apoptose ist, wie oben beschrieben, eine Abwehrstrategie des Immunsystems, um virusinfizierte Zellen zu töten. Viren haben ihrerseits im Gegenzug Strategien entwickelt, um der Apoptose, die ihren Vermehrungszyklus inhibieren würde, zu entgehen. Gewisse Viren tragen daher Gene, deren Genprodukte den apoptotischen Signaltransduktionsmechanismus blokkieren (Shen, Y. & Schenk, Curr. Biology 5, 105-111, 1995). Als Beispiele seien hierfür die Genprodukte CrmA des Kuhpockenvirus oder das Protein p35 des Baculovirus genannt (Shen, Y. Schenk, Curr. Biology 5, 105-111, 1995). Diese Genprodukte blockieren als Inhibitorproteine erwartungsgemäß insbesondere die Effektorproteasen des apoptotischen Signaltransduktionsmechanismus. Dabei ist die inhibitorische Wirkung auf die Cysteinproteasen, die zur ICE-ähnlichen (oder Caspase-) Proteingruppe gehören, besonders hervorzuheben (Henkart, Immunity 4, 195-201, 1996). Aber auch andere virale Gene mit anti-apoptotischen Eigenschaften sind identifiziert worden. Sie zeigen Ähnlichkeiten zum bcl-2-Gen der Säuger (Shen, Y. & Schenk, Curr. Biology 5, 105-111, 1995).

Aus den Veröffentlichungen von Boldin M.P. et al. (J. Biol. Chem. 270, 7795-7798 (1995)) und Chinnaiyan et al. (Chinnaiyan, A.M., O'Rourke, K., Tewari, M. & Dixit, V.M., Cell 81, 505-512 (1995)) ist bekannt, daß Deletionsmutanten des Adaptormoleküls FADD, das dann nur eine Todesdomäne enthält, oder der Protease FLICE, die dann nur zwei Todeseffektordomänen enthält, dominant negative inhibitorische Eigenschaften auf die frühen Ereignisse der apoptotischen Signalkaskade haben.

Aufgabe der vorliegenden Erfindung ist es, jene Gene bzw. deren Genprodukte zu identifizieren, die gleichfalls eine blockierende Wirkung auf die Zellapoptose haben und damit regulierend auf die apoptotischen Ereignisse einwirken.

Im Rahmen der vorliegenden Erfindung werden Gene, d.h. DNA-Sequenzabschnitte, sowie die von diesen Genen kodierten Genprodukte, d.h. Proteine mit inhibitorischen Eigenschaften auf die Signalkaskade, offenbart. Insbesondere offenbart die Erfindung virale, humane und murine Gene, verschiedene Gentranskripte und die entsprechend exprimierten Proteine, die die proteolytische Signaltransduktion unterbrechen und somit die Apoptose blockieren.

Gegenstand der vorliegenden Erfindung ist daher eine solche DNA-Sequenz, die für ein Genprodukt oder einen Genproduktabschnitt codiert, das (der) die Zellapoptose inhibiert und mindestens eine Todeseffektordomäne aufweist. Unter Genprodukt bzw. Genproduktabschnitt sind insbesondere das codierte Protein oder der codierte Proteinabschnitt zu verstehen. Als Genprodukt kann aber auch ein Primärtranskript, z.B. mRNA, bezeichnet werden.

Im Rahmen der vorliegenden Erfindung gehören auch alle Derivate oder Allele der erfindungsgemäßen DNA-Sequenz zum Gegenstand des Anspruches 1, sofern sie zur nativen Sequenz funktionshomolog sind. Als Allele werden alle im Reich der belebten Natur vorhandenen DNA-Sequenzen mit gleicher Funktion, aber mit charakteristischen Mutationen der nativen DNA-Sequenz, bedingt durch die evolutionäre Entwicklung, angesehen.

Gleichfalls werden alle Derivate der nativen DNA-Sequenz, seien sie nativ oder artifiziell, gemäß Anspruch 1 beansprucht. Artifizielle Veränderungen der nativen DNA-Sequenz können durch alle bekannten Methoden eingeführt werden. Die Mutationen können an einem bestimmten Locus der DNA-Sequenz durch die Synthetisierung entsprechender Oligonukleotide, die eine bestimmte Mutationssequenz enthalten, eingeführt werden. Sie sind in diesem Fall flankiert von Restriktionsstellen, die die Verbindung des synthetisierten Oligonukleotids mit der nativen Sequenz erlauben. Nach Ligation erhält man eine veränderte Sequenz, die eine bestimmte Aminosäure oder eine bestimmte Aminosäureinsertion, -substitution oder -deletion mit sich bringt.

Alternativ können Oligonukleotide durch eine "Site specific mutagenesis" zur Veränderung der Gene eingesetzt werden. Hierdurch erreicht man, daß gewisse Codons verändert sind, womit eine Substitution, Deletion oder Insertion gewünschter Art möglich ist. Beispielhafte Methoden für das Einführen von Veränderungen in eine DNA-Sequenz sind etwa bei Walder et al. (Gene 42:133, 1986), Bauer et al. (Gene 37:73, 1985), Craik (Bio Techniques, January 1985, 12-19), Smith et al. (Genetic Engineering: Principles and Methods, Plenum Press, 1981) offenbart.

Die als Derivate bezeichneten Varianten können beliebige Substitutionen, Insertionen oder Deletionen einer nativen DNA-Sequenz aufweisen, solange die erfindungsgemäße inhibierende Funktion auf die Signalkaskade gegeben ist. Insbesondere können sie konservative Substitutionen aufweisen, d.h., daß eine Aminosäure gegen eine andere Aminosäure mit ähnlichen physikochemischen Eigenschaften ausgetauscht ist. Beispiele für konservative Substitutionen sind die Substitution einer aliphatischen Aminosäure gegen eine andere aliphatische Aminosäure, so etwa Isoleucin, Valin, Leucin oder Alanin jeweils gegeneinander oder auch die Substitution polarer Aminosäuren gegen andere polare Aminosäuren, z.B. die Substitution von Lysin gegen Arginin.

Im Rahmen dieser Erfindung werden auch DNA-Sequenzen beansprucht, bei denen die erfindungsgemäße DNA-Sequenz mit anderen DNA-Sequenzen rekombiniert ist. Dies kann durch eine Fusion am C- oder am N-terminus der erfindungsgemäßen DNA-Sequenz geschehen. So etwa könnte man sich vorstellen, daß gewisse "Reportergene" mit einer erfindungsgemäßen DNA-Sequenz über den C- oder den N-terminus verbunden sind. Über die Reportergene ließe sich eine Aussage über die Quantität der Expression innerhalb gewisser Grenzen gewinnen. Andererseits sind am N- und/oder am C-Terminus, insbesondere am N-Terminus des Genproduktes der erfindungsgemäßen DNA-Sequenz "Leadersequenzen" möglich. Diese erlauben dann eine gezielte Steuerung des Im- oder Exports des Genprodukts in gewisse Zellorganellen oder auch in den extrazellulären Raum.

Unter Abschnitten des Genprodukts, die mit einer entsprechenden erfindungsgemäßen DNA-Sequenz korrelieren, versteht man verkürzte Formen des physiologischen Genprodukts. Diese verkürzten Formen können am N- oder am C-Terminus der nativen Sequenz durch an sich bekannte Verfahren verkürzt sein. Denkbar sind aber auch verkürzte Formen, denen ein Aminosäuresequenzabschnitt im internen Sequenzbereich fehlt. Derart können auch mehrere interne Aminosäuresequenzstücke aus der nativen Aminosäuresequenz entfernt sein. Die dazugehörigen DNA-Sequenzen, wobei die Degenerierung des genetischen Codes stets zu einer Vielzahl von erfindungsgemäßen DNA-Sequenzen für eine Aminosäuresequenz führt, entsprechen in allen oben genannten Fällen dem Erfindungsgedanken.

Voraussetzung dafür, daß eine DNA-Sequenz, sei es ein Derivat der nativen DNA-Sequenz oder ein Allel der nativen DNA-Sequenz oder ein beliebig zusammengesetzter Abschnitt der nativen DNA-Sequenz, durch den Anspruch 1 beansprucht ist, ist, daß die erfindungsgemäße DNA-Sequenz für ein Genprodukt oder den Abschnitt eines Genproduktes kodiert, das (der) die Zellapoptose inhibiert.

Im Rahmen dieser Erfindung werden erstmals DNA-Sequenzen offenbart, die unter physiologischen Bedingungen mit Hilfe mindestens einer Todeseffektordomäne die Zellapoptose inhibieren. Todeseffektordomänen waren bislang nur als Bindungsdomänen von Proteinen mit der Aufgabe, das extrazelluläre Signal zur Auslösung der Apoptose weiterzuleiten, bekannt. In diesem Fall dient die Todeseffektordomäne zur Bindung von caspase-Proteinen an Adaptormoleküle zur Auslösung der apoptotischen Ereignisse. Somit wirkt die Todeseffektordomäne aktivierend auf die Weitergabe des Signals, das schließlich zur Apoptose der Zelle führt. Derart ist auch die bisherige funktionale Definition der Todeseffektordomäne. Sie dient nämlich als signalübertragende Bindungsdomäne im Rahmen der Signalkaskade zur Aktivierung der Apoptose (Nagata, Cell, 88, 355-365, 1997). Die erfindungsgemäßen DNA-Sequenzen kodieren dagegen für Proteine oder Proteinabschnitte, die nicht die Weitergabe des die Apoptose auslösenden Signals zur Aufgabe haben, sondern vielmehr die Signalkaskade zur Auslösung der apoptotischen Ereignisse blockieren. Daher gehören alle DNA-Sequenzen, die inhibierend auf die schon durch ein äußeres Signal zur Apoptose vorbereiteten Zellen wirken, zum Gegenstand dieser Erfindung, sofern sie mindestens für eine Todeseffektordomäne codieren. Beliebige Derivate oder Allele, wie sie durch eine keinesfalls abschließende Aufzählung von einigen wenigen Beispielen beschrieben worden sind, gehören dann zum Gegenstand der Erfindung, wenn sie gleichfalls die apoptotische Signalkaskade unterbrechen. Sie erweisen sich dann als funktionshomolog zum Genprodukt der nativen erfindungsgemäßen DNA-Sequenz. Damit erfüllen also die erfindungsgemäßen DNA-Sequenzen mit ihren Genprodukten solche Funktionen innerhalb der Regulation der Signalkaskade, die den Funktionen der bekannten Proteine mit Todeseffektordomänen, wie etwa FLICE oder Mch4, genau entgegengesetzt sind.

Damit ist völlig überraschend durch die Lehre der vorliegenden Erfindung gezeigt worden, daß in verschiedensten Lebensformen, z.B. in Viren oder Säugern, DNA-Sequenzen zur Codierung von Proteinen mit Todeseffektordomänen existieren, die die Apoptose von Zellen weitgehend verhindern. So etwa kann die Expression einer erfindungsgemäßen DNA-Sequenz 70%, vorzugsweise mehr als 90% der Zellen nach Zugabe eines die Apoptose stimulierenden Agens, z.B. durch Zugabe des CD95-Liganden, retten.

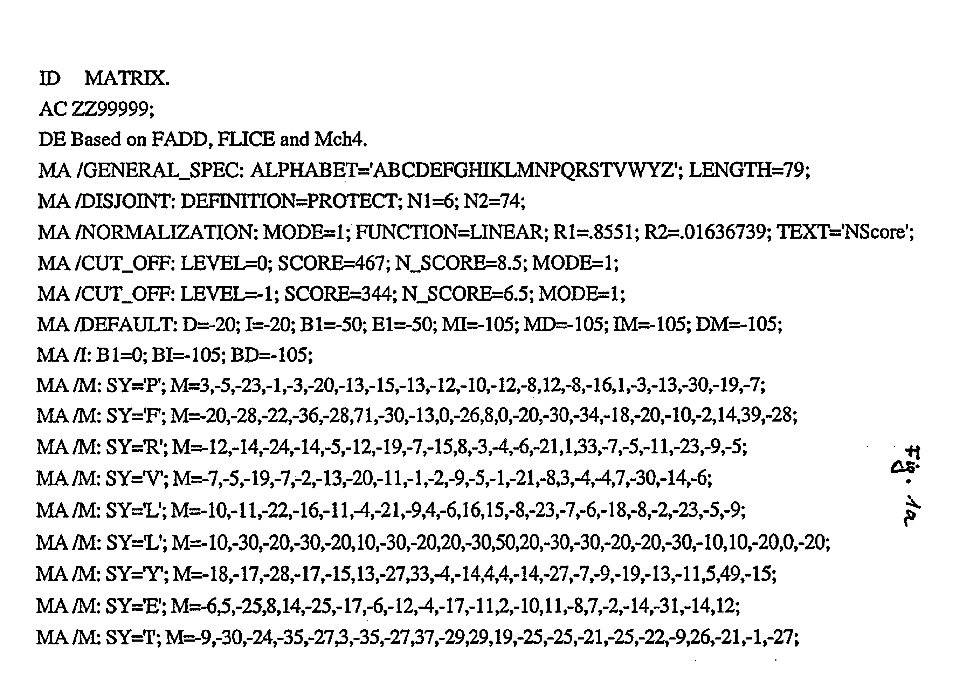

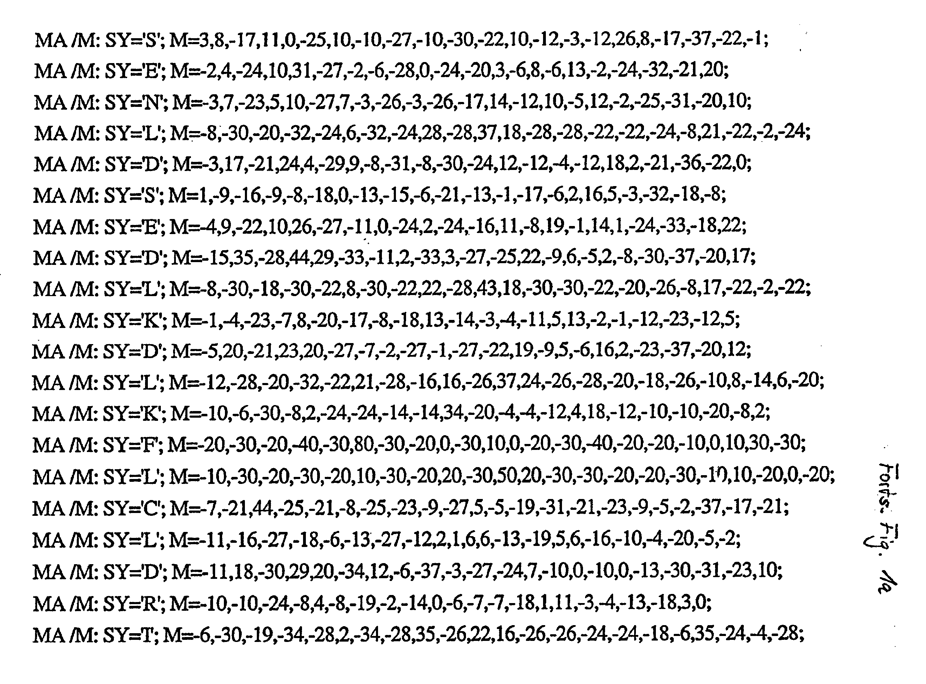

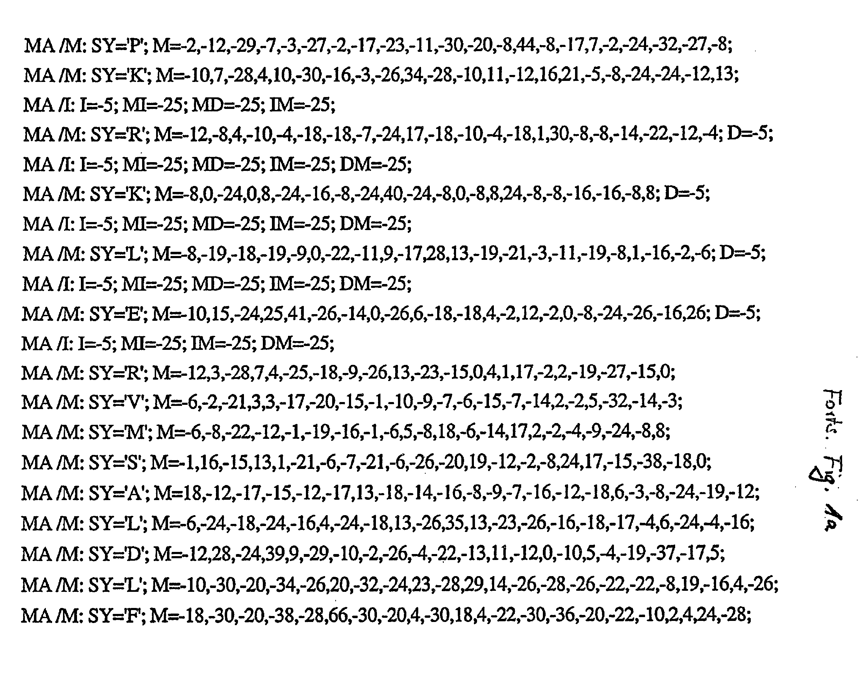

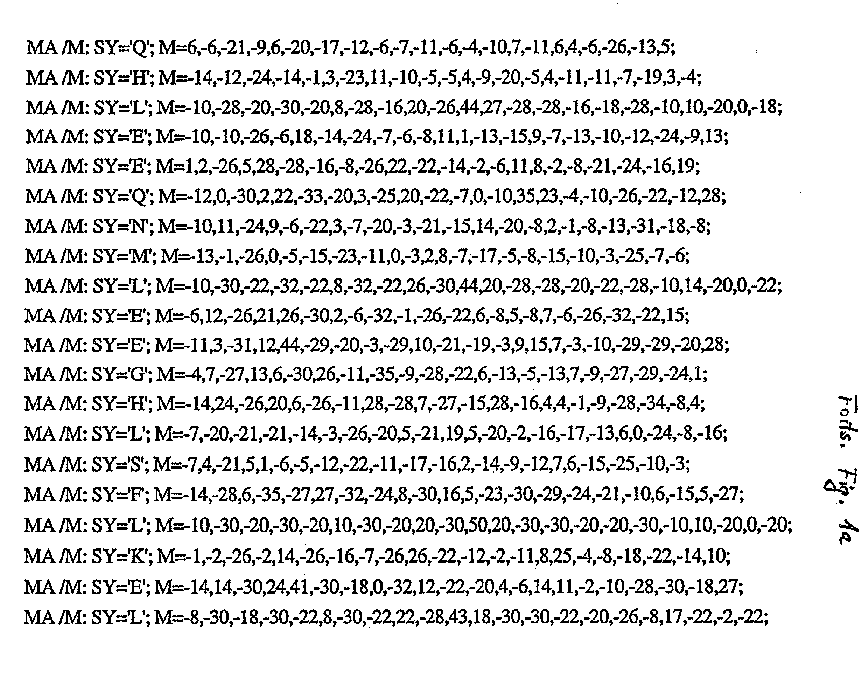

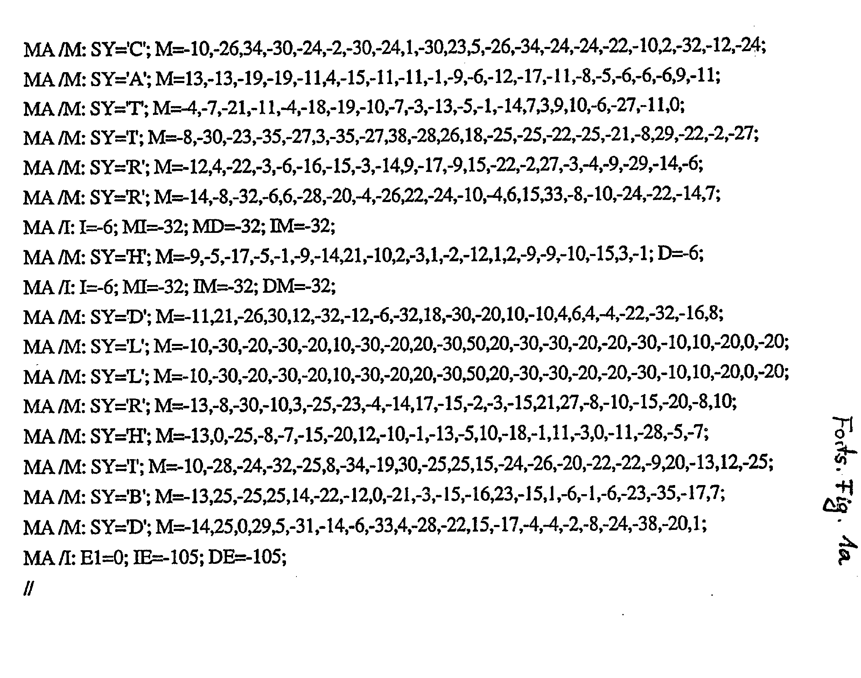

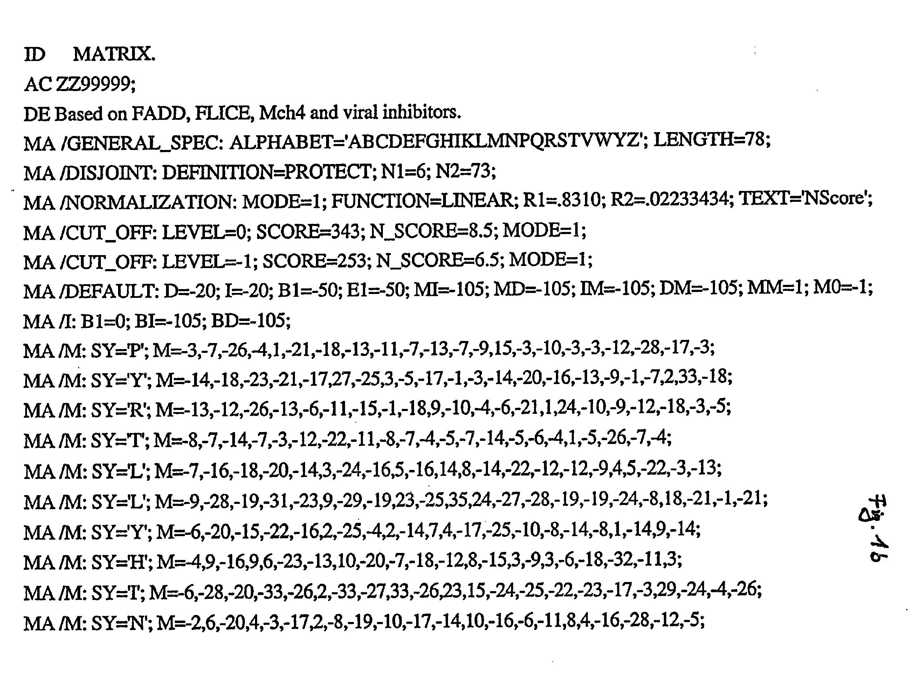

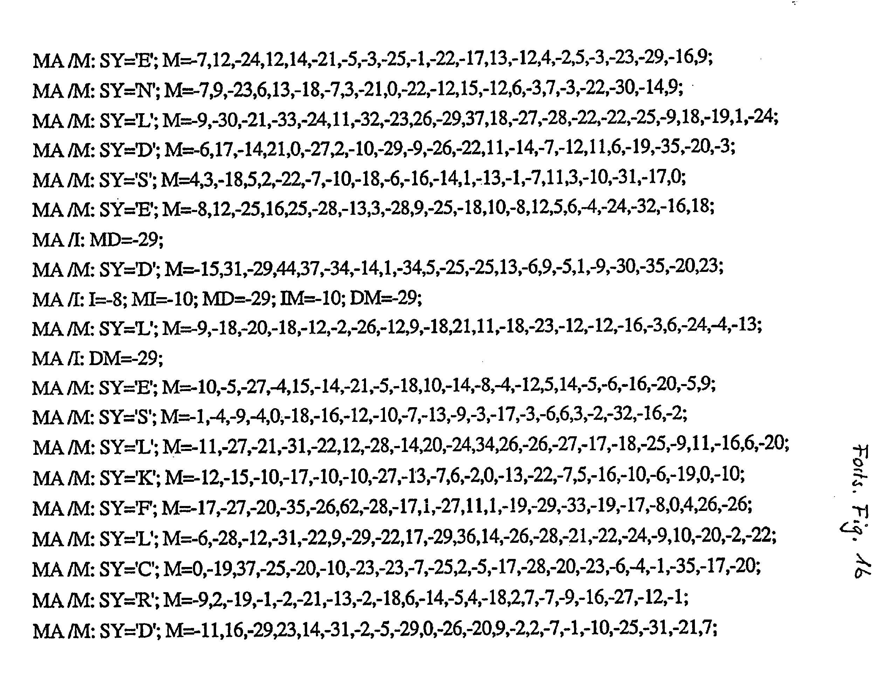

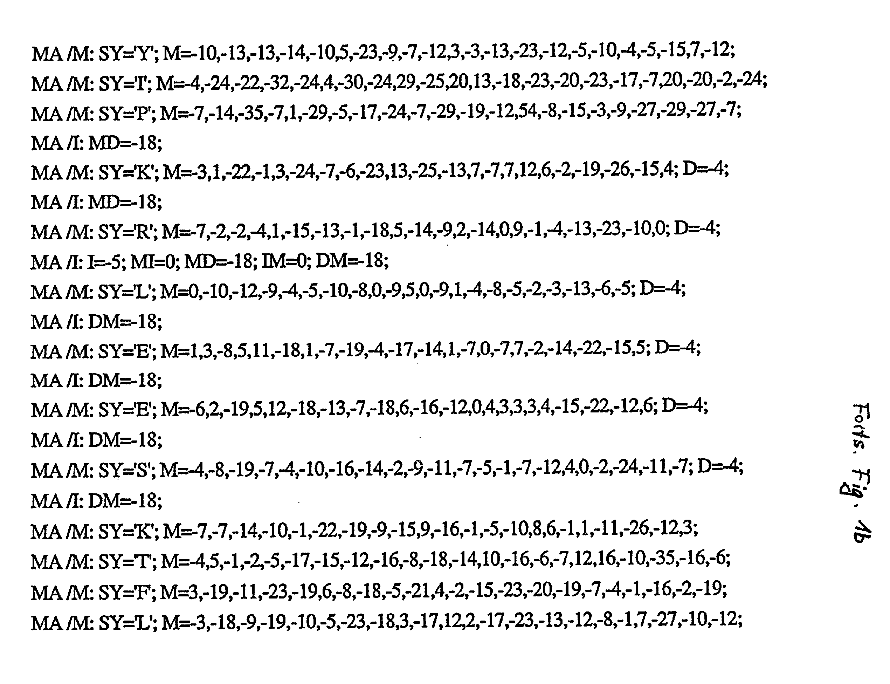

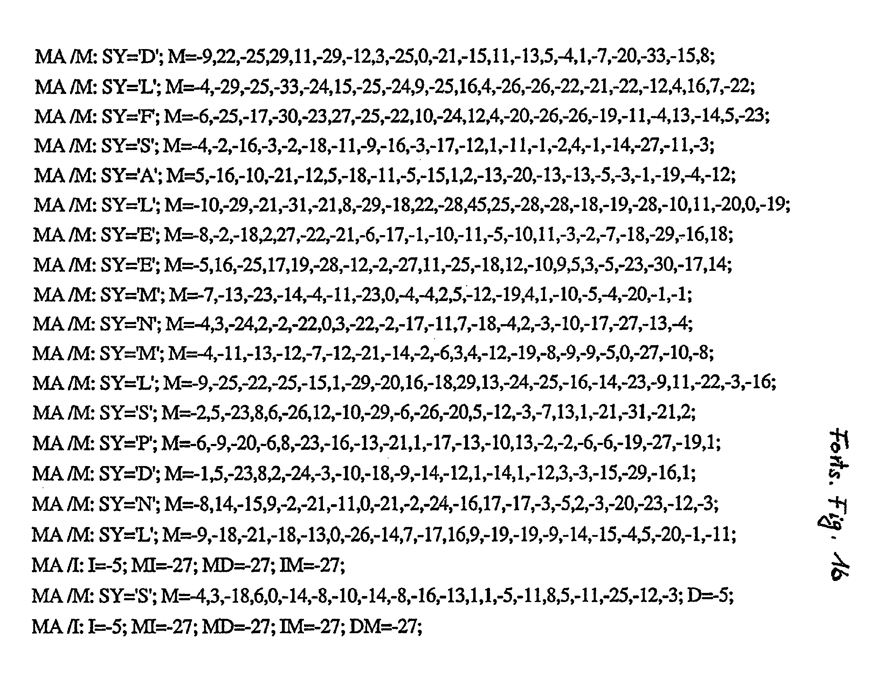

In einer bevorzugten Ausführungsform der erfindungsgemäßen DNA-Sequenz nach Anspruch 1 wird eine solche DNA-Sequenz beansprucht, die ein Signifikanzniveau von $p < 10^{-2}$ aufweist, wenn die Sequenz der Todeseffektordomäne mit einem Suchprofil nach Figur 1a oder einem Suchprofil nach Figur 1b verglichen wird. Ein solches Suchprofil wird dann angelegt, wenn im Rahmen einer Datenbanksuche verwandte Sequenzen mit Funktionshomologie identifiziert werden sollen.

Tatsächlich ist heute eine Vielzahl von Proteinsequenzen in Datenbanken hinterlegt. Beispielsweise seien die Datenbanken SWISSPROT, die von der EMBO (Eur. Mol. Biol. Org.) eingerichteten Datenbanken, oder auch die Datenbank "GenBank" (GenBank database, s. Benson et al., Nucleic Acids Research 25: 1-6 (1997)) jeweils mit hinterlegten DNA-Sequenzen und/oder Aminosäuresequenzen von Proteinen, genannt. Bei den meisten dieser DNA- und/oder Aminosäuresequenzen ist die physiologisch eng verwandte Funktion nicht bekannt. Mit Hilfe von Sequenzhomologien wird versucht, beruhend auf Sequenzen bekannter Funktionen, ähnliche, in der Datenbank vorhandene Sequenzen mit einer Sequenzhomölogie zu identifizieren, die möglicherweise gleiche oder zumindest physiologisch eng verwandte Funktionen haben. Der Erfolg einer solchen Datenbanksuche ist zwar einerseits von der Wahl des Suchalgorithmus abhängig, andererseits aber ist insbesondere das Suchprofil, das nach Maßgabe der Sequenz oder der Sequenzen bekannter Funktion erstellt wird, von zentraler Bedeutung. Erfindungsgemäß wurden zwei Suchprofile für Proteine entworfen, die, wie oben bereits beschrieben, sog. Todeseffektordomänen aufweisen. Als Grundlage für den Profilentwurf der vorliegenden Erfindung dienten dabei einerseits die von den jeweiligen DNA-Sequenzen codierten Aminosäuresequenzen der Proteine FADD, FLICE und Mch4 (auch Caspase-10 genannt) (Fernandes-Alnemri, T., *et al.*, Proc. Natl. Acad. Sci. USA 93, 7464-7469 (1996)). Dieses Profil ist in Fig. 1A dargestellt.

In Fig. 1b ist andererseits ein Suchprofil beschrieben, das auf einem verallgemeinerten Suchprofil auf der Basis von sechs Aminosäuresequenzen, codiert durch die erfindungsgemäßen DNA-Sequenzen viraler Herkunft, beruht (siehe unten). Diese erfindungsgemäßen DNA-Sequenzen stellen Gene dar, die im folgenden als FLIP-Gene, bei viraler Herkunft aber als vFLIP-Gene bezeichnet werden. Diese Gene codieren für die vFLIP-Proteine. Die Profile sind hierbei auf die Aminosäuresequenzen der bekannten Todeseffektordomänen ausgerichtet. Neu identifizierte Proteine bzw. DNA-Sequenzen umfassen dann mindestens eine profilgemäße Todeseffektordomäne.

Die Datenbanksuche wurde mit dem Algorithmus von Bucher *et al.* (Bucher, P., Karplus, K., Moeri, N. & Hofmann, K.A., Computer Chem. 20, 3-24 (1996)) durchgeführt. Die vorliegenden Aminosäuresequenzen und die ihnen zugrundeliegenden DNA-Sequenzen wurden in der Datenbank "GenBank" identifiziert. Als Datenbanken für die Homologiesuche kommen jedoch alle kommerziellen oder nicht-kommerziellen Datenbanken mit Aminosäuresequenzeinträgen in Betracht. Auch eine Suche in DNA-Datenbanken mit einem DNA-Suchprofil für Todeseffektordomänen ist denkbar. Die Erfindung wird auch nicht durch den derzeitigen Stand der Eintragungen, d.h. durch die zum Prioritätszeitpunkt der Anmeldung vorliegende Zahl der hinterlegten Proteinsequenzen, beschränkt. Alle zukünftigen Datenbankeintragungen mit den dazugehörigen erfindungsgemäßen DNA-Sequenzen und den entsprechenden Proteinsequenzen, sofern sie den entworfenen Profilen der Fig. 1A oder 1B genügen, sind durch diese bevorzugte Ausführungsform der Erfindung eingeschlossen.

Erfindungsgemäß muß nach der bevorzugten Ausführungsform des Anspruchs 2 ein Signifikanzniveau von $p < 10^{-2}$ gegeben sein, wenn die Sequenz der Todeseffektordomäne mit einem Suchprofil nach Figur 1A oder einem Suchprofil nach Figur 1B verglichen wird. Zur Berechnung der statistischen Signifikanz der aufgrund des Suchprofils ermittelten Treffer in der Datenbank geben die p-Werte hierbei statistische Signifikanzwerte an, die sich aus der Umwandlung von normalisierten Profilscores (Nscores) ergeben. Die Programme *pfscan* und *pfsearch* (beide sind Bestandteile des Programmpakets pftools) liefern in der Ausgabedatei sogenannte "normalisierte scores" (Nscores) als Maß für die Signifikanz eines Profil-/Sequenzvergleichs. pftools ist ein frei verfügbares Programmpaket für "Generalized Profile"-Anwendungen. Beschrieben ist es in der Publikation von Bairoch et al. (Nucleic Acids Research 25: 217-221 (1997)). Erhältlich ist es im Internet unter ftp://ulrec3.unil.ch/pub/pftools oder auf die Anfrage bei Dr. Philipp Buchner, Swiss Institute of Exp. Cancer Research, CH-1066 Epalinges, Schweiz. Dabei dient das Programm pfsearch für die Suche mit einem Profil gegen eine Sequenzdatenbank, während das Programm pfscan als Mittel zur Suche mit einer Sequenz gegen eine Sammlung von Profilen dient. Die normalisierten scores werden aus Rohscores (raw scores) durch Anwendung von Skalierungsparametern berechnet. Die Skalierungsparameter werden zum Teil aus der Profilkonstruktion durch die Suche in einer randomisierten Datenbank gewonnen, wobei sie dann zu einem Bestandteil des Profils werden. Dabei wird die randomisierte Datenbank, die z.B. auf der Datenbank SWISSPROT beruht, durch ein sogenanntes "Abschnittsshuffling" mit einer Fensterbreite von 20 Residuen gewonnen (Pearson und Lipman, Proceedings of National Academy of Science, USA, 85, 2444-2448, 1988). Nähere Erläuterungen zum Algorithmus finden sich auch in den Veröffentlichungen von Bucher *et al.* (Computer Chemistry 20, 3-24, 1996) und Hoffmann *et al.* (TIBS 20, 347-349, 1995).

Nscores kann man als dekadischen Logarithmus der Anzahl von Aminosäureresten in einer randomisierten Datenbank interpretieren, in der man genau ein Protein der geforderten Qualität per Zufall finden würde. Beispielhaft läßt sich sagen, daß ein Nscore von 9,0 in einer randomisierten Datenbank von $10^9$ Resten per Zufall einmal erwartet werden kann. Da die Proteindatenbank gegenwärtig etwa 58000000 Reste enthält, das entspricht einer Größe von $10^{7.76}$, würde man ein Protein mit einem Nscore von 7,76 einmal im statistischen Mittel erwarten. Höhere Nscores bedeuten bessere Protein-/Profiltreffer als sie in einer Datenbank der aktuellen Größe durch reinen Zufall zu erwarten wären. In der Praxis hat sich ein Nscore von 8,5 bis 8,7 als Signifikanzgrenze bewährt. Die Wahrscheinlichkeit p, einen solchen Treffer in der Proteindatenbank per Zufall zu finden, ist etwa 0,1. Dies ergibt sich aus folgender Rechnung:

$$p = 10^{-(\text{Nscore} - \log(\text{Datenbankgröße}))}.$$

Diese Gleichung gilt für Nscores > 8,5.

Zusammenfassend läßt sich sagen, daß die Irrtumswahrscheinlichkeit hierbei als Wahrscheinlichkeit dafür definiert ist, daß ein Treffer der geforderten Qualität durch Zufall bedingt ist und keine evolutionäre Verwandtschaft zur Ursache hat. Nähere Ausführungen zur angewandten Suchmethode mit Hilfe eines Suchprofils in einer Datenbank finden sich auf den World-Wide-Webseiten (WWW-Seiten): http://ulrec3.unil.ch/profile/profile.html und http://ulrec3.unil.ch/profile/scoredoc.html.

In einer besonderen Ausführungsform codieren die erfindungsgemäßen DNA-Sequenzen für Proteine, die zwei Todeseffektordomänen aufweisen. Die Präsenz von zwei Todeseffektordomänen erlaubt eine starke Bindung an das zu bindende Protein, also z.B. das Adaptormolekül FADD. Die beiden Todeseffektordomänen der erfindungsgemäßen DNA-Sequenz zeigen zwar deutliche Ähnlichkeiten, sind jedoch bevorzugt nicht identisch. Durch Aminosäuresequenz-Abweichungen der beiden Todeseffektordomänen wird eine Spezifizierung jeder Domäne erreicht, die funktional von Bedeutung ist. Somit kann das Bindungsverhalten der durch die erfindungsgemäßen DNA-Sequenzen codierten Proteine an Proteine des Apoptosesignaltransduktionsweges optimal reguliert werden. Über die Bindungskonstante kann bei vorgegebener zellulärer Konzentration des erfindungsgemäß codierten Proteins die Intensität des apoptotischen Signals beeinflußt werden. Im Rahmen dieser Erfindung werden allerdings auch solche artifiziellrekombinanten DNA-Sequenzen beansprucht, die für Proteine mit zwei identischen Todeseffektordomänen codieren. Durch Rekombinantion bestimmter erfindungsgemäßer DNA-Sequenzen (z.B. DED1 von HHV-8 und DED2 von MCV (159L) oder Duplizierung von DED1 von HHV-8 oder ähnliche Kombinationen) können bestimmte differenzierte Inhibitionswirkungen der apoptotischen Reaktion erreicht werden.

In einer besonderen Ausführungsform der vorliegenden Erfindung binden die durch die erfindungsgemäße DNA-Sequenz codierten Proteine (oder Genprodukte) an ein Protein des Apoptosesignaltransduktionsweges. Auf diese Weise können die durch die erfindungsgemäße DNA-Sequenz codierten Genprodukte in den Apoptosesignaltransduktionsweg eingreifen und ihn damit regulieren. Als Zielprotein für die Bindung kommen insbesondere Todeseffektordomänen tragende Proteine in Frage. Im Signaltransduktionsweg können z.B. diese Zielproteine als Adaptorproteine, wie bspw. FADD, oder auch als Proteasen, ganz besonders als caspase-ähnliche Proteasen, wirken. Derart kann die Signalweiterleitung von einem Protein des Apoptosesignaltransduktionsmechanismus an die folgenden Elemente der Signalkaskade blockiert werden. Dann mündet das extrazellulär ausgelöste und über den Rezeptor weitergegebene Apoptosesignal in eine Sackgasse ein. Eine weitere Signalübertragung auf die proteolytischen Glieder der Signalkaskade ist damit nicht mehr möglich. Die Apoptose der Zelle wird verhindert.

Insbesondere bevorzugt ist die spezifische Bindung der Genprodukte der erfindungsgemäßen DNA-Sequenzen über eine oder mehrere Todeseffektordomänen mit der (den) Todeseffektordomäne(n) von Proteinen des Apoptosesignalstransduktionsweges.

Ganz besonders bevorzugt sind solche DNA-Sequenzen, deren Genprodukt den dargestellten Suchprofilen genügt und die gleichzeitig im frühestmöglichen intrazellulären Stadium der Apoptosesignalweitergabe bereits zu einer Blockade des extrazellulären Apoptosesignals führen. Dies geschieht durch solche Genprodukte, die an den cytoplasmatischen Abschnitt eines membranständigen, zellulären Rezeptors des Apoptosesignaltransduktionswegs direkt oder indirekt binden.

Insbesondere kommen in diesem Zusammenhang die Rezeptoren des Tumornekrosefaktortyps in Frage. Zu nennen sind hier die Rezeptoren TRAMP, CD95 und TNFR-1, sowie der Rezeptor für den todesinduzierenden Liganden TRAIL (Wiley *et al*., Immunity 3, 673-682 (1995)).

Weiterhin sind insbesondere bevorzugte DNA-Sequenzen solche, die für Genprodukte codieren, die an lösliche intrazelluläre Proteine des Apoptosesignaltransduktionsweges binden. Hierbei wird beispielsweise eine Wechselwirkung zwischen dem Genprodukt der erfindungsgemäßen DNA-Sequenz mit dem Protein FADD genannt. Geschieht diese Wechselwirkung über die jeweiligen Todeseffektordomänen, so ist eine Interaktion zwischen den Adaptormolekülen, z.B. FADD oder TRADD, mit dem cytoplasmatischen Teil des Rezeptors noch möglich. Blockiert ist allerdings die Signalweitergabe vom Adaptormolekül auf die proteolytisch wirkenden Proteine der Signalkaskade. Dies bedeutet, daß die Wechselwirkung zwischen den Todesdomänen des cytoplasmatischen Teils des Apoptose-Rezeptors und dem Adaptormolekül noch möglich sind, allerdings der darauf folgende Signalweitergabeschritt, z.B. von FADD an FLICE, blockiert ist.

Zusammenfassend kann gesagt werden, daß bei einer ganz besonders bevorzugten Ausführungsform der erfindungsgemäßen DNA-Sequenz sich deren Genprodukt an ein Adaptormolekül so anlagert, daß sich ein Komplex aus Rezeptor, in Form seines cytoplasmatischen Teils, aus dem Adaptormolekül und dem Genprodukt der erfindungsgemäßen DNA-Sequenz bildet und damit die Inhibition der apopotischen Signalkaskade gegeben ist.

Im Zuge der weiteren Signalkaskade der Apoptose kommt es zu der Bildung des oben bereits beschriebenen DISC-Komplexes. Dieser DISC-Komplex setzt sich aus mehreren Elementen, die an das CD95 Protein angelagert sind, zusammen. Sie tragen die Bezeichnung CAP1 bis CAP6, wobei CAP1 ein FADD-Protein ist, CAP2 ein hyperphosphoryliertes FADD, CAP3 ein bislang unbekanntes Protein mit einem N-Terminus des FLICE-Proteins, CAP4 ein pro-FLICE Protein und CAP5 bzw. CAP6 bereits abgespaltene FLICE-Prodomänen sind, die als Indikatoren für eine Aktivierung von FLICE-Protein innerhalb des DISC-Komplexes dienen können. Im Falle einer agonistischen Stimulierung des Rezeptors CD95 lagern sich diese Komponenten mit dem CD95-Protein zusammen. In Anwesenheit eines Genprodukts der erfindungsgemäßen DNA-Sequenz kommt es dagegen nicht zu einem vollständigen Aufbau des DISC-Komplexes, obwohl sich CAP1 erwartungsgemäß an das CD95-Protein anlagert, weil gleichzeitig die Präsenz von CAP4 und CAP3 (jeweils FLICE oder FLICE-ähnliche Komponenten) stark reduziert ist. Darüber hinaus sind die Komponenten CAP5 und CAP6 in den entsprechenden Versuchen nicht detektierbar.

In einer besonderen Ausführungsform ist somit durch das Genprodukt einer erfindungsgemäßen DNA-Sequenz die Anlagerung und Aktivierung des FLICE-Proteins nahezu blockiert. Damit können auch keine in der Signalkaskade später folgenden proteolytisch wirkenden Komponenten aktiviert werden. Das Apoptosesignal ist somit unterbrochen. Genauso wie im Falle des CD95-Rezeptors gilt auch für den Rezeptor TRAMP, daß das FLICE-Protein durch die Assoziierung mit den Adaptorproteinen TRADD und FADD aktiviert wird. Auch hier tritt eine Inhibierung des exogen stimulierten Zelltods durch eine Koexpression von Genprodukten der erfindungsgemäßen DNA-Sequenzen ein. Diese Genprodukte der erfindungsgemäßen DNA-Sequenzen können auch die Apoptosesignalweitergabe dann blokkieren, wenn exogen das Protein TRAIL, ebenfalls ein Mitglied der TNF-Ligandenklasse, den Zellen als Apoptosestimulanz zugeführt wird. In allen genannten Fällen ergibt sich jeweils eine dosisabhängige Korrelation zwischen der Blockade des Apoptosesignals und der Konzentration des Genprodukts der erfindungsgemäßen DNA-Sequenzen.

In einer besonders bevorzugten Ausführungsform inhibiert das Genprodukt der erfindungsgemäßen DNA-Sequenzen die Zellapoptose vollständig, indem es die Signalweitergabe an die proteolytischen Proteine in der Kaskade verhindert.

In einer weiteren Ausführungsform treten die erfindungsgemäßen DNA-Sequenzen in viralen, prokaryotischen oder eukaryotischen DNA-Erbinformationsmolekülen auf.

Die erfindungsgemäßen DNA-Sequenzen können also in allen Lebensformen auftreten.

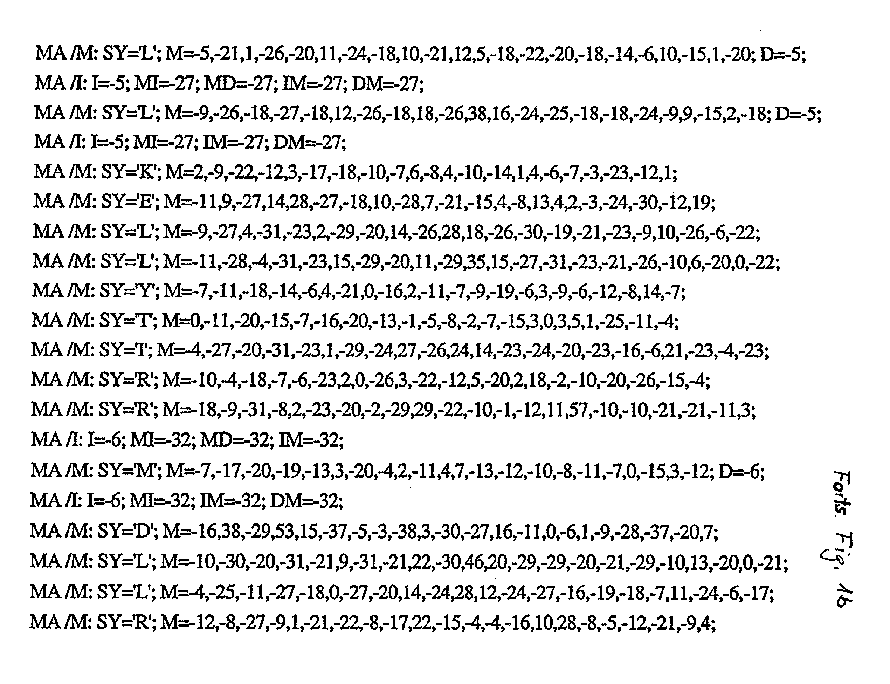

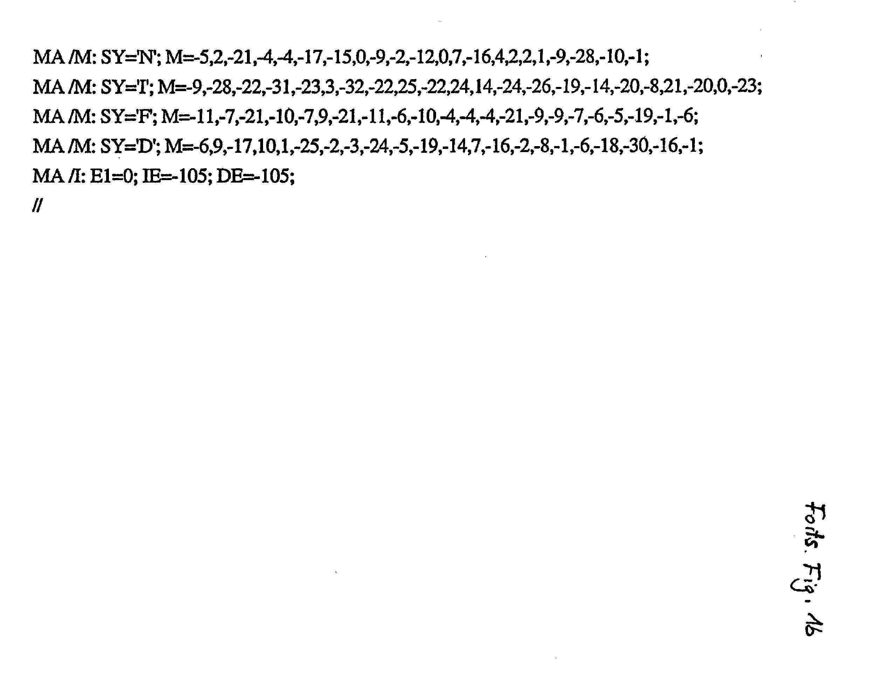

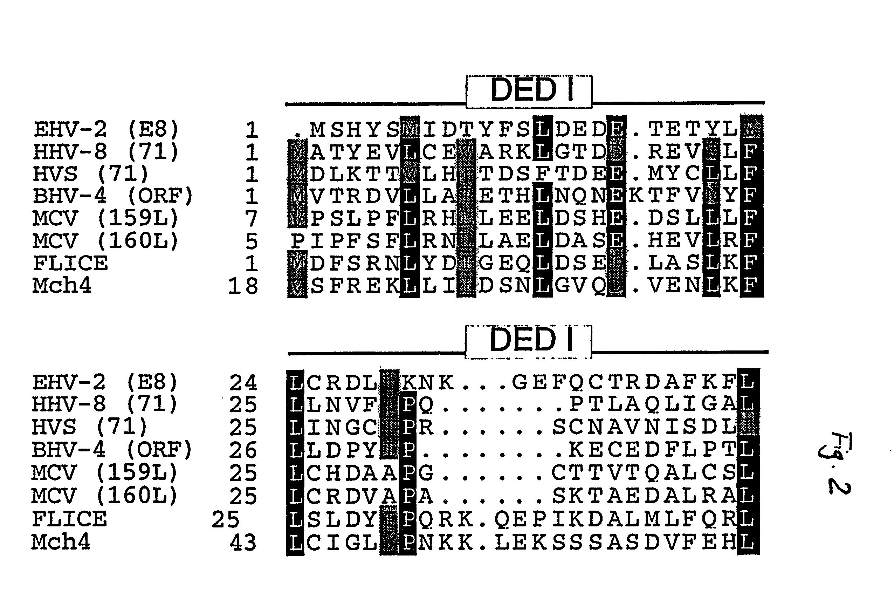

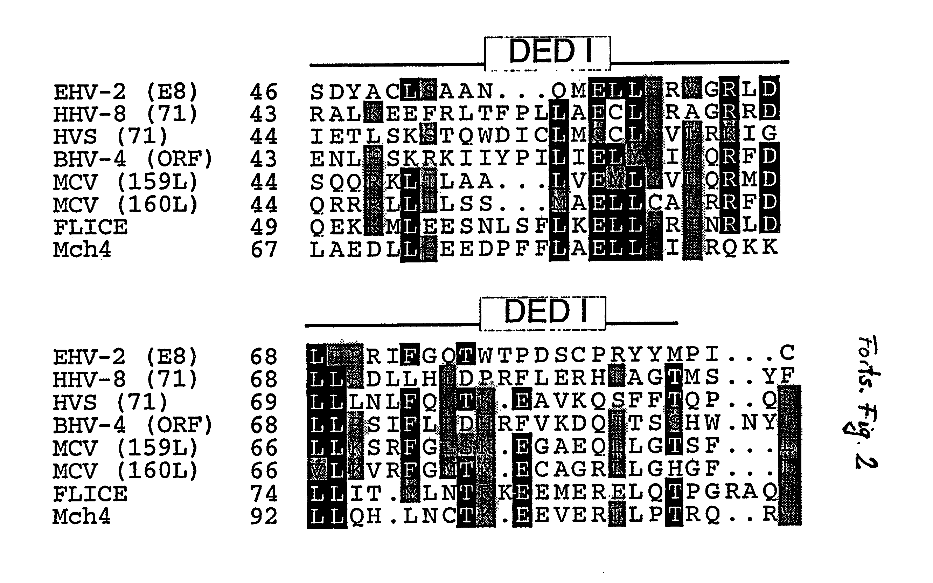

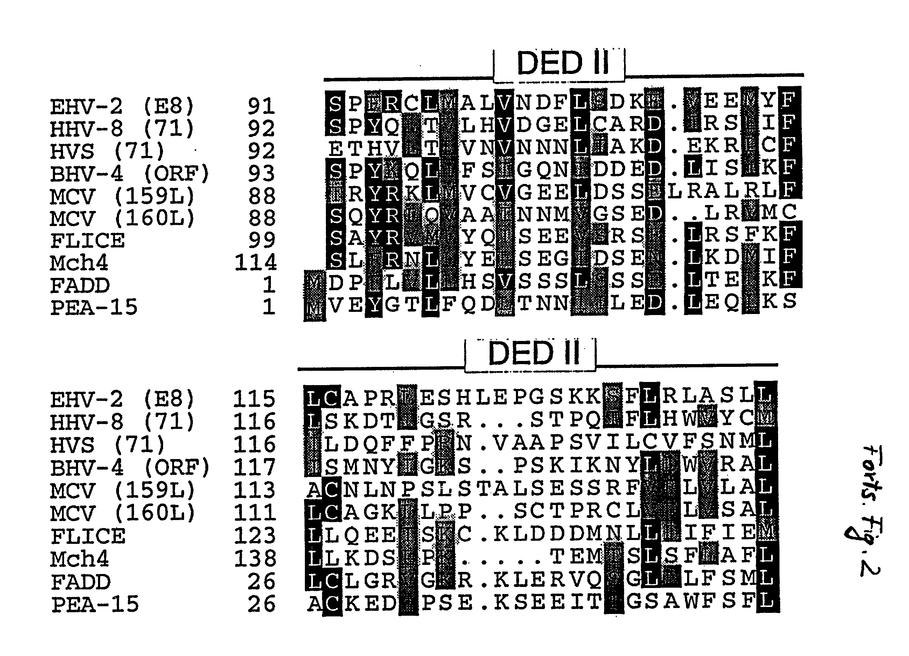

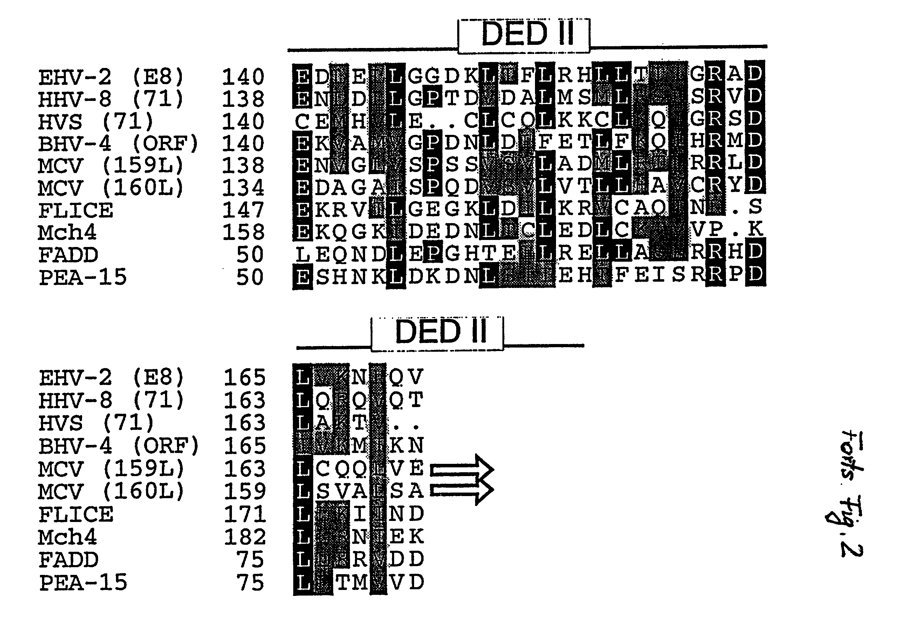

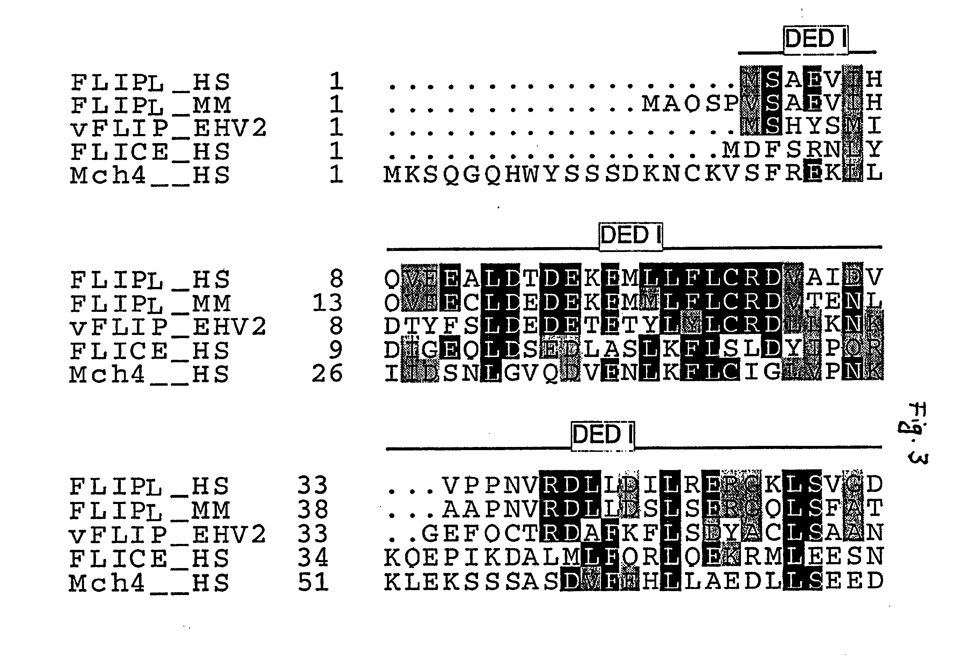

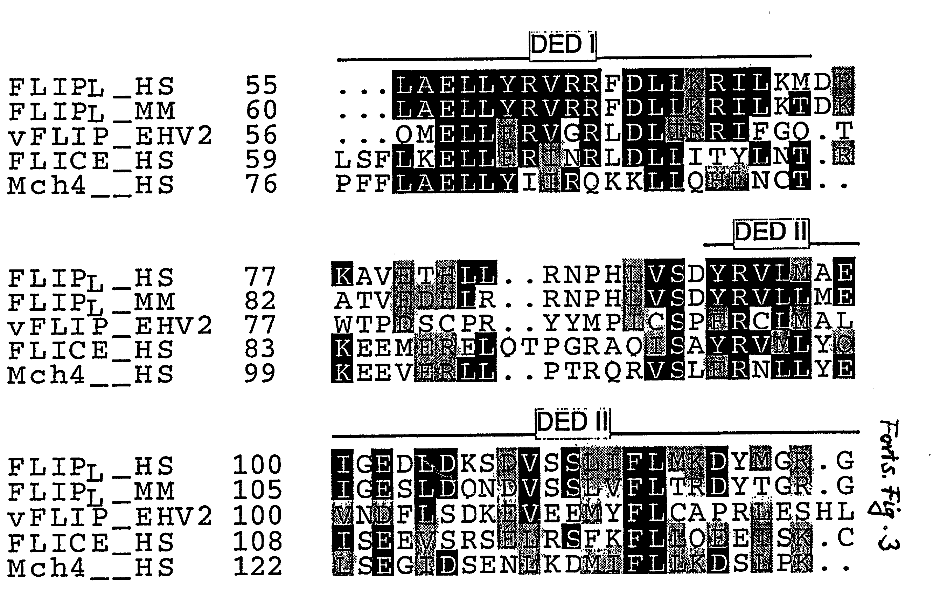

Insbesondere treten DNA-Sequenzen, die zur Expression von Genprodukten führen, wobei diese mindestens eine Todeseffektordomäne aufweisen und die Zellapoptose inhibieren, bei Viren auf. Hier hat der Einsatz des Suchprofils nach Fig. 1a oder Figur 1b zur Identifikation mehrerer Proteine mit Sequenzhomologie zu den bekannten zellulären Apoptosesignalproteinen FLICE und Mch4 geführt. Erfindungsgemäße DNA-Sequenzen treten zum Beispiel beim Molluscum cantagiosum Virus (MCV) auf. Hier ist insbesondere das humane MCV als Träger der erfindungsgemäßen Sequenzen zu nennen. Bei diesem Virus tritt eine erfindungsgemäße DNA-Sequenz im ORF 159L oder auch im ORF 160L auf. Diese ORFs codieren für Proteine, die die Zellapoptose inhibieren können. Aminosäuresequenzen erfindungsgemäßer DNA-Sequenzen sind in der Datenbank GenBank unter dem Zugangscode U60315 (für das ORF 159L bzw. für das ORF 160L des MCV) eingetragen. Darüber hinaus sind in Figur 2 jeweils N-terminale Aminosäuresequenzen der beiden ORFs angegeben. In diesem Bereich liegen zwei Todeseffektordomänen. Beide Aminosäuresequenzen weisen eine C-terminale Verlängerung auf, die in der Figur 2 durch Pfeile angedeutet ist. Die Extensionen haben eine Länge von 66 Aminosäuren (ORF 159L) oder von 102 Aminosäuren (ORF 160L).

In einer besonders bevorzugten Ausführungsform treten die erfindungsgemäßen DNA-Sequenzen bei Herpesviren, insbesondere bei γ-Herpesviren auf. Bei den Herpesviren handelt es sich um Viren mit doppelsträngiger DNA, deren Morphogenese im Kern der Wirtszelle abläuft.

Nach Anspruch 15 wird insbesondere eine DNA-Sequenz, deren Genprodukt eine Aminosäuresequenz nach dem "GenBank"-Zugangscode U20824 (E8), wobei E8 das ORF angibt, enthält, beansprucht. Diese Aminosäuresequenz stammt vom Virus EHV-2. Bei dieser Sequenz fällt auf, daß an vier Stellen der Domäne DED I keine Übereinstimmung mit den jeweils sonst unter den weiteren in Figur 2 gelisteten Sequenzen besteht. An diesen Positionen zeigt nur die Sequenz von EHV-2 Abweichungen.

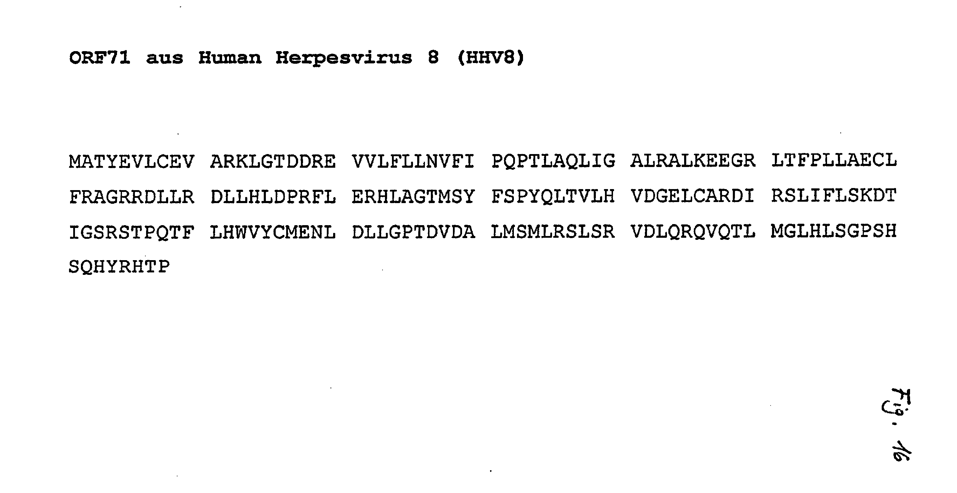

Nach Anspruch 16 wird insbesondere eine DNA-Sequenz, deren Genprodukt eine Aminosäuresequenz nach Figur 16 umfaßt, beansprucht. Sie stellt eine Aminosäuresequenz des Virus HHV-8 dar. Diese Sequenz liegt im ORF 71. Eine ihr zugrunde liegende, erfindungsgemäße DNA-Sequenz ist in der GenBank unter dem Zugangscode (U90534 (71)) verzeichnet.

Insbesondere wird auch eine DNA-Sequenz beansprucht, deren Genprodukt eine Aminosäuresequenz nach dem GenBank Zugangscode X64346 (ORF 71) umfaßt. Diese Sequenz liegt beim HSV-Virus vor.

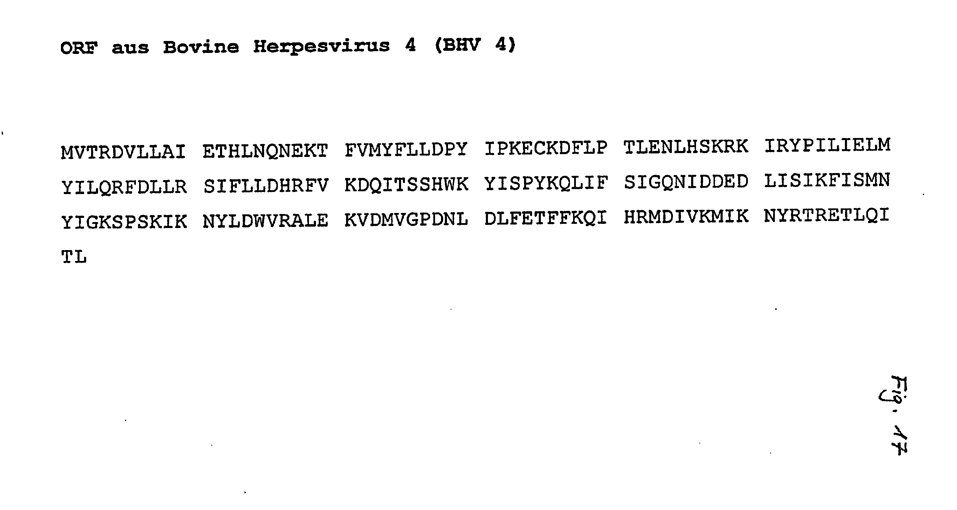

Schließlich wird insbesondere auch eine DNA-Sequenz beansprucht, deren Genprodukt eine Aminosäuresequenz nach Figur 17 umfaßt. Für diese virale Sequenz (BHV-4) gibt es unter Z46385 einen GenBank Dateneintrag einer erfindungsgemäßen DNA-Sequenz.

Im Sinne dieser Erfindung tragen diese Gene bzw. Genprodukte die Bezeichnung vFLIP-Gene oder vFLIP-Proteine (Abkürzung für virale FLICE-Inhibitionsproteine).

Hier finden sich zwei Sequenzmotive mit einer hochsignifikanten Homologie zu den Todeseffektordomänen. Das Signifikanzniveau p für das Suchprofil nach Figur 1a oder 1b ist dabei erfindungsgemäß kleiner als $10^{-2}$. Sie alle weisen jeweils zwei Todeseffektordomänen auf (DED I bzw. DED II). Diese sind über das in Fig. 1a gezeigte Suchprofil mit Hilfe des Algorithmus von Bucher at al. (s.o.) gefunden worden. Damit zeigen sie die charakteristischen Sequenzmotive einer Todeseffektordomäne.

Für die Wirksamkeit der viralen FLIP-Proteine ist mindestens eine Todeseffektordomäne, vorzugsweise sind zwei Domänen dieser Art, wie unter physiologischen Bedingungen, erforderlich. Bei diesen fünf Viren (EHV-2, HHV-8, HVS, BHV-4 und MCV) werden die beiden Todeseffektordomänen durch eine Sequenz von mindestens 16 Aminosäuren verbunden. In dieser Verbindungssequenz liegt nur eine schwache Homologie zwischen den fünf genannten Viren vor. Vorzugsweise hat die Verbindungssequenz eine Länge von 16 bis 19 Aminosäuren.

Die erfindungsgemäßen DNA-Sequenzen können für Genprodukte codieren, die mindestens einen oder beide dieser Todeseffektordomänen Aminosäuresequenzabschnitte enthalten.

In einer Ausführungsform der vorliegenden Erfindung liegt die erfindungsgemäße DNA-Sequenz in der Erbinformation von Säugetierzellen vor. In diesem Zusammenhang kommen alle denkbaren Säugetierzellen in Frage. Insbesondere betrifft die Erfindung humane DNA-Sequenzen der erfindungsgemäßen Art.

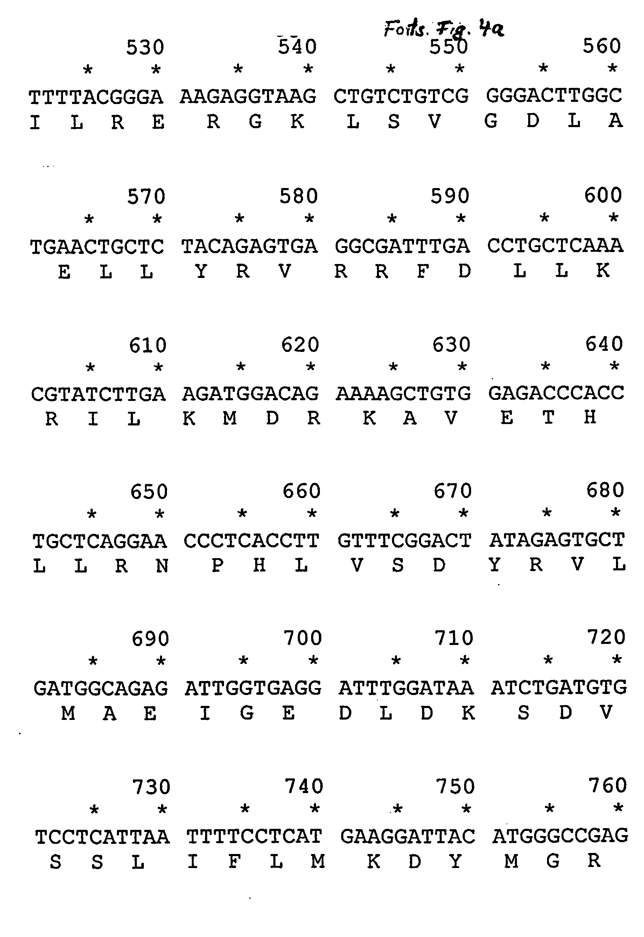

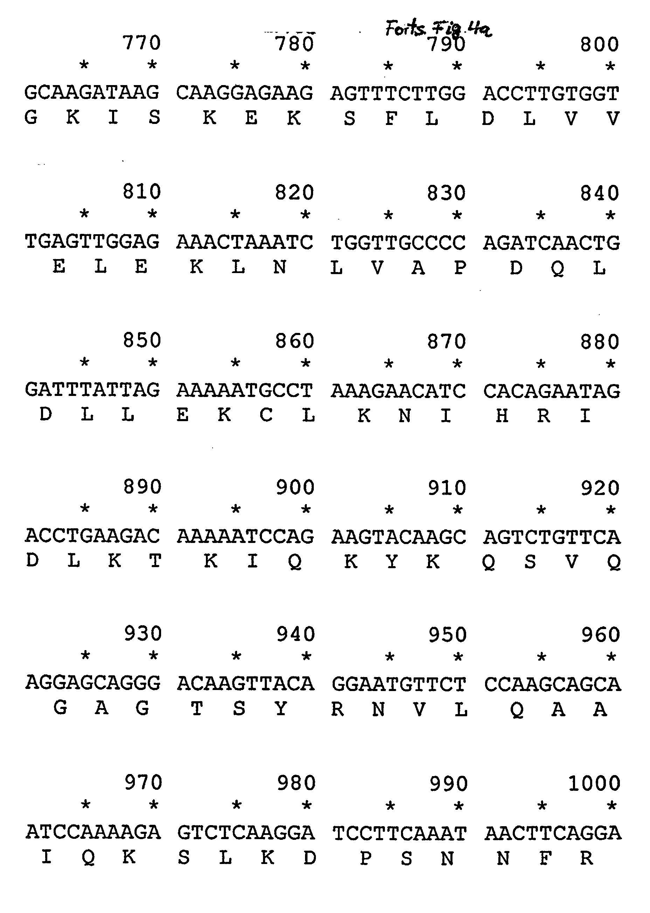

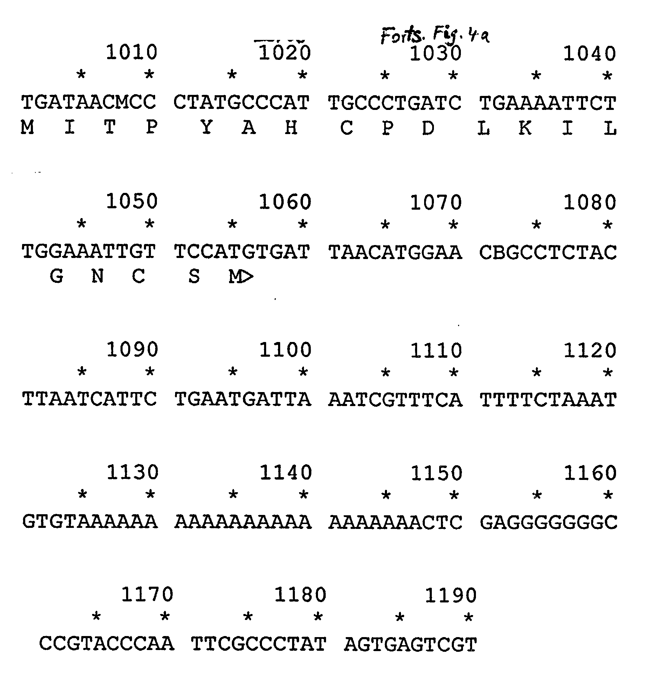

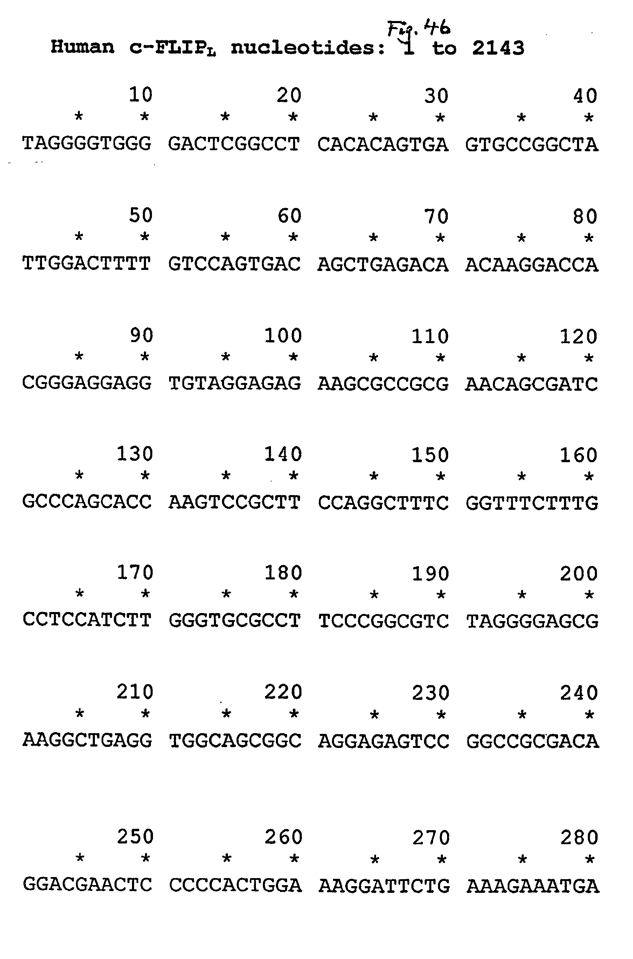

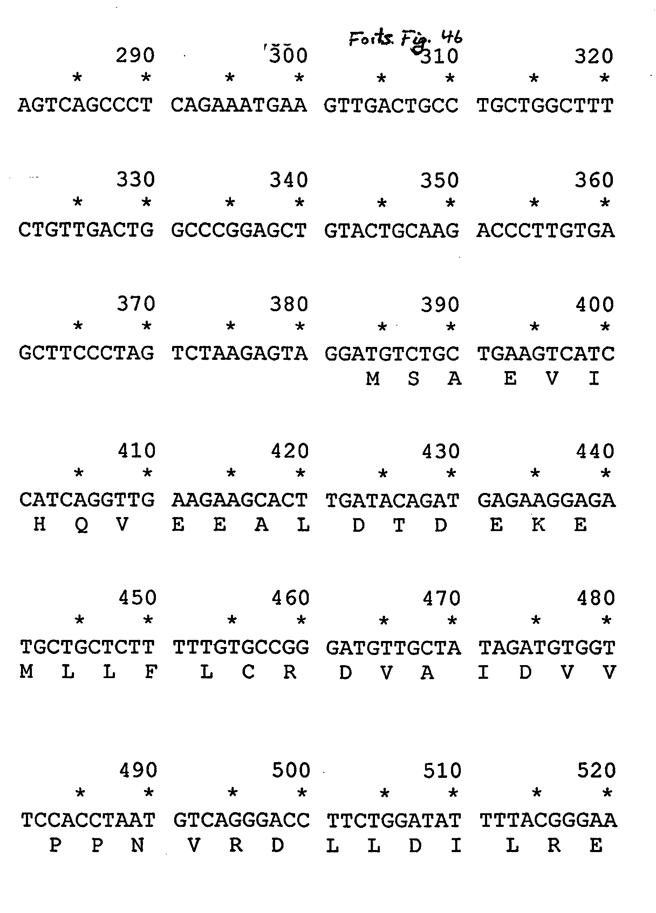

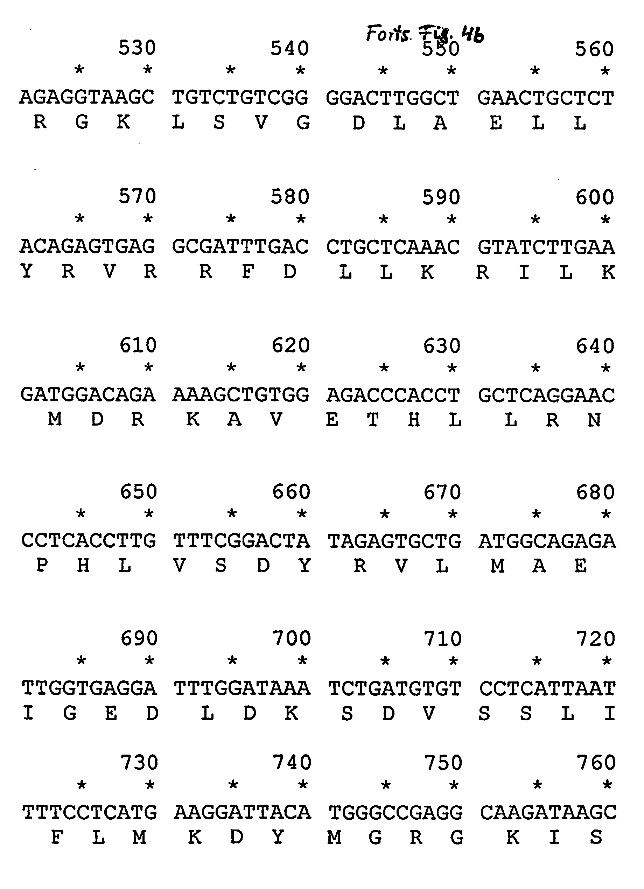

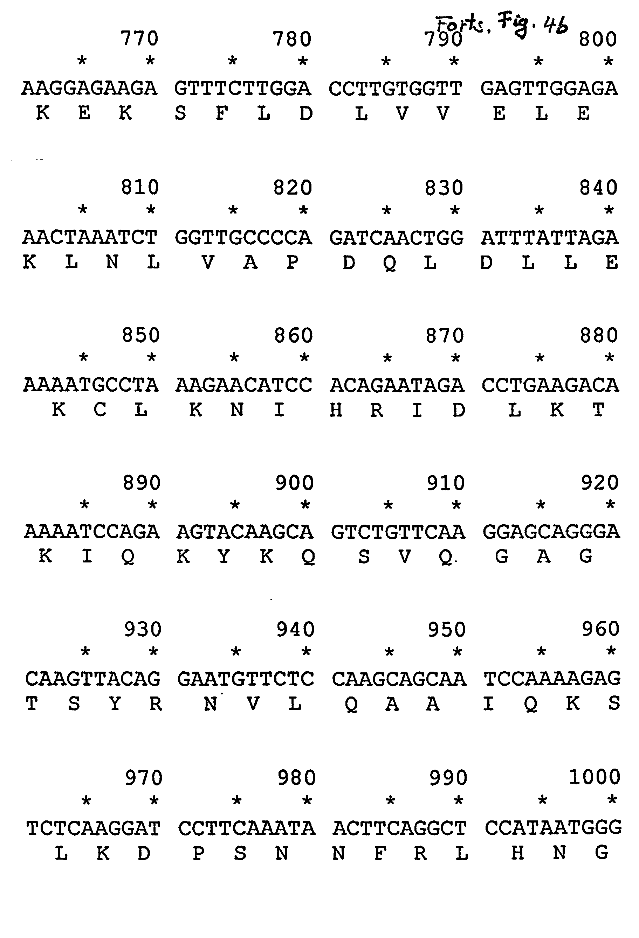

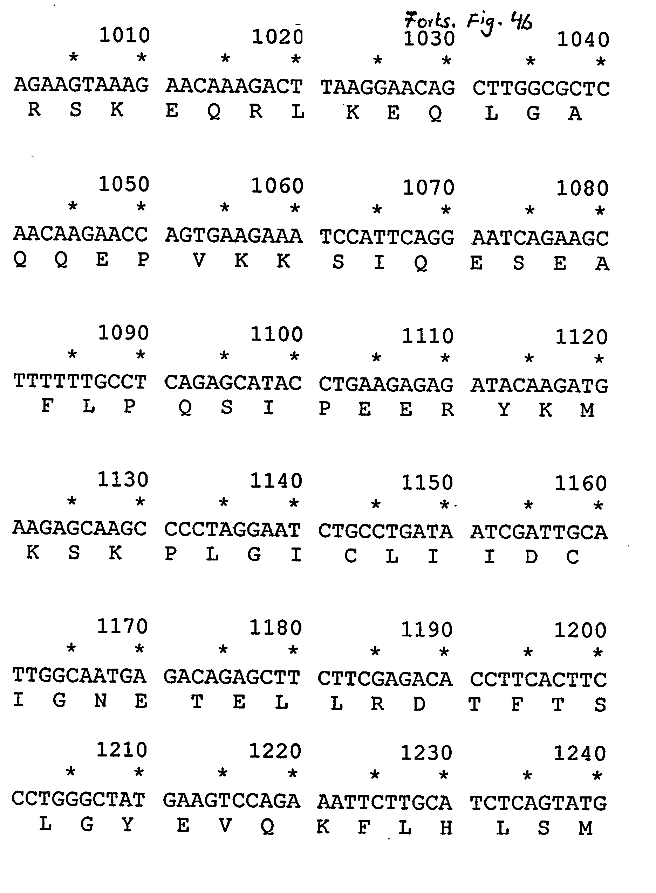

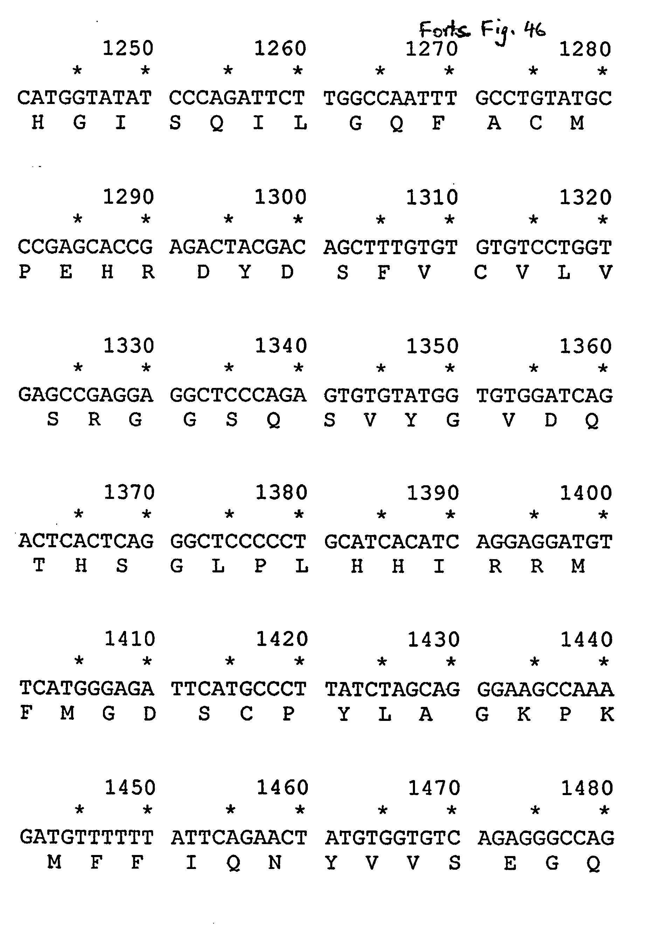

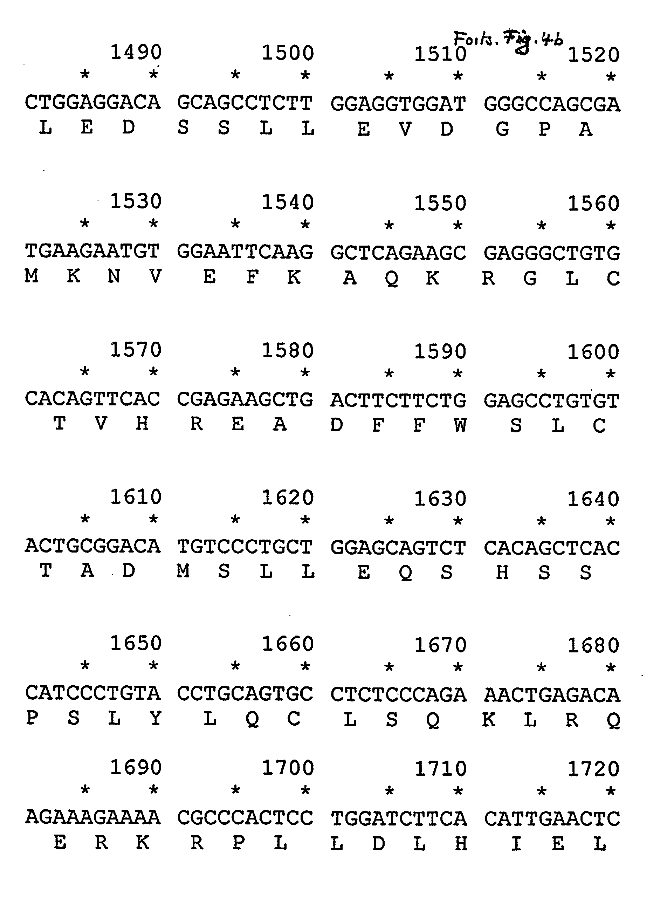

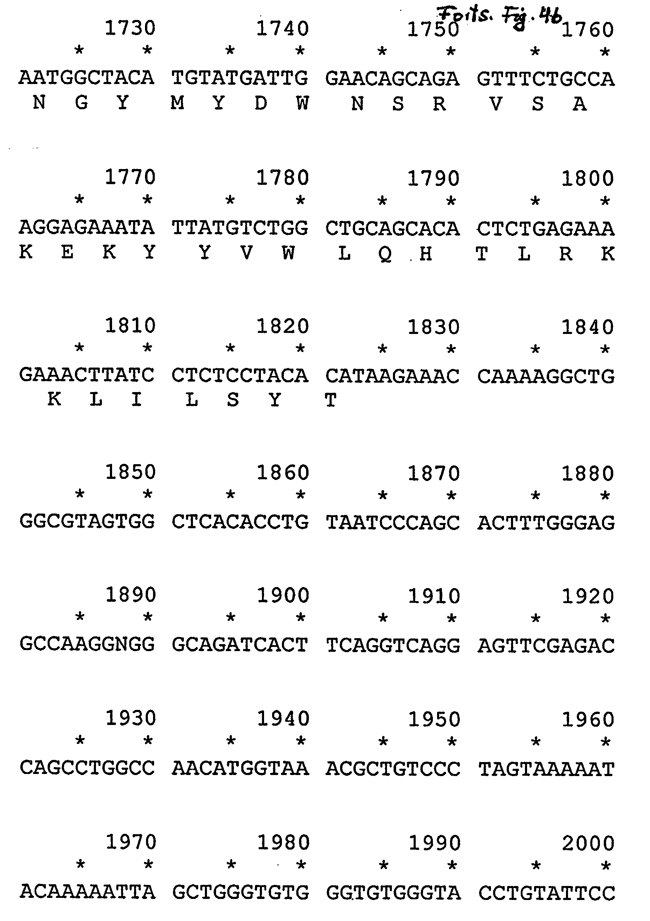

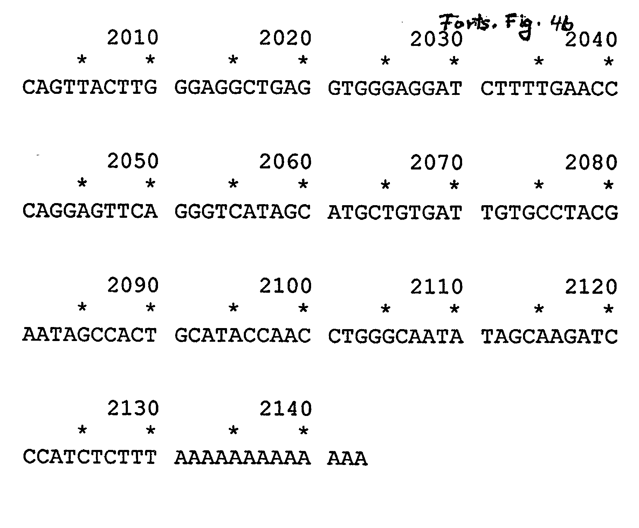

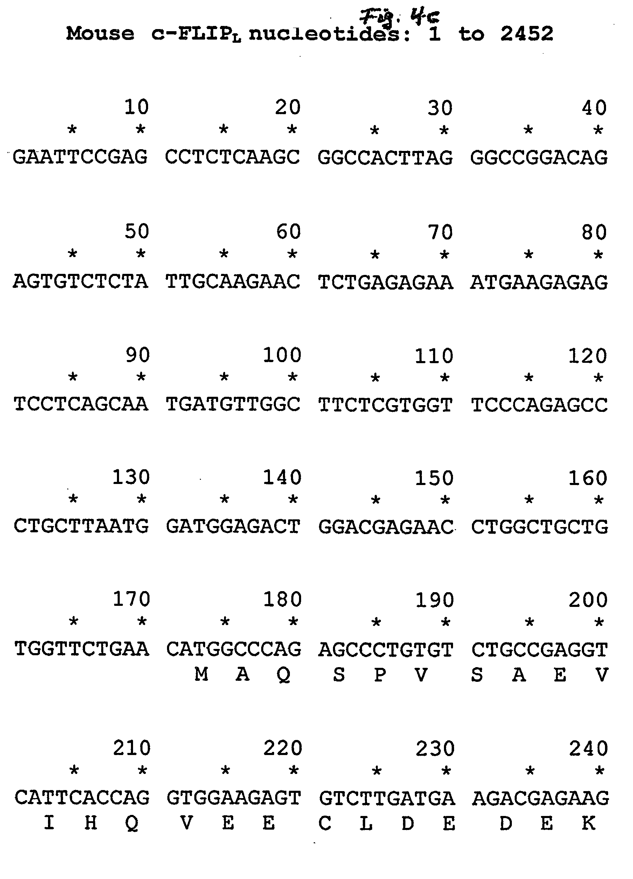

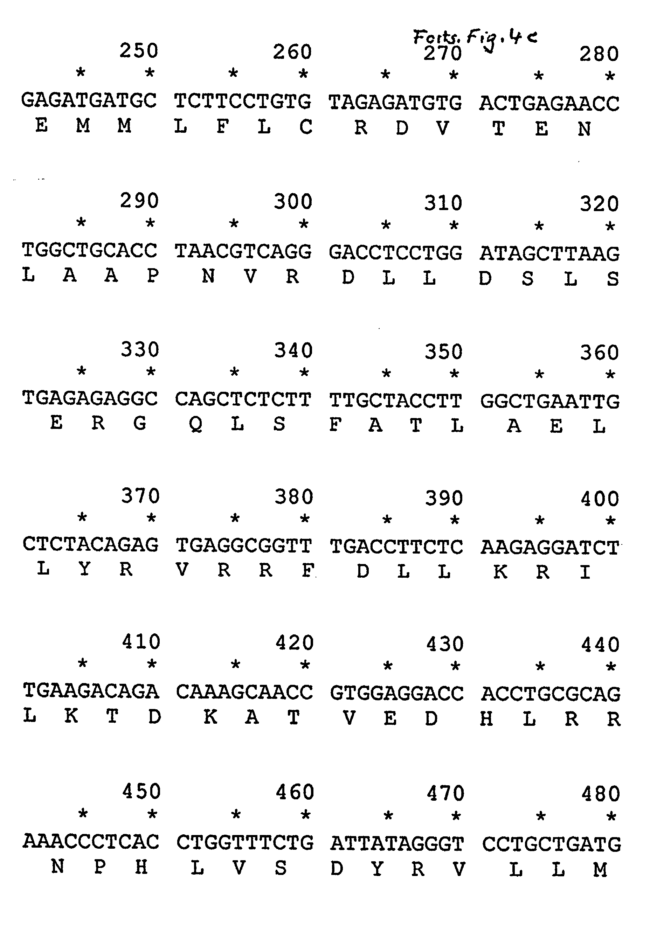

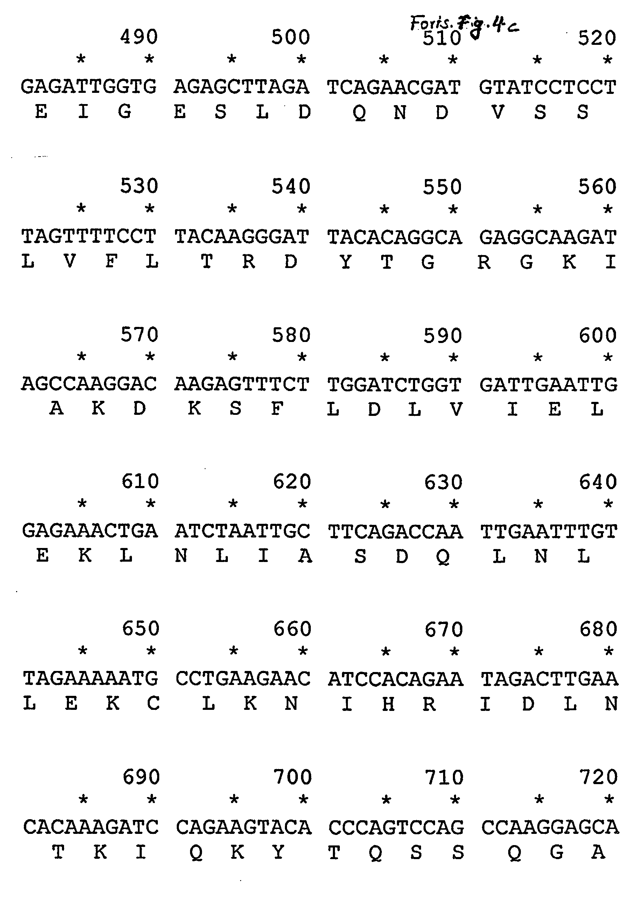

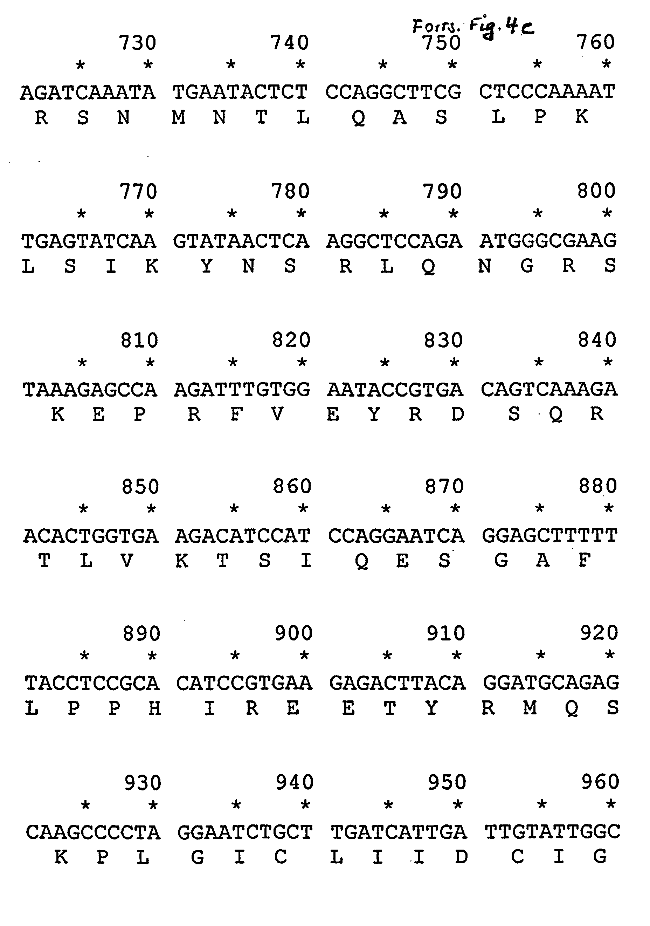

Eine DNA-Sequenz gehört insbesondere dann zum Erfindungsgegenstand, wenn deren Genprodukt eine Aminosäuresequenz aufweist oder umfaßt, wie sie in einer der Figuren 4a oder 4b oder 4c dargestellt ist. In den Figuren 4a und 4b sind die Aminosäuresequenzen sowie die dazugehörigen DNA-Sequenzen von humanen Proteinen, die zu den viralen vFLIP-Proteinen homolog sind, dargestellt. Die Sequenzen wurden ermittelt, indem cDNA Bibliotheken von aktivierten humanen peripheren Blutlymphozyten (PBL) gescreened wurden. Dabei wurden mehrere Klone identifiziert, die für ein Protein codieren, das mit dem viralen FLIP-Protein eng verwandt ist. Es weist zwei Todeseffektordomänen (DED) auf, an die sich nur eine kurze C-terminale Sequenz anschließen kann. Dieses humane Protein wird im folgenden FLIP$_S$ genannt. Eine erfindungsgemäße humane FLIP$_S$ DNA-Sequenz ist in der Figur 4a dargestellt. Sie umfaßt 1190 Nukleotide.

Eine längere Version einer humanen FLIP-DNA-Sequenz ist in Figur 4b dargestellt. Eine solche erfindungsgemäße DNA-Sequenz zur Codierung eines Proteins, im folgenden FLIP$_L$ genannt, gleichfalls aus aktivierten peripheren Blut- lymphozyten isoliert, umfaßt 2143 Nukleotide. Bei FLIP$_L$ und FLIP$_S$ handelt es sich um alternative SPLICE-Varianten der erfindungsgemäßen DNA-Sequenz. Erfindungsgemäß unterfallen alle SPLICE-Varianten mit den erfindungsgemäßen Merkmalen, nämlich der Codierung für ein Genprodukt, das mindestens eine Todeseffektordomäne aufweist und gleichzeitig die Zellapoptose inhibiert, unter den vorliegenden Erfindungsgedanken. Erfindungsgemäß wurde gezeigt, daß drei verschiedene RNA-Species als Transkripte der erfindungsgemäßen DNA-Sequenz, also einer DNA-Sequenz mit mindestens einer, vorzugsweise zwei Todeseffektordomänen mit Apoptose-Inhibitionsaktivität, existieren. Das kürzeste RNA Transkript (1,1 bis 1,3 kB) wird insbesondere im Muskelgewebe und in den peripheren Blutlymphozyten stark exprimiert. Dieses kürzeste Transkript enthält keine caspase-homologe Domäne, wie sie bei der längeren Version auftritt. Darüber hinaus wurden zwei längere Transcripte detektiert (2,2 kB bzw. 3,8 kB), die beide eine caspase-homologe Domäne aufweisen. Verglichen mit der Zahl der kürzeren FLIP$_S$-Transkripte wird in der Zelle eine geringere Menge an längeren FLIP$_L$ Transkripten synthetisiert. Sie sind im wesentlichen auch auf Muskel- und Lymphgewebe, vor allen Dingen auf Milz und Dünndarm, bzw. auf periphere Blut- lymphozyten beschränkt. Geringe Mengen an Transkripten können aber auch in anderen Geweben detektiert werden.

In Figur 4c ist darüber hinaus erfindungsgemäß eine Mäuse-DNA-Sequenz, eine lange Version mit einer caspase-homologen Domäne, dargestellt. Ebenso ist auch die dazugehörige Aminosäuresequenz in Figur 4c offenbart. Die DNA-Sequenz dieses zellulären FLIP-Proteins hat 2452 Nukleotide. Das Mäusegen wurde in einer cDNA-Bibliothek von Mäuseherzenzellen mit Hilfe der humanen cDNA-Proben identifiziert.

Im Sinne der vorliegenden Erfindung sind solche DNA-Sequenzen aus zellulärer DNA von Eukaryoten FLIP$_L$-Sequenzen, die neben mindestens einer Todeseffektordomäne auch eine caspase-homologe Domäne besitzen. FLIP$_S$-Sequenzen weisen dagegen nur mindestens eine Todeseffektordomäne auf.

In einer Ausführungsform der vorliegenden Erfindung wird eine DNA-Sequenz beansprucht, deren Genprodukt zwei Todeseffektordomänen und eine caspase-homologe Domäne aufweist, wobei die caspase-homologe Domäne infunktionell ist. Unter caspase-homologer Domäne wird eine Domäne der Proteasen von Caspasetyp (d.h. vom Typ der Cystein-Proteasen) verstanden, wobei diese homologe Domäne den Caspasen ihre proteolytische Aktivität verleiht. Eine caspase-homologe Domäne, die infunktionell ist, zeichnet sich dadurch aus, daß sie keine Cystein-proteolytische Aktivität mehr aufweist. Ein solcher Funktionsverlust kann auf einer sequenzspezifischen Mutation beruhen. Bei den besonders bevorzugten DNA-Sequenzen für Genprodukte der langen zellulären FLIP-Proteine ist der für die Proteolyseaktivität kritische Cysteinrest im aktiven Zentrum, der z.B. dem FLICE-Protein seine proteolytische Aktivität verleiht, mutiert.

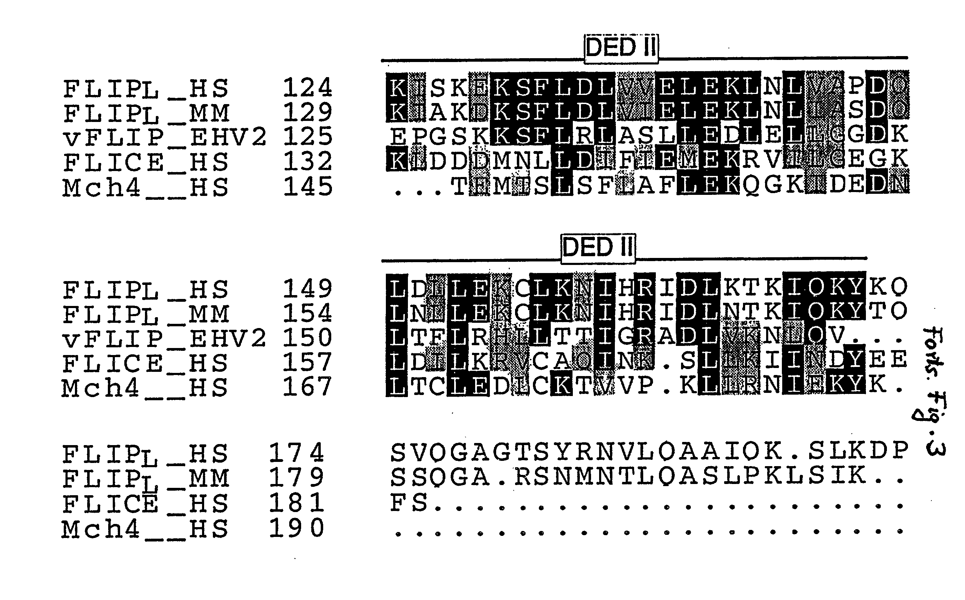

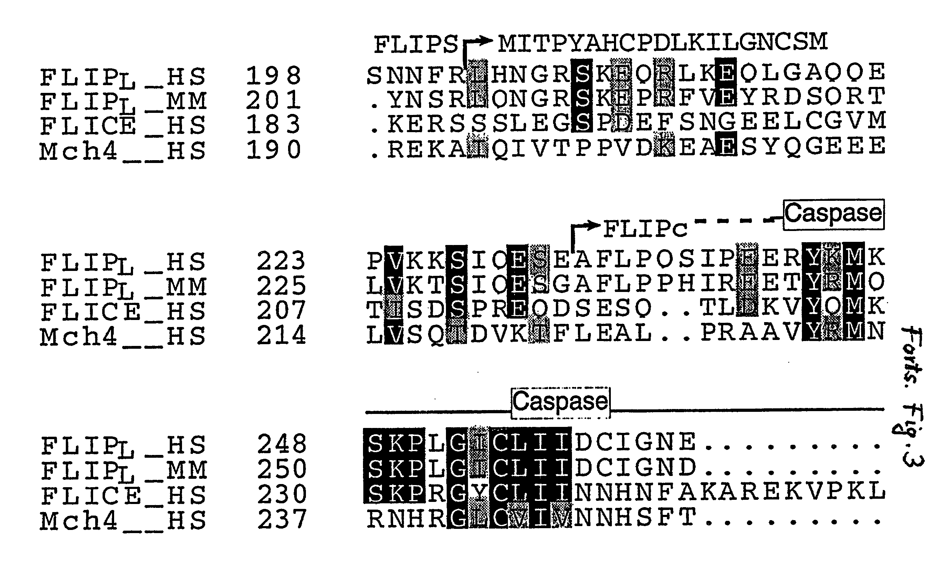

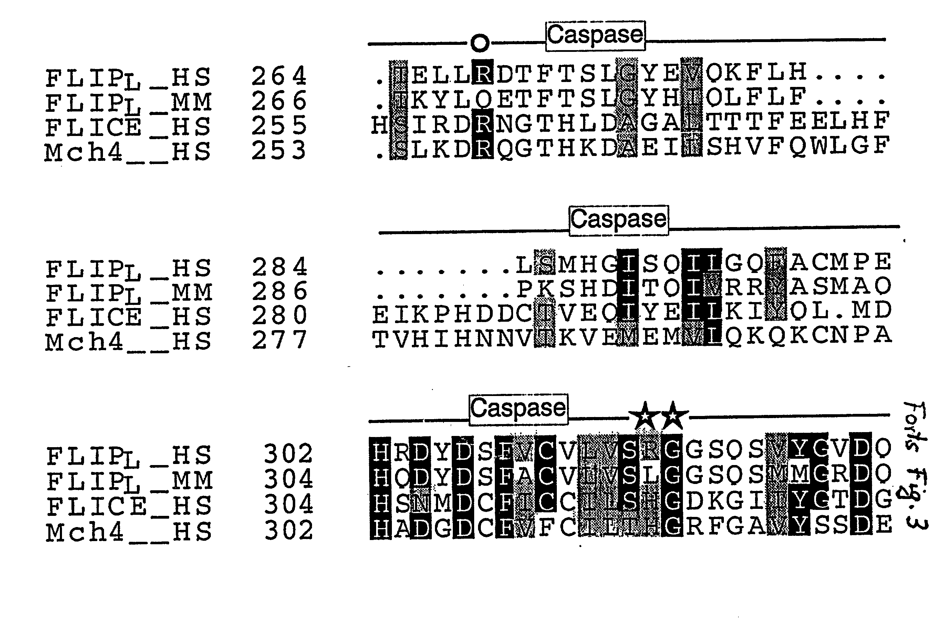

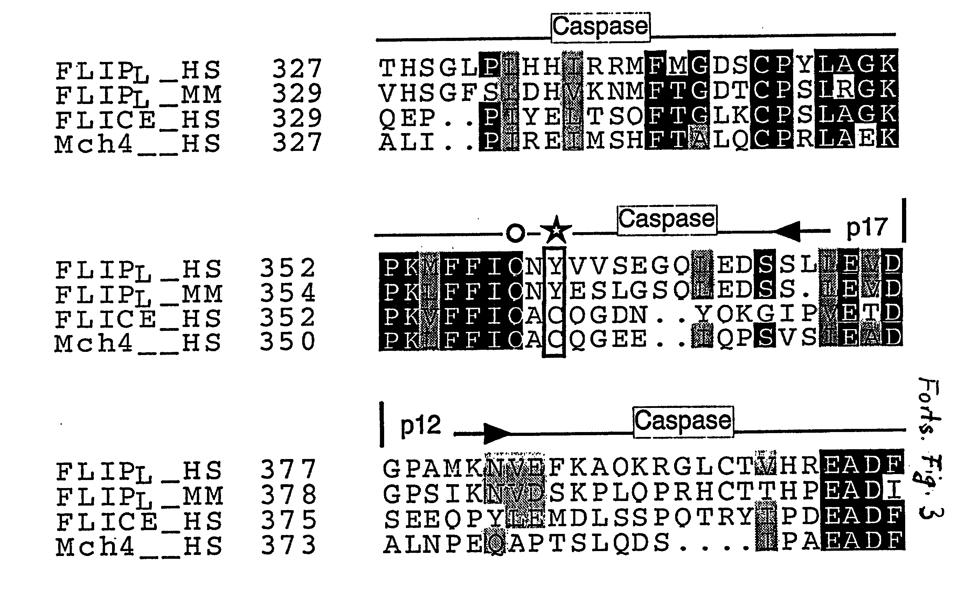

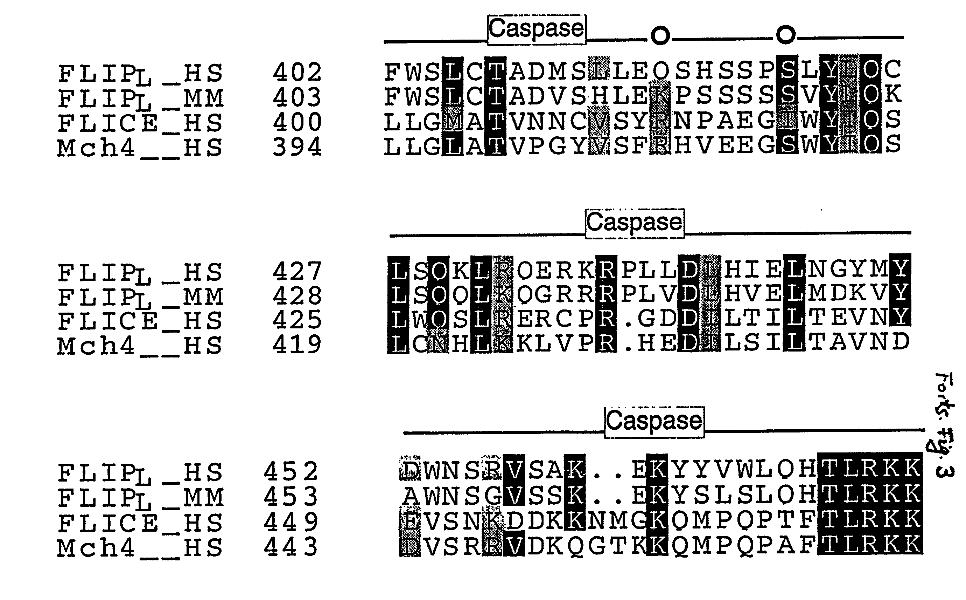

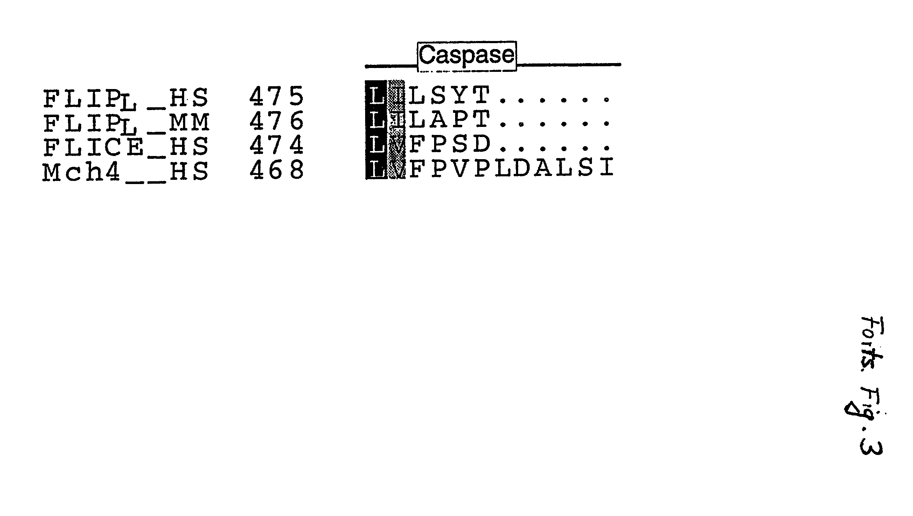

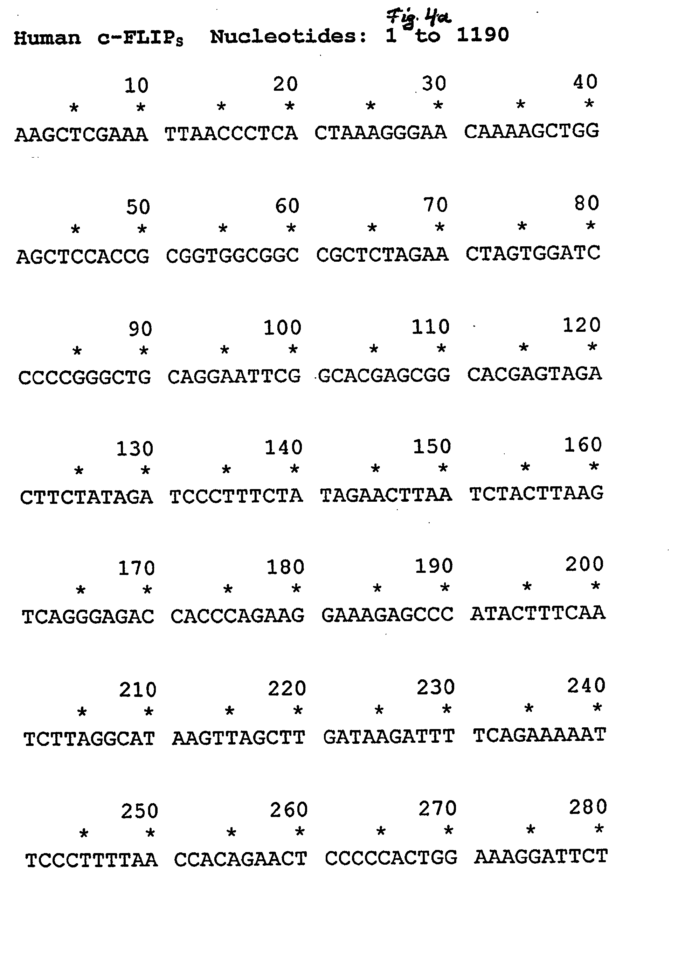

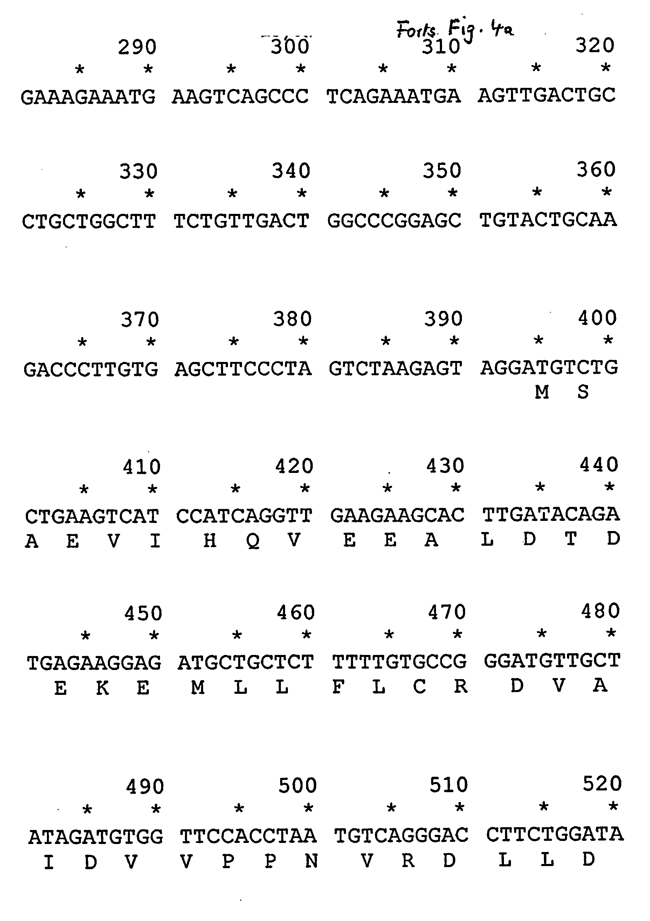

Aus der Figur 3 ist zu entnehmen, daß bei den vorliegenden Sequenzen der Cysteinrest gegen einen Tyrosinrest substituiert ist. Darüber hinaus treten charakteristische Mutationen im Vergleich zu den funktionellen Proteasedomänen von z.B. FLICE an anderen Stellen der caspase-homologen Domäne beim langen humanen FLIP-Protein auf. Zu nennen sind hier Mutationen an der Position -1, bezogen auf den kritischen Cysteinrest, und an den Positionen +1 und +2, gleichfalls bezogen auf den kritischen Cysteinrest. Der kritische Cysteinrest, in Figur 3 durch einen Stern angedeutet, ist auch in der Sequenz des Mäuse-FLIP$_L$-Proteins mutiert. Auch in diesem Fall kann die caspase-homologe Domäne also keine durch den Cysteinrest bedingte proteolytische Funktion ausüben. Unter Infunktionalität einer caspase-homologen Domäne wird im Sinn der vorliegenden Erfindung aber nur der Verlust der durch den kritischen Cysteinrest bedingten proteolytischen Aktivität verstanden. Gleichwohl kann die caspase-homologe Domäne jedoch eine andere Funktionalität besitzen. Auch solche caspase-homologen Domänen sind nach dem Sinn dieser Erfindung infunktionell.

Die beiden Todeseffektordomänen der humanen bzw. Mäuse-FLIP-Proteine interagieren ebenfalls mit Adaptorproteinen, z.B. FADD. Sowohl die lange Proteinversion (55 kD) als auch die kurze Form (34 kD) assoziieren stark mit dem FADD-Protein im Falle einer Coexpression. Dagegen zeigt der caspase-homologe Sequenzabschnitt des langen FLIP-Proteins keine Bindungsaffinität an FADD. Bevorzugt erfolgt also die Bindung eines in Säugetierzellen identifizierten FLIP-Proteins mit einer erfindungsgemäßen DNA-Sequenz an ein anderes Protein, unabhängig von der Anwesenheit der caspase-homologen Domäne, über mindestens eine Todeseffektordomäne. Vorzugsweise wird diese Bindung über zwei Todeseffektordomänen ermöglicht. Entsprechende Experimente belegen auch, daß die Proteine der erfindungsgemäßen DNA-Sequenzen aus Eukaryotenzellen, insbesondere aus Säugetierzellen bzw. humanen Zellen auch einen stabilen Tri-Komplex mit Fas/FADD bilden können. Auch die Genprodukte dieser erfindungsgemäßen zellulären DNA-Sequenzen lagern sich in den DISC-Komplex im Zuge der Aktivierung der apoptotischen Signalkaskade an den membranständigen Todesrezeptoren an. Sobald FADD an FLIP und Fas gebunden ist, werden große, auch in SDS unlösliche Aggregate beobachtet.

Ohne an eine wissenschaftliche Theorie gebunden zu sein, ist festzustellen, daß die Caspasen im Zuge der Signalweiterleitung als Dimer zunächst aktiviert und dann durch Autokatalyse prozessiert werden, wobei sich schließlich ein stabiler Komplex der Form (p10/p20)$_2$ bildet. Dagegen zeigen die durch die erfindungsgemäßen DNA-Sequenzen codierten langen FLIP-Proteine aus Eukaryotenzellen, insbesondere Säugetier- oder humanen Zellen, keine Dimer- oder Oligomerbildung. Vielmehr interagieren die FLIP-Proteine mit den FLICE-Proteinen derart, daß Heterodimere oder Heterooligomere zwischen dem aktiven FLICE und dem FLIP$_L$-Protein ohne Cysteinproteolyse-Aktivität entstehen. Somit ist ein weiterer Aspekt der vorliegenden Erfindung die Verwendung von FLIP-Proteinen mit infunktioneller, caspase-homologer Domäne und mindestens einer Todeseffektordomäne zur Bindung an Proteasen, insbesondere an Cysteinproteasen, die durch ihre Todeseffektordomänen eine Funktion als Element der apoptotischen Signalkaskade besitzen. Diese Bindung kann dann die apoptotische Signalweitergabe blockieren.

Folgender detaillierter Mechanismus liegt der Inhibition der Signalkaskade zugrunde: das FLICE-Protein wird durch das am Todesrezeptor ausgelöste Signal zur Apoptose aktiviert, gegebenenfalls durch Homodimer oder Homooligomerbildung. Im Zuge der Aktivierung kann nun entweder eine autokatalytische Spaltung an der p10/p20-Schnittstelle in dem Sequenzabschnitt der Caspase-Domäne erfolgen oder aber zunächst das FLIP-Protein gebunden werden. In jedem Fall kommt es nach der Bindung des FLIP-Proteins mit der infunktionellen caspase-homologen Domäne zu einer Proteolyse des FLIP-Proteins durch die proteolytische Aktivität eines FLICE-Proteins im FLICE/FLIP Heterodimer oder Heterooligomer. Auf bestimmte, die Apoptose auslösende Situationen, z.B. die Hyperexpression des CD95-Rezeptors, kann es dann zu einer weiteren proteolytischen Zerlegung (Processing) des FLICE-Proteins kommen. Dabei nimmt das FLICE-Protein eine Form an, die dem kleinen zellulären FLIP-Protein (FLIP$_S$) weitgehend entspricht. Es kommt daher zu einer proteolytischen Spaltung ungefähr an der Verbindungstelle zwischen der C-terminalen Todeseffektordomäne und der Caspase-Domäne des FLICE-Proteins.

In den Ausführungsbeispielen wird, wie auch für das virale FLIP-Protein, für die zellulären Formen des durch die erfindungsgemäßen DNA-Sequenzen codierten FLIP-Proteins (human oder von Mäusen, langes oder kurzes FLIP-Protein), gezeigt, daß sie auf zahlreiche, durch oder über die Todesrezeptoren ausgelösten Apoptosesignale inhibierend wirken.

Ein wesentlicher Aspekt für alle erfindungsgemäßen DNA-Sequenzen ist somit ihre Verwendung zur Immortalisierung von Zellen. Diese Immortalisierung kann in vitro oder in vivo erfolgen; sie kann alle denkbaren Zelltypen, die einer apoptotischen Reaktion unterliegen können, betreffen. In vivo-Verfahren schließen alle dem Fachmann geläufigen gentherapeutischen Methoden ein.

In einer Ausführungsform der vorliegenden Erfindung ist eine erfindungsgemäße DNA-Sequenz operabel mit einem Promotor verbunden. In dieser Ausführungsform ist also ein, vorzugsweise stromaufwärts angeordneter Promotor mit einer erfindungsgemäßen DNA-Sequenz verbunden. Dieser Promotor kann, abhängig vom experimentellen Ziel, ein prokaryotischer oder eukaryotischer Promotor sein.

Promotoren, die in prokaryotischen Wirtszellen eingesetzt werden, sind etwa der β-Lactamase- oder der Lactose-Promotor, ferner das Tryptophan-Promotorsystem oder auch Hybrid-Promotoren wie etwa der tac-Promotor. Je nach Wahl der bakteriellen Wirtszelle werden die geeigneten Promotoren vom Fachmann gewählt. Ihre Nukleotidsequenzen sind veröffentlicht, und durch Angaben in der Literatur ist der Fachmann in der Lage, die Promotoren an die erfindungsgemäßen DNA-Sequenzen anzuhängen (Siebenlist et al., Cell, 20: 269, 1980). Promotoren in bakteriellen Systemen werden im allgemeinen auch eine Shine-Dalgarno (SD) Sequenz enthalten.

Geeignete Promotorsequenzen in Hefewirtszellen sind z.B. der 3-Phosphoglycerat-Kinase-Promotor oder Promotoren anderer glykolytischer Enzyme (als Beispiele seien genannt: Enolase, Glycerinaldehyd-3-Phosphat-D-Hydrokinase, Hexokinase, Pyruvatdecarboxylase, Phosphofructokenase und andere).

Die Transkription einer erfindungsgemäßen DNA-Sequenz in Zellen höherer Eukaryoten, insbesondere Säugetierzellen, wird von Promotoren kontrolliert, die aus verschiedenen nativen Systemen stammen können. Es bietet sich beispielsweise an, Promotoren aus den Genomen von Viren zu verwenden. Zu nennen wären beispielsweise Polyoma-Viren, SV40, Adenoviren, Retroviren, Hepatitis-B-Viren, Cytomegaloviren und ähnliche. Aus Säugetierzellen kommt z.B. der β-Aktin-Promotor in Frage. Insbesondere bevorzugt ist in der vorliegenden Erfindung der SRα-Promotor. Als Promotor der Cytomegaloviren kommt insbesondere auch der frühe Promotor des humanen Cytomegalovirus in Frage, der eine HindIII-E Restriktionsschnittstelle enthält (Greenaway et al., Gene, 18: 355-360 (1982)). Selbstverständlich kommen auch Promotoren der jeweiligen Säugetier- oder humanen Wirtszellen in Frage.

In einer besonders bevorzugten Ausführungsform sind weitere Steuerungselemente für die Transkription und/oder Translation an die erfindungsgemäße DNA-Sequenz angefügt. So etwa wird die Transkription einer erfindungsgemäßen DNA-Sequenz bei höheren Eukaryoten entscheidend erhöht, wenn ein Enhancer-Element im Expressionsvektor vorhanden ist. Enhancer sind cis-wirkende Elemente der DNA, die gewöhnlich 10 bis 300 Basenpaare umfassen und auf den Promotor zur Erhöhung der Transkriptionsrate einwirken. Sie können in 3'- oder 5'-Position der erfindungsgemäßen DNA-Sequenz angeordnet sein, aber auch in der codierenden Sequenz selbst oder in einem Intron, das erst durch SPLICE-Vorgänge herausgeschnitten wird, vorhanden sein. Typischerweise wählt man einen Enhancer aus einem Virus eukaryotischer Zellen. Beispiele wären der SV40-Enhancer oder der Enhancer des frühen Promotors des Cytomegalovirus. In Frage kommen aber auch Enhancer von Adenoviren. Natürlich sind zahlreiche Enhancer auch aus Genen von Säugern bekannt (z.B. Globin, Elastase oder Albumin). Die Enhancer sind typischerweise an 5'- oder 3'-Position des codierenden Bereichs einer erfindungsgemäßen DNA-Sequenz in den Expressionsvektor integriert. Vorzugsweise liegt der Enhancer 5' zum Promotor. Weitere Steuerungselemente können zur Regulation der Termination der Transkription dienen, so daß die Expression der mRNA betroffen ist.

Ein weiterer Aspekt der vorliegenden Erfindung sind Expressionsvektoren, die eine erfindungsgemäße DNA-Sequenz, typischerweise mit einem Promotor und gegebenenfalls mit weiteren der oben beschriebenen Steuerungselemente der Transkription und/oder Translation enthalten. Sie dienen dazu, daß die erfindungsgemäße Nukleotidsequenz in spezifischen Wirtszellen vermehrt und exprimiert werden kann. Im allgemeinen werden Expressionsvektoren verwendet, die sich unabhängig vom Wirtschromosom autonom replizieren können. Sie besitzen einen eigenen "origin of replication". Solche Sequenzen sind in Bakterien, in Hefe oder in Viren vorhanden. Dagegen sind die Origins in Expressionsvektoren von Säugern nicht erforderlich.

Gegebenenfalls werden die Expressionsvektoren mit der erfindungsgemäßen DNA-Sequenz als sogenannte "Shuttle"- Vektoren gestaltet, d.h., sie können sich in einem Wirtssystem zunächst replizieren und können dann in ein anderes Wirtssystem zum Zweck der Expression transfiziert werden. Zum Beispiel kann ein Vektor zunächst in E.coli kloniert und dann in eine Hefe- oder Säugetierzelle zur Expression gebracht werden. In einem solchen Fall ist es dann nicht mehr zwingend notwendig, daß die Replikation unabhängig von der Replikation des Wirtszellchromosoms möglich ist.

Besonders bevorzugt ist der Expressionsvektor pCR-3 bzw. alle Derivate dieses Vektors, der eine EcoR1-Schnittstelle aufweist. Bevorzugterweise wird eine erfindungsgemäße DNA-Sequenz dann in einen weiteren Vektor, der die stabile Expression z.B. in Jurkat- und Raji-Zellen erlaubt, eingebracht. Typischerweise haben solche Expressions- und Klonierungsvektoren dann ein Selektionsgen, das eine Markerfunktion ausübt. Es handelt sich dabei um ein Gen, das für ein Protein codiert, das das Überleben oder das Wachstum solcher Wirtszellen gestattet, die mit dem Vektor transformiert wurden. Typische Selektionsgene codieren für Proteine, die eine Resistenz gegenüber Antibiotika oder anderen Toxinen zulassen. Zu nennen wären beispielsweise Puromycin oder Ampicillin oder Neomycin. Im Rahmen dieser Erfindung sind Puromycin-Resistenzen zur Selektion der transformierten Wirtszellen besonders bevorzugt.

Ein weiterer Aspekt der vorliegenden Erfindung ist ein Verfahren zur Isolierung von Genprodukten mit mindestens einer Todeseffektordomäne, wobei die Wirtszellen mit einem erfindungsgemäßen Expressionsvektor transformiert und dann unter geeigneten, die Expression fördernden Bedingungen kultiviert werden, so daß das Genprodukt schließlich aus der Kultur aufgereinigt werden kann. Das Protein der erfindungsgemäßen DNA-Sequenz kann dabei, abhängig von dem Expressionssystem, aus einem Kulturmedium oder aus Zellextrakten isoliert werden. Der Fachmann kann ohne weiteres erkennen, daß die jeweiligen Isolierungsmethoden und das Verfahren bei der Aufreinigung des von einer erfindungsgemäßen DNA codierten, rekombinanten Proteins stark vom Typ der Wirtszelle oder auch von dem Umstand, ob das Protein in das Medium sekretiert wird, abhängt. Zum Beispiel können Expressionssysteme eingesetzt werden, die zur Sekretion des rekombinanten Proteins führen. Das Kulturmedium muß in diesem Fall durch kommerziell erhältliche Proteinkonzentrationsfilter, z.B. Amicon oder Millipore Pelicon, aufkonzentriert werden. Nach dem Konzentrationsschritt kann ein Reinigungsschritt erfolgen, z.B. ein Gelfiltrationsschritt. Alternativ kann aber auch ein Anionenaustauscher eingesetzt werden, der eine Matrix mit DEAE aufweist.

Als Matrix dienen dabei alle aus der Proteinreinigung bekannten Materialien, z.B. Acrylamid oder Aggarose oder Dextran oder ähnliches. Es kann aber auch ein Kationenaustauscher eingesetzt werden, der dann typischerweise Carboxymethyl-Gruppen enthält. Zur weiteren Reinigung eines durch eine erfindungsgemäße DNA codierten Proteins können dann HPLC-Schitte dienen. Es kann sich um einen oder mehrere Schritte handeln. Insbesondere wird die "Reversed-Phase"-Methode eingesetzt. Diese Schritte dienen zum Erhalt eines im wesentlichen homogenen rekombinanten Proteins der erfindungsgemäßen DNA-Sequenz.

Neben bakteriellen Zellkulturen zur Isolierung des Genprodukts können auch transformierte Hefezellen eingesetzt werden. In diesem Fall kann das translatierte Protein sekretiert werden, so daß die Proteinreinigung vereinfacht wird. Sekretiertes rekombinantes Protein aus einer Hefewirtszelle kann durch Methoden erhalten werden, wie sie bei Urdal et al. (J. Chromato. 296: 171 (1994)) offenbart sind.

Ein weiterer Aspekt der vorliegenden Erfindung ist ein Verfahren zur Expression von Genprodukten mit mindestens einer Todeseffektordomäne, wobei Wirtszellen mit einem Expressionsvektor, der eine erfindungsgemäße DNA-Sequenz enthält, transformiert werden. Dieses Verfahren zur Expression von Genprodukten, die auf einer erfindungsgemäßen DNA-Sequenz beruhen, dient nicht dazu, das entsprechende Genprodukt zu konzentrieren und aufzureinigen, sondern vielmehr dazu, den Zellstoffwechsel durch das Einführen der erfindungsgemäßen DNA-Sequenzen über die Expression des dazugehörigen Genprodukts zu beeinflussen. Hier ist insbesondere an die Verwendung der mit Hilfe von Expressionsvektoren transformierten Wirtszellen zum Zwecke der Immortalisierung zu denken. Durch die Verwendung eines sogenannten konstitutiven Promotors können in diesen Zellen gleichbleibende Konzentrationen von auf erfindungsgemäßen Sequenzen beruhenden Proteinen exprimiert werden. Auf diese Weise wird dauerhaft eine von oder über die Todesrezeptoren ausgelöste Apoptose verhindert. Die entsprechenenden Zellinien werden dadurch gegenüber einer Vielzahl von apoptotischen Stimulanzen resistent. Diese Zellen können auch gegebenenfalls wieder in den Säuger- oder humanen Organismus überführt werden. Auf diese Weise wird durch in vitro mit den erfindungsgemäßen Expressionsvektoren manipulierter Zellen und die anschließende Übertragung in den Organismus ein gentherapeutischer Einsatz der erfindungsgemäßen DNA-Sequenzen möglich. Dabei werden Expressionsvektoren mit den erfindungsgemäßen Sequenzen in solche Zellen transfiziert, die krankheitsbedingt durch apoptotische Einflüsse im Organismus eliminiert werden. Durch ein solches Verfahren ist eine ständige Restitution dieser Apoptose gefährdeten Zellen möglich.

Mit dem Erfindungsgedanken geht aber ein auch gentherapeutisches Verfahren, das in vivo durchgeführt werden kann, einher. Zu diesem Zwecke werden Vektoren eingesetzt (z.B. Liposomen oder Adenoviren oder Retroviren oder ähnliche), die die erfindungsgemäßen DNA-Sequenzen gezielt in jede Zellen des Organismus insertieren, die aufgrund pathologischer Einflüsse eine verstärkte Disposition zum Zelltod zeigen.

Die erfindungsgemäßen DNA-Sequenzen, deren Allele, Derivate oder Fragmente können auch im Labormaßstab als Proben verwendet werden. So etwa könnte beispielsweise die DNA-Sequenz der viralen FLIP-Gene zum Nachweis der entsprechenden Viren in in vitro Testsystemen dienen. Hierbei kämen insbesondere Anwendungen für Northern- oder Southern-Blots in Frage. Die Proben weisen eine geeignete DNA-Sequenzlänge auf, je nach experimentellem Erfordernis. Zum Beispiel könnte die einer Todeseffektordomäne des erfindungsgemäßen Typs zugrundeliegende DNA-Sequenz als Detektionsprobe eingesetzt werden.

Eine andere sinnvolle Anwendung der erfindungsgemäßen DNA-Sequenzen ist ihr Einsatz als Einzelstrang-DNA-Sequenz. Dieser ist sowohl auf RNA- als auch auf DNA-Ebene möglich. Der Einsatz sowohl von DNA oder RNA des codierenden Stranges ("sense") als auch des komplimentären Stranges "antisense") ist denkbar. Es hängt von der Wahl des Zielproteins ab, ob anti-sense- oder sense-DNA zur Anwendung kommt. Die entsprechenden Oligonukleotide umfassen dabei typischerweise zwischen 20 und 40 Nukleotide der erfindungsgemäßen DNA-Sequenz. In diesem Zusammenhang kann erwähnt werden, daß die Bindung von anti-sense- oder sense-DNA zu der Ausbildung von Duplexmolekülen führt, die entweder die Translation (auf RNA-Ebene) oder die Transkription (auf DNA-Ebene) blockieren. Auf diese Weise kann durch die erfindungsgemäßen DNA-Sequenzen die Expression von FLIP-Proteinen in der Zelle verhindert werden. Eine mögliche Anwendung dieser Technik ergäbe sich bei der Konzeption von anti-viralen Substanzen. Mit dem Einsatz von diesen Einzelstrang-Oliogonukleotiden könnten insbesondere die tumorigenen Eigenschaften der FLIP exprimierenden Viren, also insbesondere von Herpes-Viren, bekämpft werden. Die Oligonukleotide können chemisch verändert werden, indem sie durch entsprechende Modifikationen gegen den enzymatischen Abbau geschützt werden. Das Einführen des Oligonukleotid-Einzelstranges in die Zelle kann über alle bekannten Verfahren, also z.B. über Vektoren zum Gentransfer oder durch das Anlegen von elektrischen Feldern möglich werden.

Ein weiterer Gegenstand der vorliegenden Erfindung sind Wirtszellen, die mit einem erfindungsgemäßen Expressionsvektor transformiert wurden. Als geeignete Wirtszellen zur Klonierung oder Exprimierung der erfindungsgemäßen DNA-Sequenzen kommen Prokaryotenhefen oder höhere eukaryotische Zellen in Frage. Bei Prokaryoten sind Gram-negative oder Gram-positive Organismen ausdrücklich eingeschlossen. Zu nennen ist hier E.coli oder Bazillen. Als bevorzugte Wirtszellen zur Klonierung der erfindungsgemäßen DNA-Sequenzen werden die Stämme E.coli 294, E.coli B und E.coli X1776 sowie E.coli W3110 offenbart. Bei den Bazillen stehen Bazillus subtilis, Salmonella typhimurium oder ähnliche. Wie oben bereits erwähnt, enthalten die Expressionsvektoren typischerweise eine Signalsequenz zum Transport des Proteins in das Kulturmedium, so werden prokaryotische Zellen eingesetzt werden. Neben Prokaryoten kommen auch eukaryotische Mikroben als Wirtszellen, die mit dem Expressionsvektor transfiziert worden sind, in Frage. So etwa können flammentöse Pilze oder Hefen als geeignete Wirtszellen für die erfindungsgemäßen DNA-Sequenzen codierende Vektoren eingesetzt werden. Zu nennen ist vor allem Saccharomycis cirevesiae oder die gewöhnliche Bäckerhefe (Stinchcomb et al., Nature, 282: 39, (1997)).

In einer bevorzugten Ausführungsform werden jedoch zur Expression von erfindungsgemäßen DNA-Sequenzen Zellen aus multizellulären Organismen gewählt. Dies geschieht auch vor dem Hintergrund einer möglicherweise erforderlichen Glykosilierung der codierten Proteine. Diese Funktion kann in höheren Eukaryotenzellen - im Vergleich zu Prokaryotenzellen - in geeigneter Weise ausgeführt werden. Im Prinzip ist jede höhere eukaryotische Zellkultur als Wirtszelle verfügbar, wenn auch Zellen von Säugern, beispielsweise Affen, Ratten, Hamstern oder Menschen, ganz besonders bevorzugt sind. Dem Fachmann ist eine Vielzahl von etablierten Zellinien bekannt. In einer keineswegs abschließenden Aufzählung werden die folgenden Zellinien genannt: 293T (Embryonennierenzellinie), (Graham et al., J. Gen. Virol., 36:59 (1997)), BHK (Babyhamsternierenzellen), CHO (Zellen aus den Hamsterovarien), (Urlaub und Chasin, P. N. A. S. (USA) 77:4216, (1980)), HELA (humane Cervexkarzinomzellen) und weitere Zellinien.

Im Sinne der vorliegenden Erfindung sind mit Expressionsvektoren, die die erfindungsgemäßen DNA-Sequenzen aufweisen, vorzugsweise Zellen des Säugetierimmunsystems, vor allem des humanen Immunsystems, transfiziert. Ganz besonders bevorzugte Wirtszellen sind dabei T- und D-Lymphozyten.

Eine Anwendung der erfindungsgemäßen DNA-Sequenzen, die die Wirtszelle immortalisieren können, ergibt sich z.B. im Fall einer HIV-Infektion. Bei einer HIV-Infektion gehen Zellen durch apoptotische Mechanismen zugrunde. Auch nicht infizierte $CD4^+$-Zellen unterliegen dabei dem Zelltod. Zur Bekämpfung einer HIV-Infektion wäre daher die Immortalisierung nicht infizierter Immunzellen wünschenswert. Auf diese Weise könnte eine Basisfunktionalität des Immunsystems erhalten bleiben.

Ein weiterer Aspekt der vorliegenden Erfindung sind die Genprodukte der erfindungsgemäßen DNA-Sequenzen. Unter Genprodukten versteht man im Sinne dieser Erfindung sowohl Primärtranskripte, also RNA, vorzugsweise mRNA, als auch Proteine. Diese Proteine weisen erfindungsgemäß mindestens eine Todeseffektordomäne auf und inhibieren die Zellapoptose. Zu diesen Proteinen gehören alle erfindungsgemäß codierten Proteine, d.h. auch die von DNA-Derivaten, DNA-Fragmenten oder DNA-Allelen exprimierten Proteine. Darüber hinaus können die Proteine chemisch modifiziert sein. So etwa kann eine Schutzgruppe am N-Terminus vorliegen. Es können Glykosylgruppen an Hydroxyl oder Aminogruppen angefügt sein, Lipide können covalent mit dem erfindungsgemäßen Protein verbunden sein, ebenso Phosphate oder Acetylgruppen und ähnliches. Auch beliebige chemische Substanzen, Verbindungen oder Gruppen können auf einem beliebigen Syntheseweg an das erfindungsgemäße Protein gebunden sein. Auch zusätzliche Aminosäuren, z.B. in Form einzelner Aminosäuren oder in Form von Peptiden oder in Form von Proteindomänen und ähnliches, können mit dem N- und/oder C-Terminus fusioniert sein. Insbesondere sind hier sogenannte Signal- oder "Leader"-Sequenzen am N-Terminus der erfindungsgemäßen Aminosäuresequenz vorliegen, die das Peptid cotranslational oder posttranslational in eine bestimmte Zellorganelle oder in den exzellulären Raum (bzw. das Kulturmedium) führen. Am N- oder am C-Terminus können auch Aminosäuresequenzen vorliegen, die als Antigen die Bindung der erfindungsgemäßen Aminosäuresequenz an Antikörper erlauben. Zu nennen ist hier insbesondere das Flag-Peptid, dessen Sequenz im Einbuchstabencode der Aminosäuren lautet: DYKDDDDK. Diese Sequenz hat stark antigene Eigenschaften und erlaubt somit eine schnelle Überprüfung und leichte Reinigung des rekombinanten Proteins. Monoklonale Antikörper, die das Flag-Peptid binden, sind von der Firma Eastman Kodak Co., Scientific Imaging Systems Division, New Haven, Connecticut erhältlich. Die erfindungsgemäßen DNA-Sequenzen können auch in zahlreichen Exons, die durch Introns voneinander getrennt sind, auf dem Strang des Erbinformationsmoleküls abgelegt sein. Damit gehören auch alle denkbaren SPLICE-Varianten (auf mRNA-Ebene) als Genprodukte zum erfindungsgemäßen Gegenstand. Auch die von diesen verschiedenen SPLICE-Varianten codierten Proteine unterfallen dieser Erfindung.

Neben den covalenten Modifikationen des Proteins gehören auch Proteinaggregate, z.B. Dimere der erfindungsgemäßen Proteine oder höhere Aggregate, in den Bereich der vorliegenden Erfindung.

In einer Ausführungsform der vorliegenden Erfindung weist das Protein als erfindungsgemäßes Genprodukt ein Signifikanzniveau von $p < 10^{-2}$ auf, wenn mit einem Suchprofil nach Figur 1a oder Figur 1b verglichen wird. Dabei wird, wie oben bereits beschrieben, ein Suchprofil angelegt, das etwaige Todeseffektordomänen in einer Datenbank identifizieren kann. Zwei Suchprofile stehen dabei zur Auswahl. Dabei kann sowohl die Datenbank mit dem Suchprofil als auch das Suchprofil mit der Datenbank verglichen werden. Erfindungsgemäß gehören alle Genprodukte oder Abschnitte dieser Genprodukte in einer Ausführungsform zum Gegenstand der Erfindung, wenn ein Primärtranskript (RNA) durch einen der Ansprüch 1 bis 24 codiert wird.

In einer bevorzugten Ausführungsform haben die erfindungsgemäßen FLIP-Proteine eine Aminosäuresequenz nach einer der Figuren 4a, 4b oder 4c (für die zellulären Proteine) oder eine Aminosäuresequenz nach Figur 16 oder nach Figur 17 oder nach den "Gen-Bank" Zugangscodes U60315 (MCV 159L), U60315 (MCV 160L), U20824 (E8) oder X64346 (ORF 71).

Zahlreiche Verwendungen werden für ein erfindungsgemäßes Protein offenbart. So kann das gereinigte erfindungsgemäße Protein nach der vorliegenden Verwendung an Patienten, insbesondere an humane Patienten bei Vorliegen einer entsprechenden pathologischen Indikation, verabreicht werden. Dabei kann das erfindungsgemäße Protein in beliebigen Kombinationen mit anderen Proteinen oder mit pharmakologischen Zusatzstoffen oder weiteren Trägermaterialien oder Cotherapeutika galenisch aufbereitet werden. Das Protein kann zur Untersuchung zellphysiologischer Vorgänge eingesetzt werden. Dabei sind Bindungsstudien mit anderen Proteinen möglich. Im Rahmen solcher Bindungsstudien kann auch die biologische Aktivität der erfindungsgemäßen Proteine getestet werden. Denkbar wäre also etwa eine Bindungsstudie der erfindungsgemäßen Proteine an Adaptorproteine, wie z.B. FADD. Des weiteren ist auch an eine Verwendung der erfindungsgemäßen Proteine im Rahmen von Proteinreinigungsvorgängen zu denken. Ein erfindungsgemäßes Protein kann dabei an ein Trägermaterial gebunden sein, z.B. auf einer Säule, wobei dieser Einsatz zur Bindung und Isolierung etwaiger physiologisch mit dem erfindungsgemäßen Protein interagierender Zellproteine dient.

Die Erfindung wird durch die nachfolgenden

Figuren näher erläutert

In Figur 1 werden zwei Suchprofile für die Identifizierung von Todeseffektordomänen beschrieben. Die Aminosäuren werden dabei, entsprechend ihrem Ein-Buchstaben-Code, alphabetisch geordnet (z.B. Alanin: A, an der ersten Position). Eine solche systematische Reihung der Aminosäuren erfolgt für jede der Sequenzpositionen. Die Sequenzpositionen sind durch "/M:" markiert. Darauf folgt eine Angabe, welche Aminosäure an dieser Sequenzposition die größte Wahrscheinlichkeit hat (SY = 'P' für die erste Sequenzposition des Profils nach Figur 1a), in einer homologen Sequenz aufzutreten. Dann folgt nach "M =" eine systematisch-alphabetische Reihung der Aminosäuren mit einer profilgemäßen Gewichtung der Wahrscheinlichkeit für jede Aminosäure an der spezifizierten Sequenzposition. Je negativer der Zahlenwert für eine Aminosäure, desto geringer ist die Wahrscheinlichkeit (bei einem Protein homologer Funktion) an dieser Sequenzposition diese Aminosäure zu finden. Auf diese Weise ist eine Matrix für alle Aminosäuren an allen Sequenzpositionen aufgebaut (http://expasy.hcuge.ch/txt/profile.txt).

In Figur 1a ist das Suchprofil, das mit Hilfe der Sequenzen für FADD Mch4 und FLICE aufgestellt wurde, dargestellt. Es umfaßt zwei Seiten.

In Figur 1b ist das Suchprofil, das aus dem Protein FADD, Mch4, FLICE und den viralen FLIP-Proteinen aufgestellt wurde, dargestellt. Es diente zur Identifikation der murinen und humanen FLIP-Sequenzen. Auch dieses Suchprofil umfaßt zwei Seiten.

In Figur 2 werden die Aminosäuresequenzen von seinerzeit bekannten Proteinen mit Todeseffektordomänen (FADD, FLICE und Mch4) verglichen mit den über eine Profilsuche gefundenen Aminosäuresequenzen von viralen Proteinen aus den Viren EHV-2 (equines Herpesvirus 2), HHV-8 (humanes Herpesvirus 8), HVS (Herpesvirus Saimiri), BHV-4 (bovines Herpesvirus 4) und MCV (Molluscum Contagiosum Virus). Der Sequenzvergleich zeigt die Homologie im Bereich der ersten und der zweiten Todeseffektordomäne (engl. Death Effector Domain, DED1 bzw. DED2), während nur eine geringe Homologie in dem mindestens 16 Aminosäuren umfassenden Verbindungsteil zwischen der ersten und der zweiten Todeseffektordomäne. In dem gezeigten Sequenzvergleich entspricht eine schwarze Unterlegung des Aminosäurekurzzeichens (Ein-Buchstaben-Code der Aminosäuren, beschrieben in Stryer, Biochemistry (1995)) mindestens 50% Sequenzidentität innerhalb der verglichenen Sequenzen, während eine graue Unterlegung des Aminosäurekurzzeichens mindestens 50%iger Sequenzähnlichkeit durch konservative Aminosäuresubstitution symbolisiert. Die Pfeile am C-Terminus der zweiten Todeseffektordomäne der MCV 159L und MCV 160L Sequenzen beziehen sich auf deren C-terminale Extensionen von 66 (ORF 159L) und 202 (ORF 160L) Aminosäuren Länge. Die Aminosäuresequenzen der viralen FLIPs sind deponiert in der Datenbank GenBank mit den Zugangsnummern U20824 (ORF E8 in EHV-2), X64346 (ORF 71 in HVS) und U60315 (ORF 159L und ORF 160L in MCV). Die Aminosäuresequenzen der viralen FLIPs aus HHV-8 (ORF 71) und aus BHV-4 sind in der Figur 16 (ORF 71 aus HHV-8) und in der Figur 17 (v-FLIP des BHV-4) gezeigt. Eine für die Aminosäuresequenz der viralen FLIPs kodierende DNA-Sequenz ist deponiert in der Datenbank GenBank mit der Zugangsnummer U20824 (ORF E8 in EHV-2), Z46385 (BHV-4), X64346 (ORF 71 in HVS), U90534 (ORF 71 in HHV-8) und U60315 (ORF 159L und ORF 160L in MCV).

Figur 3 zeigt die Homologie der Aminosäuresequenzen der humanen (HS) und der murinen (MM) Form der langen (FLIP$_L$) und der kurzen (FLIP$_S$) Version von FLIP mit den Aminosäuresequenzen von FLICE und Mch4. Die N-terminalen 202 Aminosäuren der kürzeren Splicing-Variante des humanen FLIP (FLIP$_S$) sind mit der Sequenz der längeren Form des humanen FLIP (FLIP$_L$) identisch, die Sequenz des kürzeren Proteins (FLIP$_S$) endet nach einer C-terminalen Extension von weiteren 19 Aminosäuren (nur diese Extension ist für das humane FLIP$_S$ in der Figur eingezeichnet), die nicht in der längeren Form (FLIP$_L$) enthalten sind. Wie die viralen FLIPs (beispielhaft ist die Aminosäuresequenz des durch ORF E8 von EHV-2 kodierten FLIPs gezeigt) enthalten das murine (MM) Homolog FLIP$_L$ und die humanen (HS) Homologe FLIP$_L$ und FLIP$_S$ je zwei Todeseffektordomänen (DED1 bzw. DED2). Die murine und die humane FLIP$_L$-Form enthalten außerdem eine C-terminale Domäne, die der Protease-Domäne der Caspasen homolog ist. Die strukturelle Organisation von FLIP$_L$ (2 N-terminale Todeseffektordomänen verbunden mit einer Caspase) entspricht somit der strukturellen Organisation von FLICE und Mch4, allerdings fehlt im Vergleich zu den Caspasen der konservierte Cysteinrest der aktiven Proteasedomäne: im humanen und im murinen FLIP$_L$ ist die entsprechende Aminosäureposition durch einen Tyrosinrest ersetzt (in der Figur ist die entsprechende Position durch ein Sternchen gekennzeichnet).

Figur 4a zeigt die DNA- und die Aminosäuresequenz des humanen FLIP$_S$, das aus einer cDNA-Bank von aktivierten T-Zellen durch Screening mit einer $^{32}$P-markierten DNA-Sonde (EcoRI/Rsal-Fragment des 5'-Terminus des DNA-Inserts des EST-Klons Nr. 309776) isoliert wurde.

Figur 4b (bestehend aus 2 Seiten) zeigt die DNA- und die Aminosäuresequenz des humanen c-FLIP$_L$, das aus einer cDNA-Bank von aktivierten T-Zellen durch Screening mit der in Figur 4a beschriebenen Sonde isoliert wurde.

Figur 4c (bestehend aus 3 Seiten) zeigt die cDNA- und die Aminosäuresequenz des murinen c-FLIP$_L$, das aus einer murinen Herzmuskel-cDNA-Bank durch Screening mit der in Figur 4a isoliert wurde.

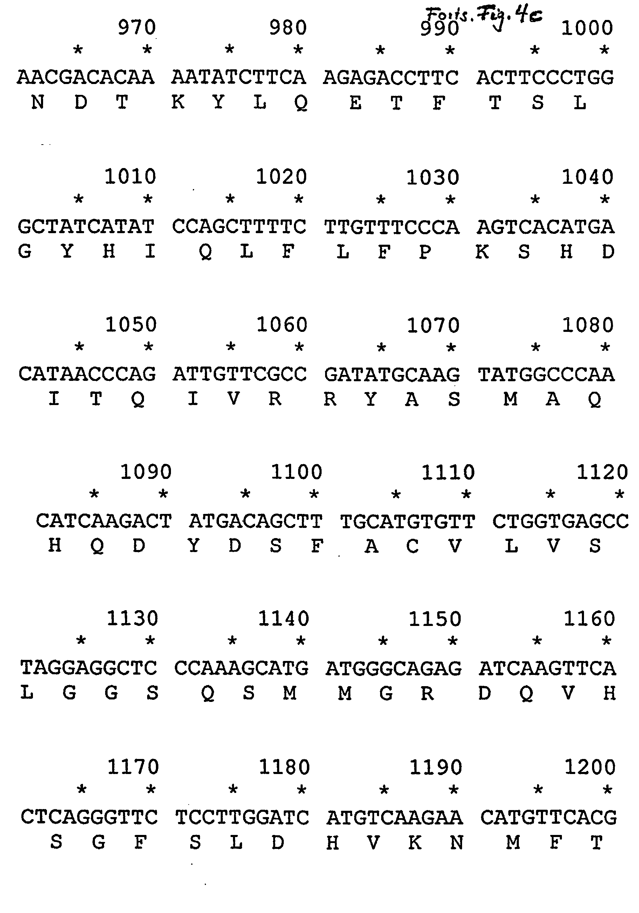

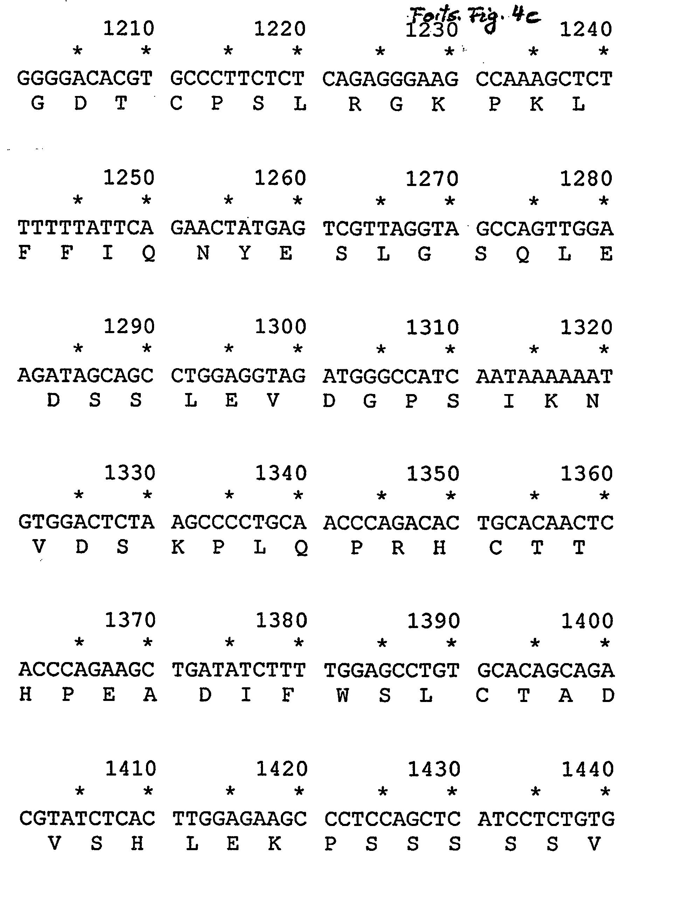

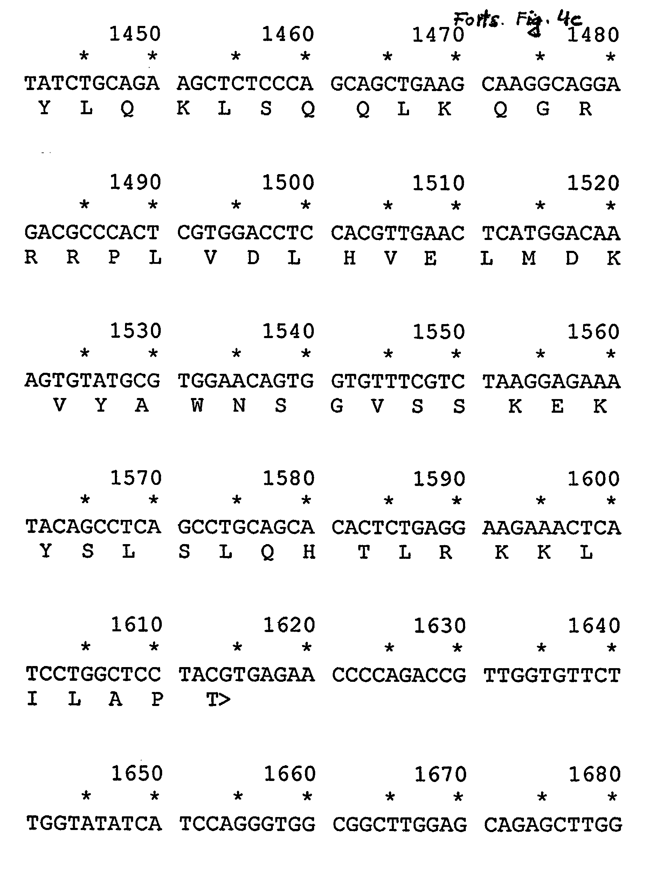

In Figur 5 ist durch Kotransfektionsexperimente in eukaryontischen 293T Zellen am Beispiel der viralen FLIPs von EHV-2 (ORF E8, Fig. 5a) und von MCV (ORF 159L, Fig. 5b) gezeigt, daß die viralen FLIPs an das Adaptorprotein FADD binden, und daß diese Bindung nicht die Anlagerung des Adaptorproteins FADD an den zytoplasmatischen, die Todesdomäne enthaltenden Proteinteil des CD95 Todesrezeptors verhindert. Über die Bindung der viralen FLIPs E8 (von EHV-2) und 159L (von MCV) an FADD wird damit eine Anlagerung der viralen FLIPs über den Adaptor FADD mit dem zytoplasmatischen Proteinteil des Todesrezeptors CD95 ermöglicht.

Im unteren Teil der Abbildungen 5a und 5b wird jeweils die Expression der entsprechenden Proteine (virales FLIP, FADD und zytoplasmatischer Proteinteil von CD95) durch die in der Literatur beschriebene Western Blot Analyse von Zellextrakten der transfizierten 293T Zellen nachgewiesen. Im oberen Teil der Abbildung 5 werden die oben beschriebenen Assoziationen (FLIP-FADD, FADD-CD95 und FLIP-FADD-CD95) durch Immunpräzipitationen (IP) demonstriert. Jede Spalte entspricht dabei einer Probe, und durch Pluszeichen oberhalb der Figur ist die für jede Probe verwendete Kombination von Expressionsvektoren für die Transfektion der 293T Zellen gekennzeichnet. In Immunpräzipitationen von Flag-markiertem E8 bzw. Flag-markiertem 159L mittels eines anti-Flag Antikörpers läßt sich FADD nur dann im anti-FADD Western Blot (jeweils zweiter Figurteil von oben) detektieren, wenn das jeweilige FLIP (E8 bzw. 159L) mit FADD kotransfiziert wurde (das Auftreten eines solchen Falles ist gekennzeichnet durch Pluszeichen in der ersten und zweiten Zeile einer Spalte). Eine Assoziation der viralen FLIPs mit dem myc-markierten zytoplasmatischen Proteinteil von CD95 (detektiert durch anti-myc Western Blot im jeweils obersten Figurteil) ist nur in Gegenwart von FADD möglich, d.h. nur wenn v-FLIP, FADD und das myc-markierte zytoplasmatische Proteinteil von CD95 gemeinsam exprimiert wurden (Pluszeichen in allen 3 Zeilen einer Spalte).

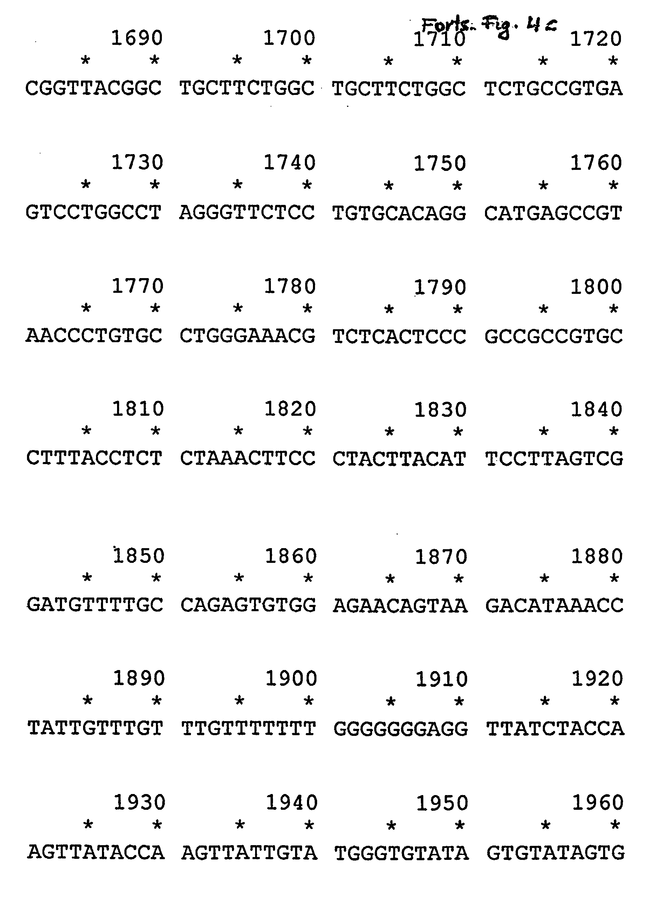

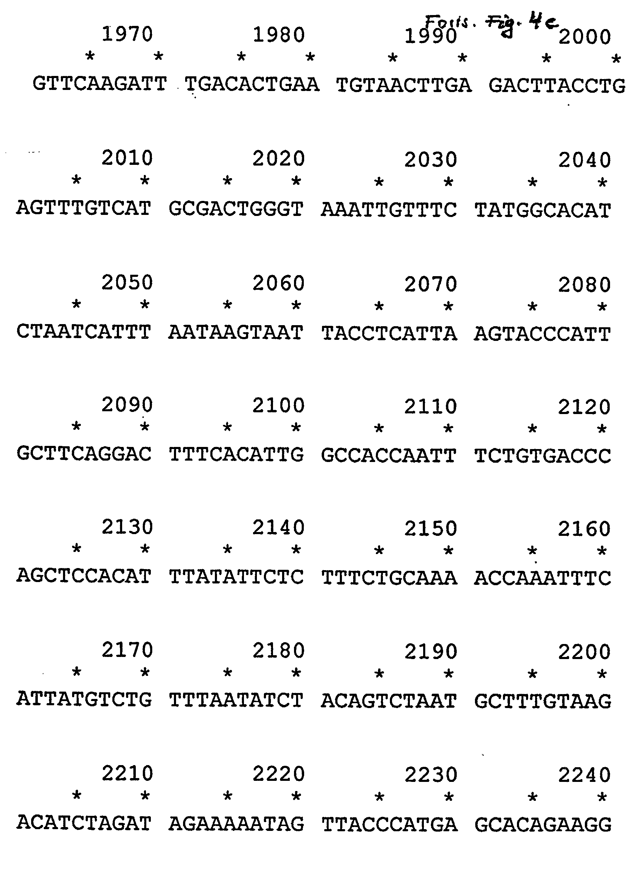

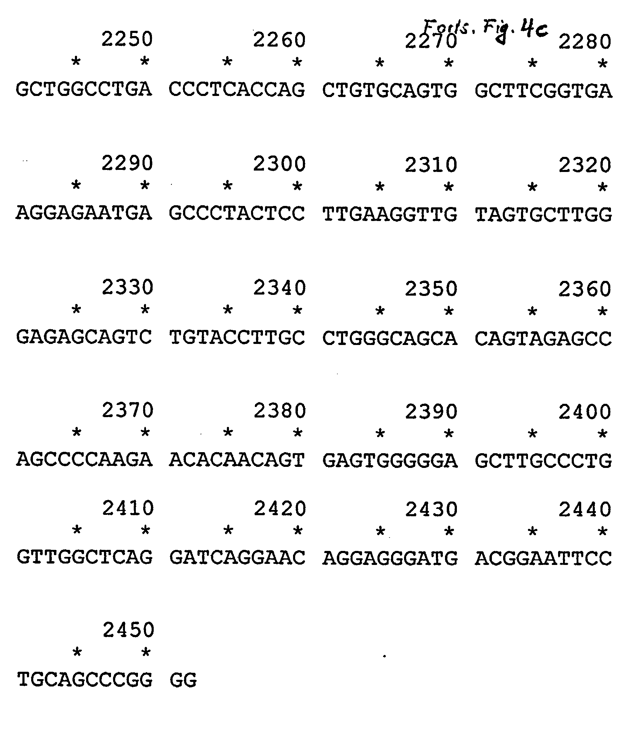

Figur 6 zeigt die Anlagerung des viralen FLIP-Proteins E8 von EHV-2 mit dem agonistisch stimulierten CD95 Rezeptorkomplex in humanen Raji B-Zell-Klonen, die stabil mit einem Expressionsvektor für das E8-Protein transfiziert wurden. Figur 6a zeigt durch Western Blot Analyse die Expression des Flag-markierten E8-Proteins in Zellextrakten von zwei mit RE8/11 und RE8/19 bezeichneten Raji B-Zellklonen, die mit einem Expressionsvektor für E8 transfiziertwurden. Kein E8-Protein wird dagegen detektiert in den mit RCo/1 und RCo/3 bezeichneten Raji Kontrollklonen, die mit dem entsprechenden Expressionsvektor ohne E8-Insertion transfiziert wurden. Figur 6b zeigt das Ergebnis der Analyse von Immunpräzipitationen des agonistisch mit dem Antikörper APO-1 stimulierten CD95 Todesrezeptors (Kischkel et al., EMBO Journal 14, 5579-5588 (1995)) aus den $^{35}$S metabolisch markierten Raji-Zellklonen RCo/1 (ein nicht E8 exprimierender Kontrollklon) und RE8/11 (ein E8 exprimierender Klon). Eine weiße Pfeilspitze markiert die Wanderungsposition des radioaktiv markierten E8 Proteins, das in dem CD95-Anlagerungskomplex des Klones RE8/11, aber nicht im entsprechenden Komplex des Kontrollklones RCo/1 enthalten ist.

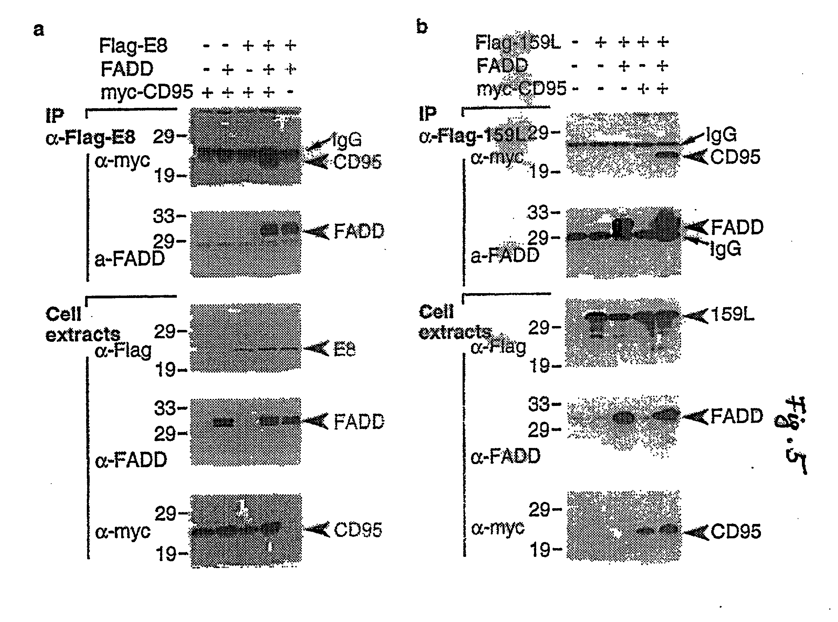

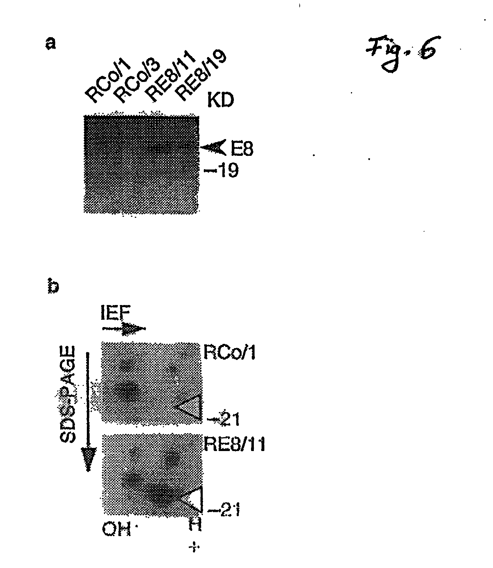

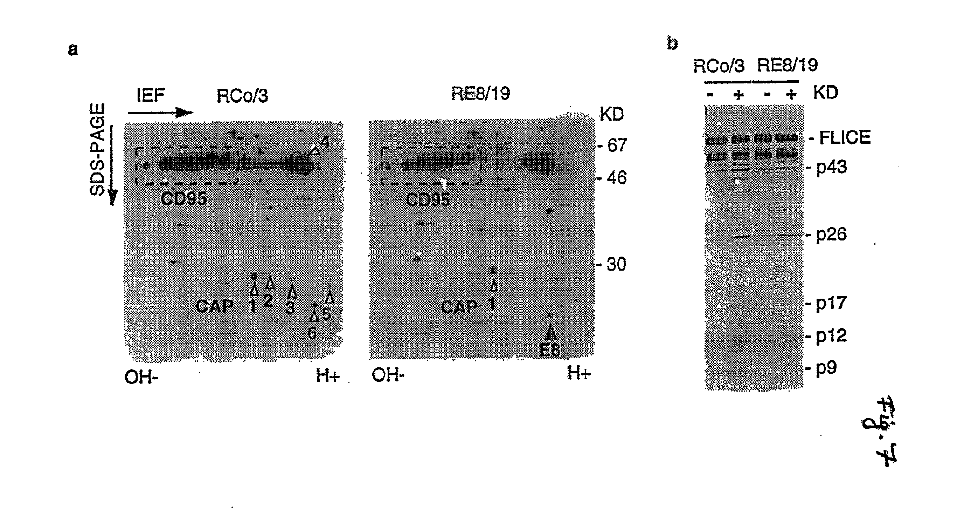

In Figur 7a wird die Assoziation von $^{35}$S-markierten Proteinen mit dem agonistisch stimulierten CD95 Todesrezeptor (Kischkel et al., EMBO Journal 14, 5579-5588 (1995)) in den nicht transfizierten Raji Kontrollzellen (Klon RCo/3, linker Teil der Abbildung) und in solchen Zellen, die mit einem Expressionsvektor für das virale FLIP E8 des EHV-2 transfiziert wurden (Klon RE8/19, rechter Teil der Abbildung), durch eine zweidimensionale Gelelektrophorese-Analyse von CD95 (anti-APO-1)-Immunpräzipitationen verglichen. Die Figur zeigt, daß das virale Protein E8 mit dem agonistisch stimulierten CD95 Todesrezeptor assoziiert, ohne dabei die Anlagerung des Adaptormoleküls FADD (CAP 1) an den Rezeptor zu beeinträchtigen (das mit der Bezeichnung CAP1 versehene FADD Protein ist im agonistisch stimulierten CD95-assoziierten Signaltransduktionskomplex sowohl des nicht E8 exprimierenden Kontrollklons RCo/3 als auch im E8 exprimierenden Raji Klon RE8/19 enthalten, man vergleiche den linken und den rechten Teil der Figur) . Ferner zeigt die Figur, daß in solchen Zellen, die das virale FLIP exprimieren, die Anlagerung von FLICE (CAP4) und von FLICE-ähnlichen Molekülen (CAP3) an den Rezeptor verhindert wird, und somit die im Rezeptoranlagerungskomplex stattfindende Umsetzung von FLICE in seine beiden Spaltprodukte CAP5 und CAP6 blockiert ist (Abwesenheit der mit 4 bis 6 markierten radioaktiv markierten Proteine im E8-exprimierenden Klon RE8/19 nur im rechten, nicht jedoch im linken Teil der Figur).

Figur 7b zeigt, daß die durch Behandlung von Zellen mit dem agonistisch stimulierenden anti-CD95-Antikoerper APO-1 induzierte FLICE-Spaltungsaktivität des agonistisch stimulierten Rezeptors des E8-exprimierenden Raji B-Zellklons RE8/19 wesentlich geringer ist als die entsprechende Aktivität des Kontrollklons RCo/3 (der kein E8 exprimiert). Die Anwesenheit von FLICE-Spaltungsaktivität im CD95 Anlagerungskomplex ist quantifizierbar durch die Bildung der FLICE-Spaltprodukte p43, p26, p17, p12 und p9 aus radioaktiv markiertem FLICE durch CD95(anti-APO-1) Immunpräzipitate von APO-1 behandelten (+), aber praktisch nicht von unbehandelten (-) Zellen. In dem hier gezeigten FLICE-Spaltungsversuch zeigen sich die oben beschriebenen Banden der molekularen Masse 43, 26, 17, 12 und 9 kD für den APO-1-stimulierten (+), aber praktisch nicht für den unstimulierten (-) Raji Kontrollklon RCo/3, der kein E8 exprimiert. Dagegen ist die durch APO-1 Behandlung induzierte FLICE-Spaltungsaktivität des CD95 Anlagerungskomplexes des E8 exprimierenden Klones RE8/19 stark beeintraechtigt (die entsprechenden FLICE-Spaltungsprodukte sind für diesen Klon kaum noch nachweisbar). Figur 7 zeigt somit, daß durch die Gegenwart des FLIPs E8 aus EHV-2 im DISC des Todesrezeptors CD95 die Anlagerung und die durch proteolytische Spaltung bewirkte Aktivierung von FLICE gehemmt ist.

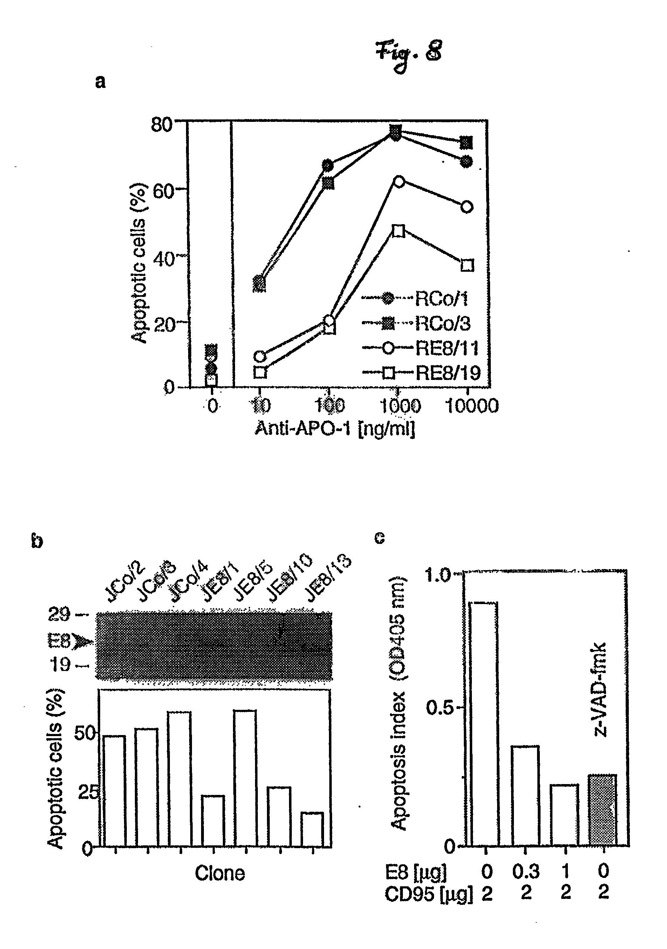

In Figur 8 wird gezeigt, daß eukaryontische Zellen durch die Expression eines viralen FLIPs (ORF E8 von EHV-2 oder ORF159L von MCV) wesentlich resistenter gegen Apoptose-Induktion durch den CD95-Todesrezeptor sind als Kontrollzellen. In Figur 8a wird der Prozentsatz der apoptotischen Raji B-Zellen (Ordinate) in Abhängigkeit von der Konzentration des agonistischen anti-CD95(APO-1)-Antikörpers (Abszisse) (anwesend im Kulturmedium während 24h bei 37°C) für zwei E8-exprimierenden Raji-B-Zellklone (RE8/11 und RE8/19) und zwei nicht E8 exprimierende Raji Kontrollklone (RCo/1 und RCo/3) verglichen. Die beiden E8-exprimierenden Klone erweisen sich dabei als deutlich resistenter gegen die CD95-vermittelte Apoptose als die Kontrollklone. Eine APO-1 Konzentration im Kulturmedium von 100ng/ml reichte unter den gewählten experimentellen Bedingungen nämlich aus, um in mehr als 60% der nicht E8 exprimierenden Kontrollklone Apoptose zu induzieren, während unter den gleichen Bedingungen nur ca. 20% der Zellen jedes der beiden E8 exprimierenden Raji Klone RE8/11 und RE8/19 apoptotisch waren. Um Apoptose in ca. 50% der Raji Zellen zu induzieren war für die beiden E8 exprimierenden Raji Klone RE8/11 und RE8/19 eine mehr als 10-fach höhere APO-1 Konzentration notwendig als für die nicht E8 exprimierenden Kontrollklone RCo/1 und RCo/3.

In Figur 8b ist dargestellt, daß auch humane Jurkat T-Zellen durch die Expression des viralen FLIPs des ORF E8 von EHV-2 eine Resistenz erwerben gegen die Apoptose-Induktion durch den CD95 Todesrezeptor. Für die Experimente wurden E8 exprimierende Jurkat Klone (JE8/1, JE8/10 und JE8/13) sowie ein nicht E8 exprimierender Jurkat Klon (JE8/5) und mit Kontrollvektor transfizierte Klone (JCo/2 und JCo/4) verwendet. Die Empfindlichkeit der Klone gegen CD95 Ligand-induzierte Apoptose wurde durch Inkubation der Klone für 3h bei 37°C mit in Kulturmedium 1/10 verduenntem Überstand von CD95L-produzierenden neuronalen Zellen (Rensing-Ehl et al., Eur. J. Immunol. 25, 2253-2258 (1995)) und anschließende Analyse der Propidiumiodid-markierten Zellen im FACScan-Durchflußzytometer (Nicoletti et al., J. Immunol. Methods 139, 271-279 (1991)) bestimmt. Figur 8b zeigt insbesondere, daß die Menge des in den Klonen exprimierten Flag-markierten viralen FLIPs (nachgewiesen durch den in der Literatur beschriebenen Western Blot von Zellextrakten mit anti-Flag Antikörper, oberer Teil von Figur 8b) mit einer Reduktion der Apoptose-Empfindlichkeit gegen den CD95 Liganden (unterer Teil von Figur 8b) korreliert, denn der Prozentsatz der durch den CD95 Liganden induzierten apoptotischen Zellen (angegeben auf der Abszisse) ist umso niedriger, je stärker die im Western Blot im oberen Teil der Figur 8b detektierte E8-Expression der Klone ist. Insbesondere war unter den gewählten experimentellen Bedingungen der Prozentsatz der apoptotischen Zellen für nicht E8-exprimierende Kontrollklone (JCo/2, JCo/3, JCo/4 und JE8/5) mindestens dreimal höher als der Prozentsatz der apoptotischen Zellen für den Jurkat Klon mit der stärksten E8-Expression (Klon JE8/13).

Die Figur 8c zeigt den schützenden Effekt von v-FLIPs (gezeigt ist ein Experiment mit dem Genprodukt des ORFs E8 von EHV-2) auf die durch Überexpression des Todesrezeptors CD95 in 293T-Zellen (einer humanen embryonalen Nierenzellinie) induzierte Apoptose. Für dieses Experiment wurden 293T Zellen mit Expressionsvektoren für CD95 zusammen mit den unter dem Figurteil 8c angegebenen Mengen von Expressionsvektoren für das oben genannte v-FLIP transfiziert und 30h nach Transfektion geerntet. Die Apoptose-Induktion in den Zellen wurde durch die photometrische Quantifizierung apoptotischer Histon-DNA Komplexe ermittelt (der aud der Ordinate angegebene apoptotische Index entspricht der mit dem Cell-Death-Detection-ELISA-System von Boehringer ermittelten optischen Dichte (OD) der Proben bei 405 nm). Als Negativkontrollen für die Apoptose-Induktion dienten Zellen die mit Expressionsvektor ohne Insertion (mock) transfiziert wurden oder mit dem Expressionsvektor für CD95 transfizierte Zellen, die nach der Transfektion bis zur Zellernte in Medium mit 25μM z-VAD-fmk inkubiert wurden. Die Figur zeigt, daß das Genprodukt von E8 des EHV-2 293T Zellen vor der Apoptose-Induktion durch CD95-Überexpression schützen, dabei steigt der Schutzeffekt mit der Konzentrationdesverwendetenv-FLIP-Expressionsvektors an (dies entspricht einer Erniedrigung des apoptotischen Indexes mit steigender v-FLIP-Expressionsvektorkonzentration in Figur 8c). In den höchsten verwendeten Dosen (Koexpression von jeweils 1µg des Expressionsvektors für E8 mit der in Figurteil 8c angegebenen Menge des CD95 Expressionsvektors) ist der Schutzeffekt der viralen FLIPs dem durch den Protease-Inhibitor z-VAD-fmk erreichten Schutz vergleichbar.

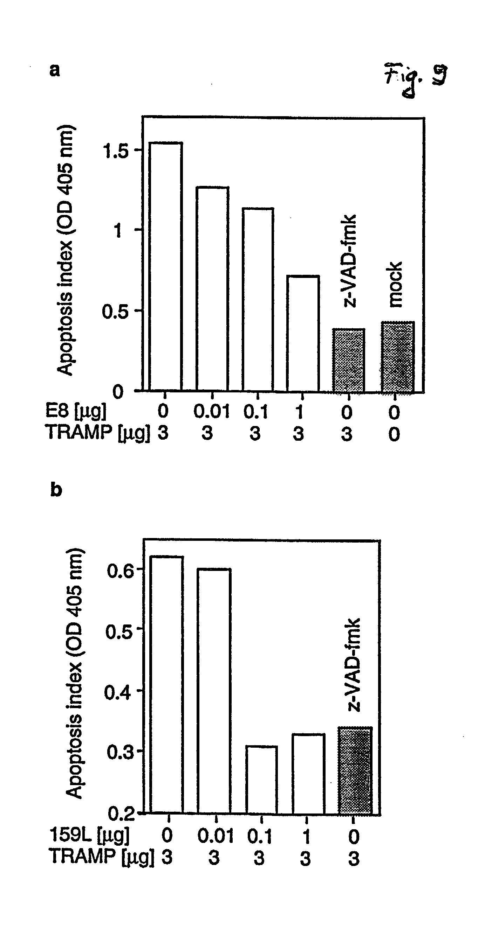

Figur 9 zeigt den schützenden Effekt von v-FLIPs (gezeigt sind Experimente mit den Genprodukten der ORFs E8 von EHV-2, ORF 159L von MCV und ORF 71 von HVS) auf die durch Überexpression des Todesrezeptors TRAMP in 293T-Zellen (einer humanen embryonalen Nierenzellinie) induzierte Apoptose. Für diese Experimente wurden 293T Zellen mit Expressionsvektoren für TRAMP (Bodmer et al., Immunity 6, 79-88 (1997)) zusammen mit den angegebenen Mengen von Expressionsvektoren für die oben genannten v-FLIPs transfiziert und 30h nach Transfektion geerntet. Als Negativkontrollen für die Apoptose-Induktion dienten Zellen die mit einem Expressionsvektor ohne Insertion (mock) transfiziert wurden oder mit dem CD95-Expressionsvektor transfizierte Zellen, die nach der Transfektion bis zur Zellernte in Medium mit 25µM z-VAD-fmk inkubiert wurden. Die Apoptose-Induktion in den Zellen wurde wie in Figur 8 beschrieben ermittelt. Für alle 3 gezeigten v-FLIPs ergibt sich mit zunehmender Konzentration des jeweiligen v-FLIP-Expressionsvektors eine Reduktion der durch TRAMP-Überexpression induzierten Apoptose (quantifiziert über die optische Dichte bei 405nm wie oben beschrieben). Für die jeweils höchsten in den Experimente verwendeten Mengen der v-FLIP Expressionsvektoren (die verwendete Menge der Expressionsvektoren ist unterhalb der Figuren in µg angegeben) wurde ein Apoptoseschutz der TRAMP-überexprimierenden Zellen von mindestens 70% erreicht (im Vergleich zu 100%igem Schutz durch den Protease-Inhibitor z-VAD-fmk).

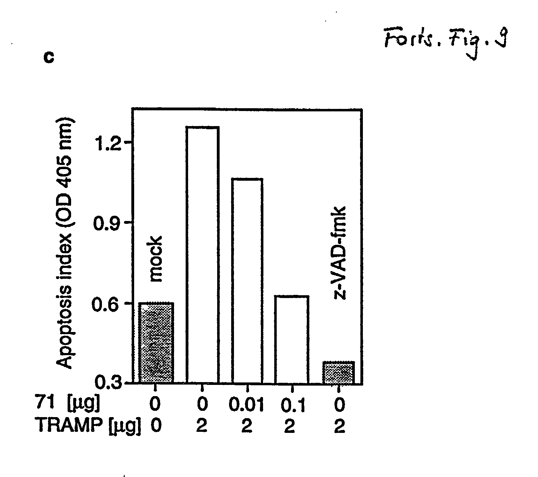

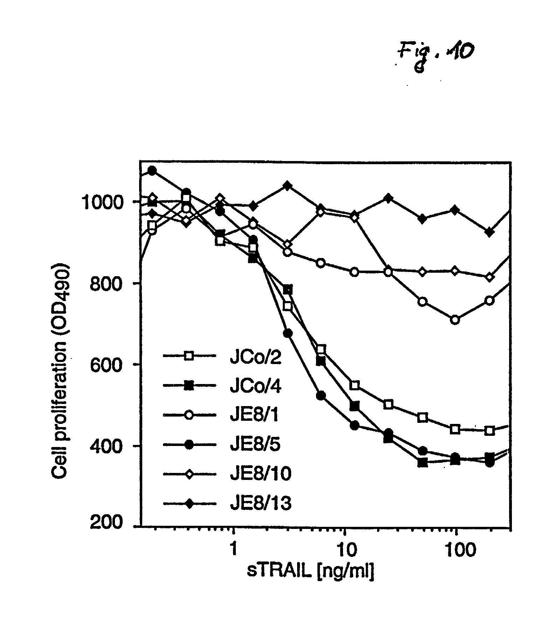

In Figur 10 ist dargestellt, daß humane Jurkat T-Zellen durch die Expression des viralen FLIPs des ORF E8 von EHV-2 eine Resistenz gegen die Apoptose-Induktion durch den Rezeptor für den Todesliganden TRAIL erwerben. Dabei korreliert die Menge des in den Klonen exprimierten viralen FLIPs (nachgewiesen im Western Blot von Zellextrakten, oberer Teil der Figur 8b) mit dem Ausmaß der Zellviabilität in Gegenwart des Apoptose-induzierenden Todesrezeptor-Liganden TRAIL (Figur 10). Die Empfindlichkeit oben beschriebener Klone gegen TRAIL-induzierte Apoptose wurde durch Inkubation mit den in der Abszisse angegebenen Konzentrationen von rekombinantem löslichem Flag-markiertem TRAIL und kreuzvernetzendem anti-Flag Antikörper für 20h bei 37°C und anschließende Bestimmung der Proliferationsfähigkeit der Zellen (Ordinate) mit einem Zellprofilierungsassay (Celltiter 96 AQ, Promega) bestimmt. Die nicht E8 exprimierenden Jurkat Klone (JCo/2, JCo/3, JCo/4 und JE8/5) wiesen in diesem Test bei zunehmender TRAIL-Konzentration eine deutliche Abnahme der Proliferation auf (sigmoider Kurvenverlauf, Abfall der optischen Dichte bei abnehmender Proliferation). Dagegen wurde eine nahezu gleichbleibende Zellproliferation des Jurkat Klons JE8/13 mit der stärksten E8-Expression beobachtet (horizontaler Kurvenverlauf). Die beiden Jurkat Klone mit intermediärem E8-Expressionsniveau (JE8/1 und JE8/10) wiesen nur eine schwache Beeinträchtigung der Proliferation durch die TRAIL-Behandlung auf.

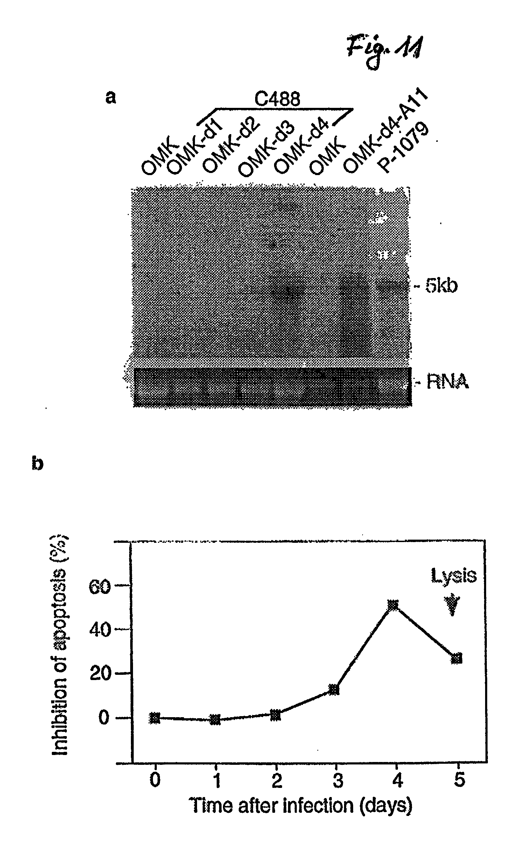

Figur 11 demonstriert die Korrelation der Expression eines viralen FLIPs (ORF 71 des HVS) im Verlaufe der viralen Infektion der Wirtszelle OMK (owl monkey kidney) mit dem Schutz der Wirtszelle gegen CD95 Ligand-induzierte Apoptose. Figur 11a zeigt eine Northern Blot Analyse zur Detektion von Transkripten des ORF71 von HVS in Proben von nicht virusinfizierten Kontrollzellen (OMK), in Proben von OMK Zellen, die mit dem HVS Stamm C488 für 1 bis 4 Tage (C488, OMK d1 bis d4) oder mit dem HVS Stamm A11 für 4 Tage (OMKd4-A11) infiziert waren, oder von Proben einer semipermissiven T-Zellinie, die geringe Mengen an Viruspartikel produziert (P-1079). In virusinfizierten OMK Zellen ist ein spezifisches Transkript von ca. 5kb am vierten Tag der Infektion detektierbar. Dies korreliert mit der in Figur 11b gezeigten Inhibierung der CD95 Ligand-induzierten Apoptose der virusinfizierten Zellen im Vergleich zu nicht infizierten Zellen zu diesem Zeitpunkt. Die scheinbare geringere Apoptose-Schutz am Tag 5 der Virusinfektion geht auf eine nun einsetzende massive infektionsbedingte Lyse der Zellen (ca. 50% Lyse am Tag 5 gegenüber weniger als 10% Lyse am Tag 4) zurück. Die Inhibition der CD95 Ligand-induzierten Apoptose von virusinfizierten OMK Zellen gegenüber nicht infizierten Kontrollzellen wurde durch Inkubation der Zellen mit rekombinantem CD95 Liganden und kreuzvernetzendem anti-Flag Antikörper für 20h und anschließender Quantifizierung apoptotischer Histon-DNA-Komplexe (Cell-Death-Detection-ELISA, Boehringer) ermittelt.

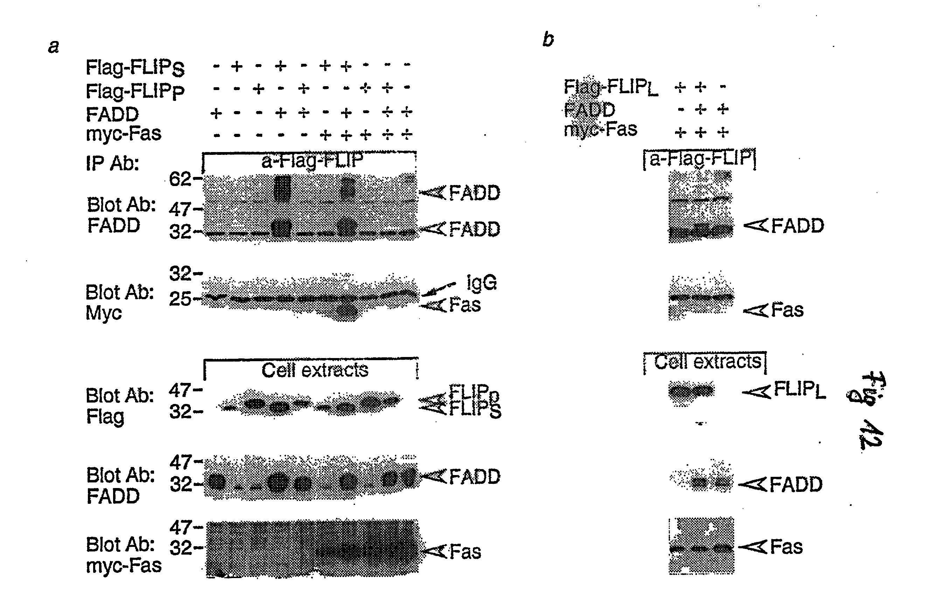

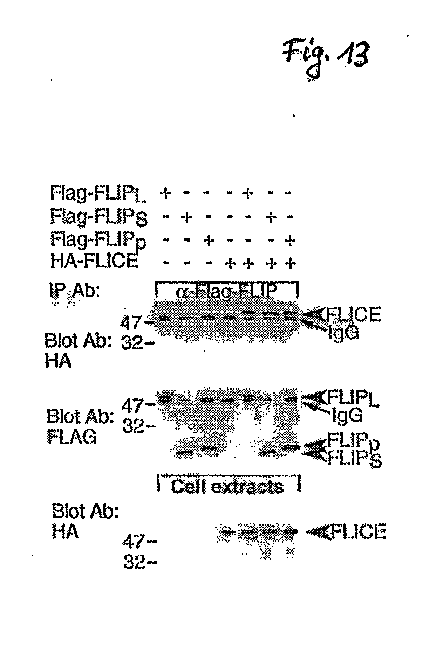

In Figur 12 wird durch Kotransfektionsexperimente in eukaryontischen 293T Zellen gezeigt, daß das die kurze und die lange Form des humanen FLIP (FLIP$_S$ und FLIP$_L$), aber nicht der Proteinteil mit Caspase-Homologie (FLIP$_P$) an das Adaptorprotein FADD binden. Die Figur zeigt weiterhin, daß die Bindung von FLIP$_S$ oder FLIP$_L$ an das Adaptorprotein FADD nicht die Anlagerung von FADD an den zytoplasmatischen, die Todesdomäne enthaltenden Proteinteil des CD95 Todesrezeptors verhindert. Über die Bindung des humanen FLIP$_S$ und FLIP$_L$ an das FADD Adaptorprotein wird damit eine Anlagerung dieser FLIP-Formen, über den Adaptor FADD, mit dem zytoplasmatischen Proteinteil des Todesrezeptors CD95 ermöglicht.

Im unteren Teil der Figuren 12a und 12b wird jeweils die Expression der entsprechenden Proteine (FLIP$_S$, FLIP$_P$, FLIP$_L$, FADD und zytoplasmatischer Proteinteil von CD95) durch Western Blot Analyse von Zellextrakten der transfizierten 293T Zellen nachgewiesen, während im oberen Teil der Figuren 12a und 12b die oben beschriebenen Protein-Assoziationen ($FLIP_S$ oder $FLIP_L$ mit FADD, FADD mit CD95 und $FLIP_S$ oder $FLIP_L$ über FADD mit CD95) durch Immunpräzipitationen (IP) demonstriert werden. Jede Spalte entspricht dabei einer Probe, und durch Pluszeichen oberhalb der Figurteile 12a und 12b ist die für jede Probe verwendete Kombination von Expressionsvektoren für die Transfektion der 293T Zellen gekennzeichnet. In Immunpräzipitationen von Flag-markiertem humanem FLIP mittels eines anti-Flag Antikörpers läßt sich für die die beiden N-terminalen Todeseffektordomänen enthaltenden Formen des humanen FLIPs ($FLIP_S$ oder $FLIP_L$) FADD nur dann im anti-FADD Western Blot (oberster Teil der Figuren 12a und 12b) detektieren, wenn mit einem FADD-Expressionsvektor kotransfiziert wurde (Pluszeichen in den entsprechenden Zeilen einer Spalte). Der Caspase-homologe Proteinteil ($FLIP_P$) des humanen FLIPs, der nicht die beiden Todeseffektordomänen enthält, kann nicht mit FADD assoziieren (Ergebnis des anti-FADD Western Blots der Proben in Spalte 5 und 9 von Figur 12a). Eine Assoziation der Formen $FLIP_S$ und $FLIP_L$, jedoch nicht des Proteinteils $FLIP_P$, mit dem myc-markierten zytoplasmatischen Proteinteil von CD95 (detektiert durch anti-myc Western Blot im jeweils zweiten Figurteil von oben) ist nur in Gegenwart von FADD möglich, d.h. nur wenn $FLIP_S$ bzw. $FLIP_L$, FADD und das myc-markierte zytoplasmatische Proteinteil von CD95 gemeinsam exprimiert wurden (Spalte 7 der Figur 12a und Spalte 2 der Figur 12b).

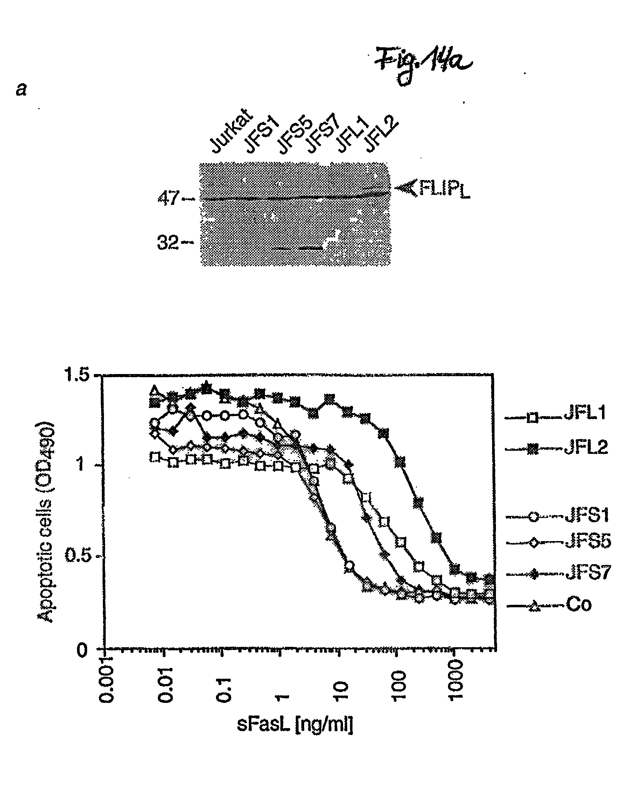

Figur 13 zeigt durch Kotransfektionsexperimente in eukaryontischen 293T Zellen, daß die humanen Proteine $FLIP_S$ und $FLIP_L$ an die Cysteinprotease FLICE binden. Die Figur zeigt weiterhin, daß zur Bindung von $FLIP_L$ an FLICE sowohl der die Todeseffektordomänen enthaltende N-terminale Proteinteil von $FLIP_L$ als auch der die Caspase-Homologie besitzende C-terminale Proteinteil von $FLIP_L$ beitragen. Die Expression des HA-markierten FLICE-Proteins in Zellextrakten der Transfizierten Proben wurde im untersten Teil der Abbildung durch einen Western Blot mit anti-HA-Antikörper nachgewiesen. Der mittlere Teil der Abbildung zeigt durch einen anti-Flag Western Blot, daß vergleichbare Mengen von 3 verschiedenen Flag-markierten FLIP-Proteinen oder Proteinteilen durch anti-Flag Immunpräzipitation aus den transfizierten Zellen präzipitiert wurden. Der oberste Figurteil zeigt durch eine anti-HA-Western Blot Analyse der anti-Flag-Präzipitate, daß das HA-markierte FLICE sowohl an $FLIP_L$ als auch an $FLIP_S$ als auch an den Caspase-homologen C-terminalen Proteinteil von $FLIP_L$ ($FLIP_P$) bindet.

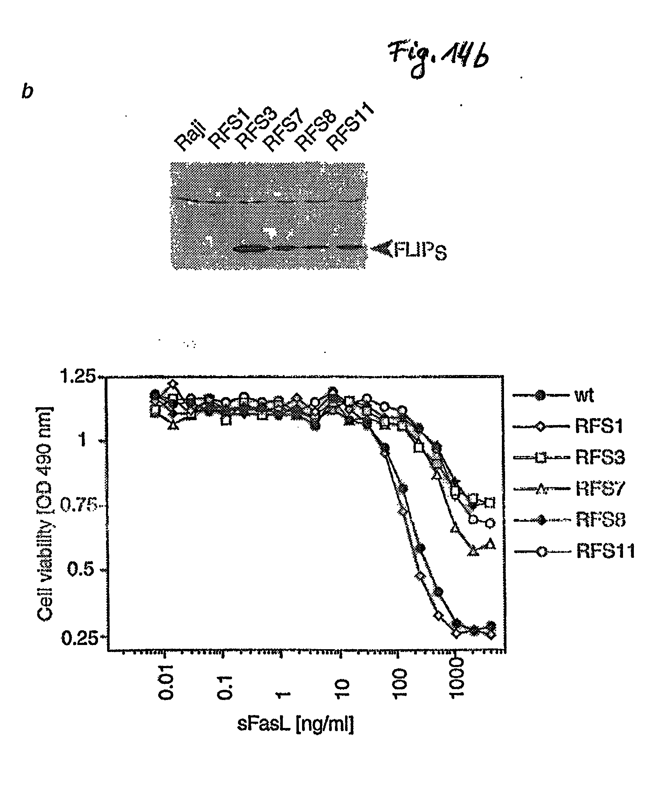

Figur 14 zeigt, daß eukaryotische Zellen durch die Expression des humanen $FLIP_S$ oder $FLIP_L$ wesentlich resistenter gegen Apoptose-Induktion durch den CD95-Todesrezeptor sind als Kontrollzellen. Ausserdem wird gezeigt, daß die längere Form des humanen FLIPs ($FLIP_L$) einen effizienteren Schutz gegen die CD95-induzierte Apoptose vermittelt als die kürzere Form $FLIP_S$. In Figur 14a ist dargestellt, daß humane Jurkat T-Zellen durch die Expression des humanen $FLIP_S$ bzw. des humanen $FLIP_L$ eine Resistenz gegen die Apoptose-Induktion durch den Fas(CD95)-Liganden (FasL) erwerben. Dabei korreliert die Menge des in den Klonen exprimierten humanen VSV-markierten FLIPS oder $FLIP_L$ (nachgewiesen im anti-VSV-Western Blot von Zellextrakten, oberer Teil der Figur 14a) mit dem Ausmaß der Zellviabilität in Gegenwart des Apoptose-induzierenden FasL (unterer Teil der Abbildung 14a). Die Empfindlichkeit der Jurkat-Klone gegen FasL-induzierte Apoptose wurde durch Inkubation mit den in der Abszisse angegebenen Konzentrationen von rekombinantem löslichem Flag-markiertem FasL (sFasL) und kreuzvernetzendem anti-Flag Antikörper für 20h bei 37°C und anschließende Bestimmung der Proliferationsfähigkeit der Zellen (Ordinate) mit einem Zellproliferationsassay (Celltiter 96 AQ, Promega) bestimmt. Die nicht transfizierten parentalen Jurkat T-Zellen (Jurkat, Co), der nicht $FLIP_S$ exprimierende Jurkat Klon JFS1 und der schwach $FLIP_S$ exprimierende Jurkat Klon JFS5 wiesen in diesem Test einen sich überlagernden sigmoiden Kurvenverlauf auf (Abnahme der Proliferation bei zunehmender sFasL-Konzentration). Dagegen wurde eine Rechtsverschiebung der Proliferationskurve für den stärker $FLIP_S$ exprimierenden Klon JFS7 beobachtet. Für eine mit dem parentalen Kontrollklon vergleichbare Abnahme der Proliferation des Klons JFS7 war eine ungefähr 5-fach höhere Konzentration an FasL nötig als für den parentalen Klon. Eine noch stärkere Resistenz gegen FasL wurde für die beiden $FLIP_L$-transfizierten Jurkat Klone JFL1 und JFL2 beobachtet. Obwohl die Proteinexpression des VSV-markierten $FLIP_L$ nur schwach (JFL2) oder gar nicht (JFL1) im Western Blot nachweisbar war (oberer Teil der Figur 14a), waren die beiden Klone ungefähr 10-fach (JFL1) bzw. mehr als 25-fach (JFL2) resistenter gegen die Behandlung mit sFasL als der parentale Jurkat-Klon.

In Figur 14b ist dargestellt, daß auch humane Raji B-Zellen durch die Expression des humanen $FLIP_S$ eine Resistenz erwerben gegen die Apoptose-Induktion durch den sFasL. Für die Experimente wurden die das humane $FLIP_S$ exprimierende Raji-Klone RFS3, RFS7, RFS8 und RFS11 sowie ein nicht $FLIP_S$ exprimierender Raji Klon (RFS1) und der nicht transfizierte parentale Raji Klon (Raji Wildtyp, wt) verwendet und die Empfindlichkeit der Klone gegen sFasL-induzierte Apoptose wie in Figur 14a beschrieben bestimmt. Figur 14b zeigt, daß Raji-Klone, die VSV-markiertes $FLIP_S$ exprimieren (siehe anti-VSV Western Blot im oberen Teil der Figur 14b) eine partielle Resistenz gegen sFasL aufweisen. Insbesondere waren diese Klone unter den gewählten experimentellen Bedingungen mindestens 5-mal stärker resistent gegen die durch sFasL induzierte Proliferationshemmung als die nicht FLIPS exprimierenden Kontrollklone Raji (wt) und RFS1.

Die Figur 14c zeigt schließlich über den in Figur 14a beschriebenen experimentellen Ansatz, daß humane Raji B-Zellen durch die Expression von humanem $FLIP_L$ eine praktisch vollständige Resistenz gegen die Behandlung mit sFasL erwerben können. Insbesondere sind die Raji-Klone mit starkem (RFL12) und mittlerem (RFL2, RFL42 und RFL47) Expressionsniveau des VSV-markierten $FLIP_L$ praktisch vollständig resistent gegen die Behandlung mit sFasL bei den in der Abszisse angegebenen Konzentrationen des sFasL (dies zeigt sich in einem nahezu horizontalen Kurvenverlauf, das heißt unverändertes Zellproliferationsverhalten der jeweiligen Klone auch bei hohen (über 1µg/ ml) sFasL-Konzentrationen).

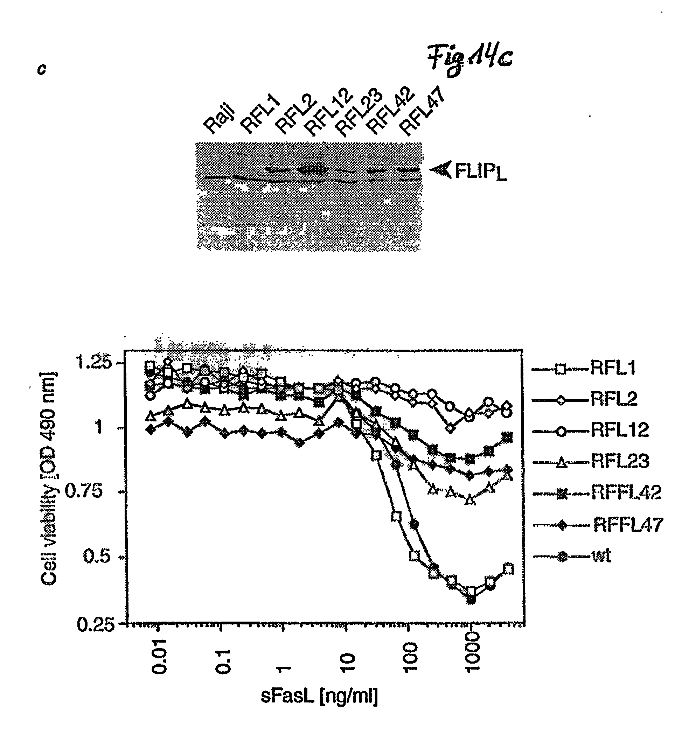

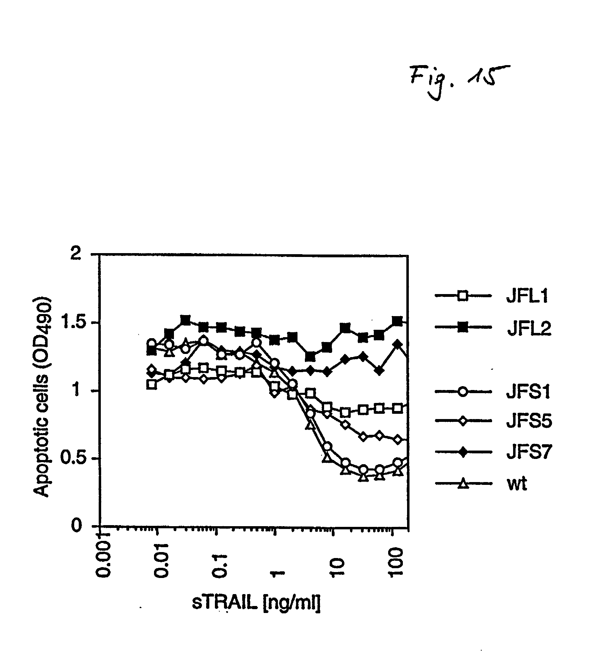

In Figur 15 ist gezeigt, daß humane Jurkat T-Zellen durch die Expression des humanen $FLIP_S$ oder $FLIP_L$ eine Resistenz gegen die Apoptose-Induktion durch den Rezeptor für den Todesliganden TRAIL erwerben. Dabei korreliert die Menge des in den Klonen exprimierten c-FLIPs (nachgewiesen im Western Blot von Zellextrakten, oberer Teil der Figur 14a) mit dem Ausmaß der Zellviabilität in Gegenwart des Apoptose-induzierenden Todesrezeptor-Liganden TRAIL (Figur 15). Die Empfindlichkeit der Jurkat Klone gegen TRAIL-induzierte Apoptose wurde durch Inkubation mit den in der Abszisse angegebenen Konzentrationen von rekombinantem löslichem Flag-markiertem TRAIL und kreuzvernetzendem anti-Flag Antikörper für 20h bei 37°C und anschließende Bestimmung der Proliferationsfähigkeit der Zellen (Ordinate) mit einem Zellproliferationsassay (Celltiter 96 AQ, Promega) bestimmt. Der parentale nicht transfizierte Jurkat Klon (wt) sowie der transfizierte, nicht $FLIP_S$ exprimierende Jurkat Klone JFS1 wiesen in diesem Test bei zunehmender TRAIL-Konzentration eine deutliche Abnahme der Proliferation auf (sigmoider Kurvenverlauf, Abfall der optischen Dichte bei abnehmender Proliferation). Klon JFS5, der im Western Blot (Figur 14a oben) ein intermediäres Niveau an VSV-markiertem $FLIP_S$ zeigte, wies eine partielle Resistenz gegen TRAIL auf, während Klon JFS7, der das VSV-markierte FLIPS stärker exprimiert, praktisch vollständig resistent genen TRAIL war (der horizontaler Kurvenverlauf zeigt, daß keine Beeinträchtigung der Proliferation durch den Todesrezeptorliganden TRAIL vorlag). Die experimentellen Kurven zeigen weiterhin, daß die beiden mit einem Expressionsvektor für humanes $FLIP_L$ transfizierten Jurkat Klone JFL1 und JFL2 trotz geringster (Klon JFL1) oder schwacher (Klon JFL2) Expression des VSV-markierten $FLIP_L$ (siehe Western Blot Figur 14a, oberer Figurteil) nahezu (JFL1) oder vollständig (JFL2) resistent gegen die Behandlung mit dem Todesrezeptorliganden TRAIL waren.

Figur 16 zeigt die Aminosäuresequenz des viralen FLIPs des HHV-8 (ORF 71) im Ein-Buchstaben-Code der Aminosäuren. Eine für diese Aminosäuresequenz codierende DNA Sequenz findet sich in der Datenbank GenBank mit der Zugangsnummer U90534 (ORF 71 in HHV-8).

Figur 17 zeigt die Aminosäuresequenz des viralen FLIPs des BHV-4 im Ein-Buchstaben-Code der Aminosäuren. Eine für diese Aminosäuresequenz codierende DNA Sequenz findet sich in der Datenbank GenBank mit der Zugangsnummer Z46385.

Die vorliegende Erfindung wird durch Ausführungsbeispiele näher erläutert.

Zunächst werden die allen folgenden Ausführungsbeispielen zugrundeliegenden experimentellen Randbedingungen erläutert:

Als Zellinien wurden eine humane embryonale Nierenzellinie (293T-Zellen), eine humane Leukämie T-Zellinie (Jurkat Zellen) oder eine humane Burkitt-Lymphoma B Zellinie (Raji Zellen) eingesetzt und aufgezogen wie bei Bodmer *et al.* (Immunity 6, 79-88 (1997)) beschrieben.

Als monoklonale Antikörper für die Immunopräzipitation und für das "Western Blotting" wurden anti-Flag Antikörper und anti-Flag Agarose (von Kodak International Bio- technologies), sowie anti-FADD Antikörper (von Transduction Laboratories), und Antikörper, die gegen das myc-Epitop (von der Firma Sigma, 9E10), gegen das VSV-Epitop (von der Firma Boehringer) und gegen das HA-Epitop gerichtet sind, verwendet.

Ein löslicher Bestandteil des humanen TRAIL Proteins, und zwar mit den Aminosäuren 95-281, wurde durch ein PCR-Verfahren aus einem EST-Klon (expressed sequence tag) hergestellt. Der Klon trägt die Bezeichnung 117926 und ist in der GenBank unter der Zugangsnummer T90422 eingetragen. Als Oligonukleotide für das PCR-Verfahren wurden folgende Sequenzen eingesetzt: das Oligonukleotid JT403 5'-TCAGCTGCAGACCTCTGAGGAAAC-3' und das Oligonukleotid JT469 5'-ACTAGTTAGCCAACTAAAAAG-3'. Die Klonierung des Abschnitts erfolgte in dem Vektor pQE-16 (von der Firma Qiagen), und zwar in eine PstI/SpeI Schnittstelle - entsprechend den Restriktionsenzymkurzbezeichnungen. Die klonierte Sequenz enthält auch eine Flag Sequenz und ein darauf folgendes Verbindungselement mit der Aminosäuresequenz GPGQVQLQ. Danach folgen die PstI und SpeI Schnittstellen zwischen den originalen BamHI/XbaI Stellen des Vektors. Die Proteinexpression in den Bakterien wurde mit 0,5 mM IPTG induziert. Nach 6 Stunden Inkubation bei 30°C wurden die Zellen geerntet und durch Beschallung lysiert. Die Lysate wurden zunächst mit 0,5%igem präkondensiertem Triton X-114 extrahiert, um die bakteriellen Lipopolysaccharide zu beseitigen, und schließlich wurde das Flag/TRAIL-Protein durch eine Chromatographiesäule mit M2 anti-Flag Agarose gereinigt; daraufhin erfolgte eine Elution mit 50 mM Zitronensäure und schließlich eine Neutralisation mit einer 1 M Trisbase und Dialyse gegen PBS (phosphate buffered saline, phosphatgepufferte Salzlösung).

Für die Isolierung von cDNA-Klonen des humanen und des murinen FLIP wurde als $^{32}$P-markierte Sonde ein durch PCR-Amplifikation erhaltenes DNA-Fragment der im EST-Klon 309776 enthaltenen DNA-Sequenz des humanen FLIPs benutzt. Dieses Fragment entspricht dem in Figur 4a mit 394 bis 903 numerierten DNA-Sequenzabschnitt. Das Screening der λ-ZAP cDNA-Bank von aktivierten humanen Lymphozyten (Fa. Stratagene, auf Anfrage erhältlich durch Hermann Eibel, Universität Freiburg, Deutschland) zur Isolierung der humanen Formen von FLIP und das Screening einer entsprechenden Bank von murinen Herzmuskelzellen (Stratagene) zur Isolierung des murinen FLIP$_L$ wurde nach Empfehlung des Herstellers durchgeführt.

Das komplette "Open Reading Frame" E8 des Virus EHV-2 (ORF E8) wurde durch PCR-Methoden aus der viralen DNA amplifiziert, wobei ein 5'-Primer mit einer EcoRI-Sequenz-Verlängerung und ein 3'-Primer mit einer Sequenz-Verlängerung für die Restriktionsenzyme BamHI und EcoRI hier verwendet wurden. Die Insertion (in die EcoR1 Schnittstelle des Vektors) erfolgte im gleichen Leseraster wie für das N-terminale Flag Epitop. Der Vektor ist abgeleitet vom pCR-3-Vektor (von der Firma Invitrogen). Analog wurde auch mit den Open Reading Frames ORF 159L vom Virus MCV (vom Institut für Medizinische Virologie der Universität Heidelberg, Deutschland) und dem Open Reading Frame ORF 71 des Virus HVS verfahren. Auch die FLIP-codierenden ORFs dieser Viren wurden mit PCR-Methoden amplifiziert und dann im richtigen Leseraster mit dem N-terminalen Flag-Epitop in die EcoR1 Schnitstelle des vom pCR-3 abgeleiteten Vektors insertiert. Ferner wurden das komplette ORF des humanen FLIP$_S$ und des humanen FLIP$_L$, sowie ein HindIII/XhoI-Fragment des 3' Terminus der DNA-Sequenz des humanen FLIP$_L$ durch PCR-Methoden amplifiziert und im richtigen Leserahmen in von pCR-3 abgeleitete Vektoren kloniert, die den jeweiligen Genprodukten ein N-terminales Flag- oder VSV-Epitop verleihen. Um eine stabile Expression vom Flag-E8, VSV-Flip$_S$ und VSV-FLIP$_L$ in Jurkat- und Raji-Zellen zu erreichen, wurden die Konstrukte aus Flag-E8, VSV-FLIP$_S$ und VSV-FLIP$_L$ in die multiple Klonierungsstelle (MCS) des Vektors pSRαpuro (ein Geschenk von R. Sekaly, IRCM, Montreal, Canada) weiterkloniert. Dieser Vektor trägt eine Puromycin-Resistenz.

Ein Expressionsvektor für die mit der myc-Sequenz versehenen cytoplasmatische Domäne des murinen Fas- Rezeptors wurde durch die Insertion eines PCR Fragments entsprechend den Aminosäuren 166-306, im richtigen Leseraster mit dem N-terminalen myc-Epitop, hergestellt und in den pCR-3artigen Vektor insertiert. Der Expressionsvektor für das humane TRAMP bzw. FADD im Vektor pCR-3 findet sich in der Veröffentlichung von Bodmer *et al.* (Immunity 6, 79-88 (1997)) beschrieben.

Humanes Fas (mit der Nukleotidsequenz -24 bis +1009) wurde durch PCR-Methoden aus einem EST-Klon (GenBank Zugangsnummer X63717) amplifiziert und dann als HindIII/XbaI Fragment in den pCR-3 Vektor subkloniert.

Um stabile Puromycin-resistente Transfektanten von Jurkatund Raji-Zellen zu erhalten, wurden die Zellen zweimal mit HeBS-Puffer gewaschen, und zwar bei einem pH Wert von 7,05 (0,8 mM NaH$_2$PO$_4$·2H$_2$O, 20 mM Hepes, 137 mM NaCl, 5 mM KCl, und 5,5 mM D-Glukose). Daraufhin wurden 8x10$^6$ Zellen in 800 µl HeBS resuspendiert, gemischt mit 20 µg des SRαpuro Plasmids (sowohl mit als auch ohne Flag-E8 Insertion) und schließlich einer Stromspannung von 250 V und einer Stromstärke von 960 µF ausgesetzt. Nach 48-stündiger Transfektion wurden die Zellen bei einer Konzentration zwischen 2000 und 20000 Zellen pro well auf Flachboden-Platten mit jeweils 96 wells verteilt und stabile Transfektanten durch Zugabe von 5 µg/ml Puromycin (von der Firma Sigma) selektiert.

Um eine vorübergehende Transfektion von 293T Zellen zu erreichen, wurden die Zellen bei einer Konzentration von 1 bis 2x10$^6$ Zellen/10 cm Platte oder 3 bis 6x10$^5$ Zellen/5 cm Platte verteilt und am darauffolgenden Tag mit Hilfe der in der Literatur beschriebenen Kalzium-Phosphat-Präzipitationsmethode transfiziert. Das Präzipitat wurde auf den Zellen für 8 Stunden belassen und die Zellen wurden schließlich 26 bis 30 Stunden nach der Transfektion geerntet.

Die 293-T Zellen von einer 10 cm Platte wurden nach der Transfektion in 200 µl Lysepuffer lysiert (1% NP40, 20 mM Tris-HCl, pH 7,4, 150 mM NaCl, wobei zusätzlich 1 mM EGTA, 1 mM pefabloc-sc (von der Firma Serva) und jeweils 10 µg/ml Leupeptin und Aprotinin (jeweils von der Firma Sigma) hinzugesetzt wurden. Postnukleäre Lysate wurden für mindestens 1 Stunde auf Sepharose 6B (von Pharmacia) vor der Präzipitation vorgereinigt. Danach wurde entweder für 2 Stunden oder über Nacht mit 3 µl von anti-Flag Agarose die Immunopräzipitation vorgenommen. Die Präzipitate wurden insgesamt viermal mit Lysepuffer gewaschen, wobei der Lysepuffer in den ersten beiden Waschgängen 1% NP40 und in den zweiten beiden Waschgängen 0,1% NP40 enthält. Die Präzipitate wurden dann in Probenpuffer erhitzt und danach durch SDS-PAGE und Western Blotting analysiert. Die Blots wurden mit 5%iger Milch in PBS mit 0,5% Tween saturiert und dann mit Hilfe von monoklonalem anti-Flag-Antikörper bei einer Konzentration von 5 µg/ml, mit anti-FADD-Antikörper (monoklonal) bei einer Konzentration von 1 µg/ml, mit monoklonalem anti-myc Antikörper (9E10) bei einer Konzentration von 5 µg/ml, oder mit monoklonalem anti-HA Antikörper bei 1 µg/ml für 1 Stunde bei Raumtemperatur inkubiert, wobei darauf ein zweiter Antikörper, der mit Peroxidase markiert war, aufgegeben wurde (zweiter Antikörper von den Jackson Laboratories). Die Detektion der Proteine wurde durch Chemiluminiszenz verstärkt (Amersham International). Jurkat-, Rajioder 293T-Zellklone wurden auf ihre Expression von transfizierten Proteinen überprüft. Dies geschah durch anti-Flag-, anti-VSV, anti-FADD-, anti-myc- oder anti-HA-Western-Blot-Analyse von postnukleären Zellysaten von äquivalentem Proteingehalt. Die metabolische Markierung von Raji-Zellen mit $^{35}$S, die anti-CD95-Immunopräzipitation und deren 2D-Gelelektrophorese wurden wie bereits beschrieben, durchgeführt (Kischkel, F.C. *et al.*, EMBO J. 14, 5579-5588 (1995)).

Die Analyse der durch FasL (CD95L) induzierten Apoptose wurde wie folgt durchgeführt. Puromycin-resistente Jurkat-Klone (ca. 3x10⁵/500 µl) wurden für 3 Stunden bei einer Temperatur von 37°C mit 50 µl eines Überstandes von Neuro-2a-Zellen, die mit einem FasL-Expressionsvektor transfiziert sind, oder aber mit einem Kontrollüberstand von Zellen, die mit einem Scheinvektor transfiziert sind, inkubiert (Rensing-Ehl, A. *et al.*, Eur. J. Immunol. 25, 2253-2258 (1995)). Die Jurkat-Zellen wurden mit FACS-Puffer (2% FCS und 0.02% Azid), gewaschen und fixiert in 70%igem eiskaltem Ethanol. Nach nochmaligem Waschen mit FACS-Puffer folgte eine Behandlung mit RNAse für 5 Minuten bei einer Temperatur von 37°C (50 µg/ml RNAse A in 100 mM Tris HCl, pH 7,4, 100 mM NaCl, 5 mM EDTA). Die Anfärbung erfolgte mit 250 µg/ml Propidiumjodid in PBS/ 1%igem NP40 zur Analyse des DNA-Gehalts. Die apoptotische Zellfraktion wurde analysiert und in einem Becton-Dickinsen FACScan-Gerät unter Verwendung der Lysis-II-Software quantifiziert. Die Suszeptibilität von Raji-Klonen auf anti-APO1-induzierte Apoptose wurde durch die Inkubation von Zellen (5x10⁵/ml) mit variierenden Konzentrationen von monoklonalem anti-APO1 Antikörper analysiert (in Medium für 16 Stunden bei 37°C). Die Quantifizierung der DNA Fragmentierung als Maß für die Ausprägung der Apoptose wurde in diesem Fall im wesentlichen so durchgeführt, wie bei Nicoletti, I. *et al.*, J. Immunol. Methods 139, 271-279 (1991) beschrieben. Zusammenfassend läßt sich sagen, daß die Zellen hierzu einmal mit PBS gewaschen wurden und vorsichtig in 0,1%igem Natriumzitrat und 0,1% Triton X-100 mit 50 µg/ml Propidiumjodid resuspendiert wurden. Nach einer Inkubation bei 4" in der Dunkelheit für die Dauer von mindestens 24 Stunden wurde der Prozentanteil der apoptotischen Zellkerne durch FACScan (R) (Becton-Dickinsen, Heidelberg, Deutschland) bestimmt.

Für eine Quantifizierung der Apoptose bei den transient transfizierten 293T-Zellen wurden Zellen aus einer 5 cm Platte in 200 µl Inkubationspuffer lysiert, und Lysate von 25000 Zellen wurden auf die Präsenz von Histon-DNA- Komplexen durch ein Cell Death Detection ELISA (Boehringer Mannheim) analysiert, wobei die entsprechenden Vorschriften des Herstellers beachtet wurden. Das Überleben von mit E8-oder mit humanem FLIP transfizierten sowie Kontroll-Jurkat-Klonen nach einer Zelltodinduktion durch TRAIL-Zugabe wurde nach Inkubation für die Dauer von 20 Stunden von ca. 50000 Zellen pro well mit den angezeigten Konzentrationen von rekombinantem TRAIL mit angehängtem Flag und 1 µg/ml von monoklonalem Antikörper anti-Flag getestet und die proliferierenden Zellen anschließend mit einem Celltiter AQ Proliferationstest (Promega) quantifiziert, gleichfalls nach den Angaben des Herstellers.

Die viralen *in vitro*-Kulturen und die Northern-Blot-Analyse von Transkripten wurden wie bei Fickenscher *et al.*, (J. Virol. 70, 6012-6019 (1996)) durchgeführt. Die Wirkung einer HVS-Infektion auf den durch CD95L ausgelösten Zelltod von Krallenaffen-Nierenzellen (owl monkey kidney, OMK) wurde dadurch getestet, daß die Zellen bei einer Konzentration von etwa 10⁴ Zellen/well auf 96 Well-Platten verteilt wurden. Zwei Tage danach wurde die Hälfte der wells derart mit Viren infiziert, daß sich als Multiplizität ungefähr ein Virus pro Zelle als infektiöses Agens ergab. Rekombinantes sCD95L (sFasL) (beschrieben bei Bodmer, J.L. *et al.*, Immunity 6, 79-88 (1997)) wurde bei einer Konzentration von 0,3 µg/ml (Alexis, San Diego) zusammen mit quervernetzenden, monoklonalen anti-Flag- Antikörper bei einer Konzentration von 1 µg/ml zu verschiedenen Zeitpunkten nach der Infektion hinzugegeben. Die Proben wurden 20 Stunden später auf die Anwesenheit von Histon-DNA-Komplexen, wie oben beschrieben, untersucht.

## 1. Experimentelles Ausführungsbeispiel

Um die inhibitorische Wirkung der viralen FLIP-Proteine auf die Apoptose zu testen und damit zu zeigen, daß Proteine mit Todeseffektordomänen auch inhibitorische Wikung auf die Apoptose haben können, wurden 293T-Zellen mit Expressionsvektoren, die für FADD bzw. N-terminal mit Flag markiertes FLIP-Protein (vom Virus EHV-2) codieren, eingesetzt. Als Promotoren für die Expressionsvektoren dienten in beiden Fällen CMV-Vektoren. Nach Transfektion der Zellen wurden Zellextrakte mit der sogenannten Western Blot Methode untersucht. Dabei werden die in den Zellysaten vorhandenen Proteine in der einen Richtung nach ihrem isoelektrischen Punkt, in der anderen Richtung der zweidimensionalen Auftragung nach ihrer Größe aufgetrennt. Durch entsprechende Antikörper kann dann die Expression der gewünschten Proteine überprüft werden. Im vorliegenden Fall dienten anti-Flag Antikörper (am N-terminalen Ende des FLIP-Proteins angefügt) und anti-FADD Antikörper zum Nachweis der stabilen Transfektion bei den 293T-Zellen. Die entsprechenden Darstellungen finden sich im unteren Teil der Figur 5a. Darüber hinaus wurden 293T-Zellen auch mit einem myc-CD95-Konstrukt (hierbei handelt es sich um einen Apoptose-Rezeptor) transfiziert. Alles in allem wurden fünf verschiedene Transfektionsklone hergestellt. Eine Darstellung des Transfektionsmusters der verschiedenen Klone findet sich im obersten Teil der Figur 5a. Beispielsweise sind dort in der viertletzten Spalte solche 293T-Zell- Transfektanten aufgetragen, die sowohl mit Flag-E8, FADD und myc-CD95 transfiziert worden sind. Nach dem Nachweis der stabilen Transfektion durch die entsprechenden Proteine wurde durch die im unteren Abschnitt der Abbildung 5a dargestellten Western Blots mit Hilfe von Coimmunopräzipitationsexperimenten die wechselseitige Assoziation der einzelnen Proteine untersucht. Dies geschieht, indem mit anti-Flag-E8 Antikörpern immunopräzipitiert wird. In der Western-Blot-Auftragung sind nur dann die Proteine FADD oder myc-CD95 zu erkennen, wenn sie zuvor als Copräzipitat mit dem anti-Flag Antikörper ausgefällt wurden. Damit ist die Detektion von FADD und/oder myc-CD95 nur dann möglich, wenn zum Zeitpunkt der Antikörperbindung auch das Flag-E8-Konstrukt mit FADD und/oder myc-CD95 assoziiert war. Somit dienten die beiden ersten Experimente (Spalte 1, 2) in Figur 5a, bei denen eine Flag-E8-Transfektion nicht vorlag, als Kontrollexperimente. Die drei letzten Spalten (jeweils Flag-E8, d. h. virales FLIP-Protein enthaltend) der Figur 5a zeigen das Bindungsverhalten von viralem FLIP an entweder FADD und/oder myc-CD95. Da es sich um ein denaturierendes Gel handelt, sind in den Western Blots keine Assoziate erkennbar. Es wird vielmehr durch die Anwesenheit von FADD und/oder myc-CD95 nach der Immunopräzipitation indirekt die Bindung der beiden Proteine an das virale FLIP-Protein im Zellextrakt gezeigt.

In Figur 5b ist ein analoges Experiment dargestellt, wobei hier einem Konstrukt aus Flag und dem FLIP-Gen des Virus MCV (Open Reading Frame 159L), die 293T-Zellen transfiziert wurden. Das experimentelle Vorgehen ist analog zum Vorgehen in Figur 5a und ausführlich in Ausführungsbeispiel 7 beschrieben. Für die Immunopräzipitation wird auf die Methode von Bodmer et al. (Immunity 6, 79-88 (1997)) hingewiesen.

Auch in Figur 5b ist im Ergebnis zu erkennen, daß nur dann eine Assoziation zwischen den Proteinen myc-CD95 und Flag/-FLIP (159L) gegeben ist, wenn die 293T-Zellen mit allen drei Expressionsvektoren stabil transfiziert worden sind. Dies ist in der rechten Spalte der Figur 5b zu erkennen. Dagegen zeigen die beiden viralen FLIP-Konstrukte (E8 oder 159L) dann keine Assoziation mit dem CD95-Rezeptor, wenn die Zellen nicht FADD-positiv sind (Figur 5a, dritte Spalte, Figur 5b, vierte Spalte). Umgekehrt verhindert das virale FLIP-Proteine die Assoziation von FADD mit myc-CD95 nicht (Figur 5a, vierte Spalte, Figur 5b, fünfte Spalte), da nach der Coimmunopräzipitation in diesem Fall sowohl FADD als auch myc-CD95 mit Hilfe der Western Blot-Technik detektiert werden können.

## 2. Experimentelles Ausführungsbeispiel

Ziel des zweiten experimentellen Ausführungsbeispiels war die Untersuchung der Inkorporation des FLIP-Proteins (in diesem Fall des E8-FLIP-Proteins) in den sogenannten DISC Komplex, der im Fall der Aktivierung der apoptotischen Signalkaskade mit dem zytoplasmatischen Teil des CD95-Rezeptors assoziiert ist. Mit dem E8-FLIP-Gen wurden stabil Raji-Klone transfiziert. Auch hierzu wurden Expressionsvektoren eingesetzt, wobei als Promotor ein SRα-Promotor verwendet wurde. Die stabil transfizierten Raji-Klone (RE8/11 und RE8/19) wurden mit Kontrollklonen verglichen, die nur mit dem Vektor, ohne Insertion des FLIP-Gens, transfiziert wurden. Diese tragen die Bezeichnung RCo/1 und RCo/3. Zum Nachweis der stabilen Transfektion wurde, analog zum ersten Ausführungsbeispiel, die Expression des transfizierten Gens durch Western Blot Analyse untersucht. In Figur 6a ist jeweils eine Bande des FLIP-Proteins bei den transfizierten Klonen RE8/11 und RE8/19 zu erkennen, dagegen zeigen die Klone RCo/1 und RCo/3, die Kontrollklone, keine Expression des E8-Proteins. Auch hier wurde eine Koimmunopräzipitation durchgeführt, und zwar mit anti-CD95 Antikörper. Die Koimmunopräzipitate wurden dann durch 2D-Gelelektrophorese unter denaturierenden Bedingungen (in der einen Achse SDS-PAGE und in der anderen Richtung durch isoelektrische Fokussierung) aufgetrennt. Mit einem anti-Flag Antikörper wurde der Blot behandelt, wobei dieser somit auch gleichzeitig gegen das virale FLIP-Protein gerichtet war.

In Figur 6b ist das Ergebnis dieses Versuchs dargestellt. Hierbei zeigt sich, daß nur im Falle der mit Flag-E8 stabil transfizierten Raji-Klone ein positives Signal für ein ungefähr 23 kD Protein bei einem pI-Wert von ca. 5,0 beobachtet werden konnte. Bei dem Kontrollklon ohne E8 Expression (oberes Bild in Figur 6b) ist kein entsprechendes Signal zu erkennen. Das E8-Flag-Konstrukt läßt einen pI-Wert von 5,0 und ein Molekulargewicht von ungefähr 23 kD erwarten.

## 3. Experimentelles Ausführungsbeispiel

In diesem experimentellen Ausführungsbeispiel wurde darauf abgestellt, zu zeigen, daß das E8-FLIP-Protein den konstitutiven Aufbau des DISC-Komplexes beeinflußt. Hierzu wurde die DISC-Bildung im einzelnen analysiert. Dies wurde vergleichsweise für den Kontrollklon RCo/3 und den mit Flag E8-FLIP stabil transfizierten Klon RE8/19 gezeigt (Figur 7a). Hierzu wurden mit 35S-markierte anti-Apo-1-Antikörper eingesetzt. Dieser Antikörper wirkt agonistisch auf dem Todesrezeptor Apo-1 (CD95). Er stammt von P.H. Krammer (DKFZ, Heidelberg, Deutschland). Im folgenden wurde das durch den oben bezeichneten Antikörper erhaltene Immunopräzipitat durch eine 2D-Gel-elektrophorese analysiert, und das autoradiographisch ausgewertet. Die angewandte Methode ist im übrigen beschrieben bei Kischkel et al., (EMBO Journal 14, 5579-5588 (1995)).

Außerdem wurde die proteolytische Aktivität gegen das FLICE-Protein im DISC-Komplex in mit E8 transfizierten Raji-Zellen und in Kontroll-Raji-Zellen untersucht. Hierzu wurden die Zellen entweder mit anti-Apo-1 Antikörper für fünf Minuten behandelt oder blieben unbehandelt. Mit dem anti-Apo-1 Antikörper wurde daraufhin eine Immunopräzipitation vorgenommen, und daraufhin die proteolytische Aktivität des Immunopräzipitats aus mit anti-Apo-1 Antikörper behandelten oder unbehandelten Zellen gegenüber FLICE überprüft. Hierzu wurde die Proteolyse in vitro translatierten, 35S-markierten FLICE-Proteins dadurch verursacht, daß es mit dem Immunopräzipitat inkubiert wurde. Daraufhin wurden die spezifischen Spaltungsprodukte des FLICE-Proteins (p43, p26, p17, p12 und p9) autoradiographisch auf einem SDS-Gel untersucht.

Im Ergebnis zeigen die Untersuchungen bei den Raji-Kontrollklonen und den mit E8 transfizierten Raji-Klonen charakteristische Unterschiede. Insbesondere fehlen die typishen DISC-Proteine CAP4 (= FLICE) und CAP3 (ein FLICE-Derivat) im DISC-Komplex der mit E8 transfizierten Raji-Klone.

Das heißt, daß ein ordnungsgemäßer Aufbau des DISC-Komplexes, wie bei den Kontrollzellen, in Anwesenheit des E8-FLIP-Proteins nicht mehr vorliegt (Figur 7a). Auch funktional ergibt sich ein deutlicher Unterschied hinsichtlich der Aktivität, mit der das FLICE-Protein gespalten wird, insofern als bei den mit anti-Apo-1 Antikörper behandelten RE8/19-Klonen im Unterschied zu den Kontrollklonen kaum noch die spezifischen Spaltungsprodukte des FLICE-Proteins nachweisbar sind (Figur 7b).

## 4. Experimentelles Ausführungsbeispiel

Zum weiteren Nachweis der Bedeutung des viralen FLIP-Proteins für die Inhibition der Apoptose wurde der Zelltod verschiedener Zellen durch unterschiedliche Agonisten in Abwesenheit und in Anwesenheit des viralen FLIP-Proteins untersucht. Hierzu wurde zunächst die Anzahl der apoptotischen Zellen in Abhängigkeit von der Konzentration der anti-Apo-1 Antikörper gemessen. Die Quantifizierung der apoptotischen Zellen erfolgte so, wie oben bereits erläutert. Auch hier wurden die apoptotischen Zellen der Kontrollklone RCo/1 und RCo/3 im Vergleich zu den mit E8-FLIPtransfizierten Raji B-Zellklonen untersucht. Die Induktion der apoptotischen Signalkaskade erfolgte durch agonistische anti-Apo-1 Antikörper (Figur 8a).

Außerdem wurden Zellextrakte von E8 Transfektanten (JE8/1, JE8/5, JE8/10 und JE8/13) und von Kontrollzellen (JCo/2, JCo/3 und JCo/4) Jurkat-Klonen analysiert. Hierzu wurde die Flag-E8-FLIP-Expression durch anti-Flag Antikörper auf Western Blots ermittelt. Wie oben beschrieben, wurde nach einer Inkubation von 3 Stunden bei 37" der CD95 L induzierte Zelltod (mit Hilfe von Überständen neuronaler Zellen) gemessen. Wie oben zu Figur 8b beschrieben, wurden die mit Propidiumjodid markierten Zellen im FACScan Durchflußzytometer im Hinblick auf ihre apoptotische Reaktion untersucht.

Als weiteres apoptosestimulierendes Agens wurde die Überexpression von CD95-Rezeptor in humanen, embryonalen Nierenzellen (293T-Zellen) ermittelt. Hierzu wurden 293T-Zell-Einzeltransfektanten mit einem für CD95 codierenden Expressionsvektor ebenso wie Doppeltransfektanten mit einem für E8 bzw. einem für CD95 codierenden Expressionsvektor hergestellt. Bei einer Überexpression von CD95 (2 μg) wird ein massiver Zelltod beobachtet. Hierzu wird die relative Menge von DNA Histonkomplexen, die in das Zytoplasma freigegeben werden, gemessen. Durch die Überexpression des CD95-Rezeptors unterliegen in einem typischen CD95- Transfektionsexperiment ungefähr 50 bis 90% der Zellen der Apoptose. Zum Vergleich wurde auch eine CD95-Einzeltransfektante mit dem Protease-Inhibitor z-VAD-fmk versetzt (25 μmol).

Im Ergebnis zeigen alle drei Versuche, daß nach einer Stimulation der apoptotischen Reaktion, etwa durch einen agonistischen anti-Apo-1 Antikörper (Figur 8a), durch CD95L oder durch Überexpression des CD95-Rezeptors, diese blockiert oder zumindest weitgehend reduziert werden kann, wenn die stimulierten Zellen zuvor mit viralem FLIP stabil transfiziert worden sind. In analogen Experimenten wurden Einzel- und Doppeltransfektanten von 293T-Zellen mit Expressionsvektoren für TRAMP (Bodmer et al., Immunity 6, 79-88, (1997)), mit jeweils unterschiedlichen Mengen von Expressionsvektoren für entweder E8-FLIP (EHV-2) oder 159L-FLIP (MCV) oder 71-FLIP (HVS), transfiziert. Zur Kontrolle wurden Scheintransfektanten (Mock) ohne TRAMP- oder FLIP-Proteinexpression gewählt. Hierbei wurden steigende Mengen von Expressionsvektoren für die FLIP-Proteine eingesetzt, während die Menge an TRAMP-Expressionsvektoren in allen Experimenten konstant gehalten wurde (3 μg). Im Ergebnis zeigen auch diese Versuche (zusammengefaßt in Figur 9), daß zunehmende Mengen von virales FLIP-Protein exprimierenden Vektoren das Maß der durch die Überexpression von TRAMP-Rezeptoren verursachten Apoptose deutlich verringern können.

## 5. Experimentelles Ausführungsbeispiel

In diesem Ausführungsbeispiel wurde untersucht, wann das virale FLIP-Protein im viralen Replikationszyklus exprimiert wird. Hierzu wurde das HVS-71-FLIP-Protein gewählt. Als Wirtszellen für die virale Infektion dienten dabei die OMK-Zellen (Owl Monkey Kidney) . In Form einer Northern Blot Analyse wurden die Transkription bei permisiven OMK-Zellen mit einer HVS-Infektion des Stammes C488 analysiert. Hierzu wurde eine mRNA-Bestimmung des viralen FLIP-Gens in den mit den HVS-Virus infizierten Zellen ein, zwei, drei oder vier Tage nach der Infektion vorgenommen. Als Kontrolle wurden nicht-virusinfizierte OMK-Zellen untersucht. Zwei weitere mRNA-Analysen wurden durchgeführt, einerseits mit HVS infizierten T-Zellen der Linie P-1079 und andererseits mit OMK-Zellen, die durch den HVS-Stamm A11 infiziert wurden. Für die letztgenannte Zellinie wurden die Proben vier Tage nach der Infektion mit dem Virusstamm in Form eines Northern Blots untersucht.

Im Ergebnis zeigt Figur 11a, daß die permisiven OMK-Zellen, die mit einem zytopathologischen HVS-Stamm (C488 oder A11) infiziert wurden, am vierten Tag nach der Infektion eine starke Präsenz eines 5kb mRNA-Fragments aufweisen. Dieses mRNA-Stück dient zur Translation des viralen FLIP-Proteins. Damit erscheint das FLIP-Transkript einen Tag vor der massiven zellulären Lyse. Dies ist aus Figur 11b zu entnehmen.

## 6. Experimentelles Ausführungsbeispiel

Zum Nachweis der Bindung von humanem FLIP$_S$ und FLIP$_L$ an CD95 über das Adaptormolekül FADD wurden Kotransfektions-Experimente in humanen embryonalen Nierenzellen (293 Zellen) unternommen, die konstitutiv SV40 large T Antigen exprimieren und somit eine verstärkte Proteinexpression von Expressionsvektoren mit einem SV40 Origin haben (293T Zellen).

Der Expressionsvektor pCR-3 von Invitrogen besitzt diese Eigenschaft. Daher wurden DNA-Fragmente, die für die humanen Proteine oder Proteinteile FLIP$_L$, FLIP$_S$ und FLIP$_P$ kodieren, für die Expression in 293T Zellen in eine modifizierte Version des Vektors pCR-3 kloniert, der diese Proteine oder Proteinteile mit einem N-terminalen Flag-Epitop versieht, während der für den zytoplasmatische Proteinteil von CD95 kodierende DNA-Bereich in einen analogen Expressionsvektor mit N-terminalem myc-Epitop inseriert wurde. Wie in Figur 12 gezeigt, wurden verschiedene Kombinationen von Expressionsvektoren für den zytoplasmatischen Teil von CD95, für FADD, für FLIP$_L$, FLIP$_S$ und FLIP$_P$ in 293T transfiziert, und die Expression der von den jeweiligen Expressionsvektoren kodierten Genprodukte im Western Blot mit spezifischem anti-Flag, anti-FADD oder anti-myc Antikörper kontrolliert. Ausserdem wurden die in Zellysaten von entsprechend transfizierten 293T Zellen vorhandenen Flag-markiertem FLIP$_L$, FLIP$_S$ oder FLIP$_P$ wie oben beschrieben mittels anti-Flag-Agarose immunpräzipitiert und diese Immunpräzipitate dann im anti-FADD oder anti-myc Western Blot auf die Assoziation von FADD oder dem myc-markierten CD95 Proteinteil analysiert.

## Ergebnis

Es wurde festgestellt, daß die humanen Proteine FLIP$_S$ und FLIP$_L$, nicht aber ein Proteinteil des humanen FLIP$_L$, der nur die caspase-homologe inaktive Proteasedomäne enthält (FLIP$_P$) an das Adaptormolekül FADD binden. Diese Bindung beeinträchtigt nicht die Anlagerung von FADD an den zytoplasmatischen Proteinteil des CD95 Todesrezeptors, und die humanen Proteine FLIP$_S$ und FLIP$_L$ können somit über den Adaptor FADD an den zytoplasmatischen Teil des Todesrezeptors CD95 binden.

## 7. Experimentelles Ausführungsbeispiel

Zum Nachweis der Bindung von humanem FLIP$_S$ und FLIP$_L$ an FLICE wurden Kotransfektions-Experimente in oben beschriebenen 293T Zellen vorgenommen (siehe Figur 13). Der Expressionsvektor für humanes FLICE (ein Geschenk von M. Peter, Heidelberg) versieht dieses Protein mit einem N-terminalen HA-Epitop, während die Expressionsvektoren für FLIP$_L$, FLIP$_S$ und FLIP$_P$ wie oben beschrieben diese Proteine oder Proteinteile mit einem N-terminalen Flag-Epitop versehen. Die Expression der von den jeweiligen Expressionsvektoren kodierten Genprodukte im Western Blot mit spezifischem anti-Flag oder anti HA-Antikörper kontrolliert. Die in Zellysaten von entsprechend transfizierten 293T Zellen vorhandenen Flag-markiertem FLIP$_L$, FLIP$_S$ oder FLIP$_P$ wurden mittels anti-Flag-Agarose immunpräzipitiert und diese Immunpräzipitate dann im anti-HA Western Blot auf die Assoziation von HA-FLICE mit FLIP$_L$, FLIP$_S$ oder FLIP$_P$ analysiert.

## Ergebnis

Es wurde festgestellt, daß die humanen Proteine FLIP$_S$ und FLIP$_L$ an die Caspase FLICE binden. Zur Bindung von FLIP$_L$ an FLICE tragen sowohl der in FLIP$_S$ enthaltene N-terminale Proteinteil mit den beiden Todeseffektordomänen als auch der C-terminale Proteinteil FLIP$_P$ bei, der die caspase-homologe inaktive Protease-Domäne enthält.

## 8. Experimentelles Ausführungsbeispiel

Um den inhibierenden Effekt von FLIP$_S$ und FLIP$_L$ auf die durch den Todesrezeptor CD95 induzierte Apoptose nachzuweisen, wurde eine humane Jurkat T-Zellinie und eine humane Raji B-Zellinie mit einem Expressionsvektor für mit N-terminalem VSV-Epitop versehenes humanes FLIP$_S$ oder FLIP$_L$ transfiziert. Für die stabile Transfektion dieser Zellen wurde ein Expressionsvektor mit SR$\alpha$-Promoter verwendet, der den transfizierten Zellen eine Puromycin-Resistenz verleiht (Der Vektor war ein Geschenk von R. Sekaly, ICRM, Montreal, Kanada). Die Zellen wurden wie oben beschrieben durch Elektroporation von $8 \cdot 10^6$ Zellen bei 250V und 960 µF in HeBS-Pufferlösung mit 20µg des zu transfizierenden Plasmides transfiziert und, nach 48h Wachstum in Kulturmedium ohne Puromycin, unter Selektion in 5µg/ml Puromycin enthaltendem Kulturmedium zu 2000-20000 Zellen pro Well in Flachboden-Zellkulturplatten mit 96 Wells ausgesäht. Puromycin-resistente Klone wuchsen innerhalb von 2-3 Wochen heran und wurden dann auf die Expression von VSV-markiertem FLIP$_S$ bzw. FLIP$_L$ im Western Blot getestet. Klone mit verschiedenen Expressionsniveaus des VSV-FLIP$_S$ bzw. VSV-FLIP$_L$ wurden dann auf ihre Resistenz gegen durch sFasL induzierte Apoptose getestet (siehe Figur 14). Dazu wurden die Klone mit den in Figur 14 angegebenen Konzentrationen von Flag-markiertem sFasL und 1µg/ml kreuzvernetzendem anti-Flag Antikörper im Kulturmedium für 20h bei 37°C inkubiert, und dann die Zellviabilität der so behandelten Zellen mit einem Zellproliferationsassy (Celltiter 96 AQ, Promega) bestimmt.

**Ergebnis**

Es wurde festgestellt, daß die humane T-Zellinie Jurkat und die humane B-Zellinie Raji durch die Expression des humanen $FLIP_S$ oder $FLIP_L$ eine Resistenz gegen die durch den CD95 Todesrezeptor induzierte Apoptose erwerben. Dabei bietet die Expression der längeren Form des humanen FLIP ($FLIP_L$) einen effizienteren Schutz gegen die durch CD95 induzierte Apoptose als die kürzere Form des humanen FLIP ($FLIP_S$), die nicht die caspase-homologe inaktive Protease-Domäne besitzt.

**9. Experimentelles Ausführungsbeispiel**

Um den inhibierenden Effekt von $FLIP_S$ und $FLIP_L$ auf die durch den Todesrezeptor-Liganden TRAIL induzierte Apoptose nachzuweisen, wurde eine humane Jurkat T-Zellinie wie im vorstenden Ausführungsbeispiel beschrieben mit einem Expressionsvektor für mit N-terminalem VSV-Epitop versehenes humanes $FLIP_S$ oder $FLIP_L$ transfiziert und Klone unter Puromycin-Selektion herangezogen, die dann auf die Expression von VSV-markiertem $FLIP_S$ bzw. $FLIP_L$ im Western Blot getestet wurden (siehe oberen Teil der Figur 14a). Klone mit verschiedenen Expressionsniveaus des VSV-$FLIP_S$ bzw. VSV-$FLIP_L$ wurden dann auf ihre Resistenz gegen durch TRAIL induzierte Apoptose getestet (siehe Figur 15). Dazu wurden die Klone mit den in Figur 15 angegebenen Konzentrationen von Flag-markiertem TRAIL und 1 µg/ml kreuzvernetzendem anti-Flag Antikörper im Kulturmedium für 20h bei 37°C inkubiert, und dann die Zellviabilität der so behandelten Zellen mit einem Zellproliferationsassy (Celltiter 96 AQ, Promega) bestimmt.

**Ergebnis**

Es wurde festgestellt, daß die humane T-Zellinie Jurkat durch die Expression des humanen $FLIP_S$ oder $FLIP_L$ eine Resistenz gegen die durch den Todesrezeptor-Liganden TRAIL induzierte Apoptose erwerben. Dabei bietet die Expression der längeren Form des humanen FLIP ($FLIP_L$) einen effizienteren Schutz gegen die durch TRAIL induzierte Apoptose als die kürzere Form des humanen FLIP ($FLIP_S$), die nicht die caspase-homologe inaktive Protease-Domäne besitzt.

Die Gewebehomöostase wird durch eine ausgewogene Balance zwischen Zellwachstum und Apoptose aufrechterhalten. Während die apoptotische Signaltransduktion für den Zelltod überflüssiger oder infizierter Zellen verantwortlich ist, gleicht das Zellwachstum etwaige Zellverluste aus. Bei zahlreichen Infektionskrankheiten oder bei Tumorerkrankungen ist diese Balance gestört. Tumorerkrankungen zeichnen sich durch eine ortsspezifische oder örtlich diffuse, beschleunigte Zellvermehrung von Zellen aus. Die Regulation der Zellteilung ist bei Tumorzellen ausgeschaltet.

Auch eine wirkungsvolle Apoptose findet im Fall von Tumorzellen nicht mehr statt. So etwa gibt es klare experimentelle Hinweise dafür, daß Tumorzellen, wie etwa Melanome oder Hepatome nicht mit dem Zelltod auf die Bindung von CD95L reagieren. Dies ist durch eine "Downregulation" der CD95-Expression oder durch eine Blockade im CD95-kontrollierten Signaltransduktionsweg möglich (Hahne, M. *et al.*, Science **274**, 1363-1366 (1996) (Strand, S. *et al.*, Nature Med. **2**, 1361-1366 (1996)).

Die Wirkung von viralen FLIP-Proteinen beruht auf einer Inhibition des apoptotischen Signaltransduktionsmechanismus, wie oben bereits beschrieben. Viren haben das vermutlich ursprünglich zelluläre Genmaterial in ihr Genom integriert, um der virenspezifischen Immunabwehr des Immunsystems zu entgehen. Durch die Präsenz des viralen FLIP-Proteins als Inhibitor der Apoptose ist die Immunabwehr außerstande, die virusinfizierte Immunzelle abzutöten und damit den Vermehrungszyklus des Virus zu unterbrechen. Die Integration des FLIP-Proteins in das virale Genom fördert somit die Virusverbreitung bzw. eine persistierende Infektion des Wirts.

Die Integration des Virus bzw. die Expression des FLIP-Proteins kann aber auch transformierende Wirkung haben. In diesem Zusammenhang ist interessant, daß gerade zahlreiche Herpesviren Transformationseigenschaften, d.h. Eigenschaften, die die normal regulierte Zelle zur Tumorzelle werden läßt, aufweisen. Erfindungsgemäß wurde durch die Identifizierung der inhibierenden viralen FLIP-Proteine somit ein entscheidender Beitrag zum Verständnis des Zusammenhangs von Transformationen und Tumorentstehung sowie viraler Vermehrung geleistet und hiermit offenbart.

Dem viralen FLIP-Protein kommt somit eine zentrale Bedeutung auch beim Verständnis der viralen Tumorigenese zu. Zahlreiche Daten bestätigen den Zusammenhang zwischen Viruspersistenz und Tumorigenese. So etwa produziert das Virus MCV epidermale Neoplasmen mit langsamem Wachstum, die lange Zeit der Immunabwehr entgehen können. Das Virus HVS verursacht Tumore in gewissen Primatenstämmen, und Hinweise aus epidemiologischen Studien zeigen an, daß auch das Virus HHV-8 als infektiöser Co-Faktor für das Kaposi Sarkom, das insbesondere bei AIDS-Patienten auftritt, sowie für gewisse Formen primärer Lymphome im Hinblick auf den Transformationsprozeß verantwortlich ist.

Interessanterweise haben alle γ-Herpesviren, die für ein FLIP-Protein codieren, auch ein bcl-2-Homolog. Die anti-apoptotischen bcl-2-Proteine blockieren allerdings den Zelltod, der durch äußere Einflüsse, wie etwa den Entzug von Wachstumsfaktoren, γ-Strahlung oder cytotoxische Substanzen ausgelöst wird. Im Unterschied zu den Genprodukten der erfindungsgemäßen viralen DNA-Sequenzen ist ihr antiapoptotischer Effekt bei Lymphozyten-Zellinien durch Stimulation des CD95-Rezeptors weniger stark ausgeprägt. Daher weisen die o.g. Viren zwei Gentypen auf, die komplementäre Eigenschaften besitzen, z.B. ein bcl-2-Homolog und ein vFLIP-Protein. Erfindungsgemäß kann daher durch die Kombination dieser beiden Gentypen, z.B. durch zwei verschiedene, diese Gentypen tragende Expressionsvektoren, eine gegenüber zahlreichen Umwelteinflüssen resistente antiapoptotische Eigenschaft der Zelle erreicht werden. Erfindungsgemäß ist auch denkbar, ein rekombinantes Protein mit wirksamen Domänen von bcl-2-Homologen und wirksamen Domänen von vFLIP-Proteinen zur Verfügung zu stellen.

Während die erfindungsgemäßen Gene oder Genprodukte zum Immortalisierungsprozeß von Tumorzellen wesentlich beitragen und damit als tumorigenes Agens wirken, kann umgekehrt mit Hilfe der erfindungsgemäßen Sequenzen oder von deren erfindungsgemäßen Genprodukten gewünschtenfalls ein Immortalisierungsprozeß ausgelöst werden. Insbesondere trifft dies für Erkrankungen zu, die, bedingt durch eine nicht regulierte Auslösung des Apoptosemechanismus, zu einem gehäuften oder auch massenhaften Zelluntergang führen. In diesem Zusammenhang muß insbesondere an Autoimmunkrankheiten gedacht werden (z.B. rheumatoide Arthritis oder Lupus Erythematosus oder Multiple Sklerose). Ein entsprechender Einsatz der erfindungsgemäßen DNA-Sequenzen, von deren erfindungsgemäßen Genprodukten, den abgeleiteten Expressionsvektoren bzw. den mit den Expressionsvektoren transformierten Wirtszellen könnte diesen Zelltod verhindern.

Insbesondere ist an eine Immortalisierung der durch die Autoimmunerkrankung betroffenen Zellen zu denken. In Form gentherapeutischer Verfahren könnten diese Zellen mit den erfindungsgemäßen DNA-Sequenzen transfiziert werden, z.B. auch *in vitro*, und diese dann retransplantiert werden.

Auch im Falle von HIV-Infektionen kommt ein Einsatz der erfindungsgemäßen Gegenstände zum Tragen. FLIP-Proteine mit ihrem Potential zur Inhibition des apoptotischen Signaltransduktionsmechanismus könnten dabei, vorzugsweise *ex vivo*, in Zellen, insbesondere in T-Zellen, integriert werden und somit - nach Retransplantation der Zellen - nach der extrazellulären Stimulierung *in vivo* am Absterben gehindert werden. Diese Zellen könnten dann *in vivo* unbegrenzt ihre Lebensfähigkeit erhalten und ihre immunologischen Funktionen wahrnehmen. Im Falle von HIV-Infektionen etwa könnten so z.B. die Zellen der Immunabwehr vor dem massenhaften Zelluntergang bewahrt werden.

Zu denken ist aber auch an den Einsatz in Labormaßstab. In der labormedizinischen und/oder biomedizinischen Fachwelt tritt seit Jahrzehnten das Problem auf, daß gewisse Zellinien sich nicht über mehrere Generationen im Labor züchten lassen. Die begrenzte Zellteilung gewisser Zellinien erlaubt keine eingehende Untersuchung der zellulären oder physiologischen Eigenschaften *in vitro*. Durch eine Transfektion von Zellinien mit den erfindungsgemäßen DNA-Sequenzen kann die Immortalität der Zellinien erreicht werden. Damit liegt ein wesentliches Anwendungsgebiet der vorliegenden Erfindung auf dem Gebiet der laborexperimentellen oder klinisch-experimentellen Anwendung, wobei ein entscheidender Beitrag zur Vereinfachung der Zellzüchtungsmethoden zu erwarten ist.

**Patentansprüche**

1. Eukaryotische DNA-Sequenz, **dadurch gekennzeichnet, daß** sie für ein physiologisch die Zellapoptose inhibierendes Genprodukt oder einen die Zellapoptose inhibierenden Abschnitt eines solchen Genprodukts mit mindestens einer Todeseffektordomäne codiert, einschließlich aller funktionshomologen Derivate der eukaryotischen DNA-Sequenz oder aller funktionshomologen Allele der eukaryotischen DNA-Sequenz.

2. DNA-Sequenz nach Anspruch 1, **dadurch gekennzeichnet, daß** sich ein Signifikanzniveau von $p<10^{-2}$ ergibt, wenn die Sequenz der Todeseffektordomäne mit einem Suchprofil nach Figur 1a oder einem Suchprofil nach Figur 1b verglichen wird, wobei die Figuren 1a und 1b Bestandteil dieses Anspruchs sind.

3. DNA-Sequenz nach Anspruch 1 oder 2, **dadurch gekennzeichnet, daß** deren Genprodukt zwei Todeseffektordomänen aufweist.

4. DNA-Sequenz nach einem der vorgenannten Ansprüche, **dadurch gekennzeichnet, daß** deren Genprodukt an ein Protein des Apoptose-Signaltransduktionsweges bindet.

5. DNA-Sequenz nach einem der vorgenannten Ansprüche, **dadurch gekennzeichnet, daß** deren Genprodukt an den cytoplasmatischen Abschnitt eines membranständigen, zellulären Rezeptors des Apoptose-Signaltransduktionsweges bindet.

6. DNA-Sequenz nach Anspruch 5, **dadurch gekennzeichnet, daß** deren Genprodukt an einen Rezeptor der Klasse der TNF-Rezeptoren bindet.

7. DNA-Sequenz nach einem der Ansprüche 1-4, **dadurch gekennzeichnet, daß** deren Genprodukt an lösliche, intrazelluläre Proteine des Apoptose-Signaltransduktionswegs bindet.

8. DNA-Sequenz nach einem der vorgenannten Ansprüche, **dadurch gekennzeichnet, daß** deren Genprodukt an eine Protease des caspase-Typs bindet.

9. DNA-Sequenz nach einem der vorgenannten Ansprüche, **dadurch gekennzeichnet, daß** deren Genprodukt an ein Protein des "DISC"-Komplexes bindet.

10. DNA-Sequenz nach einem der vorgenannten Ansprüche, **dadurch gekennzeichnet, daß** deren Genprodukt an das Protein FLICE bindet.

11. DNA-Sequenz nach einem der vorgenannten Ansprüche, **dadurch gekennzeichnet, daß** sie für Splice-Varianten eines Genprodukts codiert.

12. DNA-Sequenz nach einem der vorgenannten Ansprüche, **dadurch gekennzeichnet, daß** die DNA-Sequenz für ein Genprodukt codiert, das eine der Todeseffektordomänen-Aminosäuresequenzen FLIP_HS/DED1, FLIP_MM/DED1, FLIP_HS/DED2 oder FLIP_MM/DED2 nach Figur 3 enthält, wobei Figur 3 Bestandteil dieses Anspruchs ist.

13. DNA-Sequenz nach einem der vorgenannten Ansprüche, **dadurch gekennzeichnet, daß** deren Genprodukt zwei Todeseffektordomänen und eine caspase-homologe Domäne aufweist, die infunktionell ist.

14. DNA-Sequenz nach einem der vorgenannten Ansprüche, **dadurch gekennzeichnet, daß** sie in der Erbinformation von Säugetierzellen auftritt.

15. DNA-Sequenz nach Anspruch 14, **dadurch gekennzeichnet, daß** deren Genprodukt eine Aminosäuresequenz nach einer der Figuren 4a, 4b oder 4c enthält, wobei die Figuren 4a, 4b und 4c Bestandteil dieses Anspruchs sind.

16. DNA-Sequenz nach Anspruch 14 oder 15, **dadurch gekennzeichnet, daß** sie eine Nukleotidsequenz nach einer der Figuren 4a, 4b oder 4c enthält, wobei die Figuren 4a, 4b und 4c Bestandteil dieses Anspruchs sind.

17. DNA-Sequenz nach einem der Ansprüche 1 bis 16, **dadurch gekennzeichnet, daß** sie einen operabel mit der beanspruchten DNA-Sequenz verbundenen Promotor umfaßt.

18. DNA-Sequenz nach Anspruch 17, **dadurch gekennzeichnet, daß** sie weitere Steuerungselemente zur Transkription und/oder Translation umfaßt.

19. Expressionsvektor, **dadurch gekennzeichnet, daß** er eine DNA-Sequenz nach einem der Ansprüche 1 bis 18 enthält.

20. Verfahren zur Isolierung von Genprodukten mit mindestens einer Todeseffektordomäne, **dadurch gekennzeichnet, daß** Wirtszellen mit einem Expressionsvektor nach Anspruch 19 transformiert und unter geeigneten, die Expression fördernden Bedingungen kultiviert werden und das Genprodukt schließlich aus der Kultur aufgereinigt wird.

21. Verfahren zur Expression von Genprodukten mit mindestens einer Todeseffektordomäne, **dadurch gekennzeichnet, daß** Wirtszellen mit einem Expressionsvektor nach Anspruch 19 transformiert werden.

22. Wirtszelle, **dadurch gekennzeichnet, daß** sie mit einem Expressionsvektor nach Anspruch 19 transformiert ist.

23. Wirtszelle nach Anspruch 22, **dadurch gekennzeichnet, daß** sie eine Säugetierzelle ist.

24. Wirtszelle nach Anspruch 22 oder 23, **dadurch gekennzeichnet, daß** sie eine humane Zelle ist.

25. Wirtszelle nach Anspruch 24, **dadurch gekennzeichnet, daß** sie eine Zelle des Immunsystems, insbesondere ein T-oder B-Lymphozyt, ist.

26. Gereinigtes Genprodukt oder Abschnitt dieses Genprodukts, **dadurch gekennzeichnet, daß** es (er) durch eine eukaryotische DNA-Sequenz nach einem der Ansprüche 1 bis 18 codiert wird.

27. Gereinigtes Genprodukt oder Abschnitt dieses Genprodukts nach Anspruch 26, **dadurch gekennzeichnet, daß** es (er) ein RNA-Molekül ist.

28. Gereinigtes Genprodukt oder Abschnitt dieses Genprodukts nach Anspruch 26, **dadurch gekennzeichnet, daß** es (er) ein Protein ist.

29. Gereinigtes Genprodukt oder Abschnitt dieses Genprodukts nach Anspruch 28, **dadurch gekennzeichnet, daß** es (er) chemisch modifiziert ist, insbesondere durch Schutzgruppen am N-Terminus, durch Glykosylgruppen an Hydroxyl- oder Aminogruppen, durch Lipid-, Phosphat- oder Peptidgruppen.

30. Verwendung einer DNA-Sequenz nach einem der Ansprüche 1 bis 18, eines Expressionsvektors nach Anspruch 19 oder eines Genprodukts nach einem der Ansprüche 26 bis 29 zur Apoptose-Inhibition oder zur Immortalisierung von Zellen.

31. Verwendung einer DNA-Sequenz nach einem der Ansprüche 1 bis 18, eines Expressionsvektors nach Anspruch 19, einer Wirtszelle nach einem der Ansprüche 22 bis 25 oder eines Genprodukts nach einem der Ansprüche 26 bis 29 zur Bekämpfung von Erkrankungen, die zu gehäuftem oder massenhaftem Zelluntergang führen, insbesondere Autoimmunkrankheiten oder HIV-Infektionen.

32. Verwendung einer DNA-Sequenz nach einem der Ansprüche 1 bis 18 als Probenreagens.

33. Verwendung eines "anti-sense"-Oligonucleotids einer DNA-Sequenz nach einem der Ansprüche 1 bis 18 zur intrazellulären Blockierung der Expression von Genprodukt, für das eine DNA-Sequenz nach einem der Ansprüche 1 bis 18 codiert.

34. Verfahren zur in vitro Kultivierung von Zellinien, **dadurch gekennzeichnet, daß** zu kultivierende Zellen mit einer DNA-Sequenz nach einem der Ansprüche 1 bis 18 transfiziert werden.

ID  MATRIX.

AC ZZ99999;

DE Based on FADD, FLICE and Mch4.

MA /GENERAL_SPEC: ALPHABET='ABCDEFGHIKLMNPQRSTVWYZ'; LENGTH=79;

MA /DISJOINT: DEFINITION=PROTECT; N1=6; N2=74;

MA /NORMALIZATION: MODE=1; FUNCTION=LINEAR; R1=.8551; R2=.01636739; TEXT='NScore';

MA /CUT_OFF: LEVEL=0; SCORE=467; N_SCORE=8.5; MODE=1;

MA /CUT_OFF: LEVEL=-1; SCORE=344; N_SCORE=6.5; MODE=1;

MA /DEFAULT: D=-20; I=-20; B1=-50; E1=-50; MI=-105; MD=-105; IM=-105; DM=-105;

MA /I: B1=0; BI=-105; BD=-105;

MA /M: SY='P'; M=3,-5,-23,-1,-3,-20,-13,-15,-13,-12,-10,-12,-8,12,-8,-16,1,-3,-13,-30,-19,-7;

MA /M: SY='F'; M=-20,-28,-22,-36,-28,71,-30,-13,0,-26,8,0,-20,-30,-34,-18,-20,-10,-2,14,39,-28;

MA /M: SY='R'; M=-12,-14,-24,-14,-5,-12,-19,-7,-15,8,-3,-4,-6,-21,1,33,-7,-5,-11,-23,-9,-5;

MA /M: SY='V'; M=-7,-5,-19,-7,-2,-13,-20,-11,-1,-2,-9,-5,-1,-21,-8,3,-4,-4,7,-30,-14,-6;

MA /M: SY='L'; M=-10,-11,-22,-16,-11,-4,-21,-9,4,-6,16,15,-8,-23,-7,-6,-18,-8,-2,-23,-5,-9;

MA /M: SY='L'; M=-10,-30,-20,-30,-20,10,-30,-20,20,-30,50,20,-30,-30,-20,-20,-30,-10,10,-20,0,-20;

MA /M: SY='Y'; M=-18,-17,-28,-17,-15,13,-27,33,-4,-14,4,4,-14,-27,-7,-9,-19,-13,-11,5,49,-15;

MA /M: SY='E'; M=-6,5,-25,8,14,-25,-17,-6,-12,-4,-17,-11,2,-10,11,-8,7,-2,-14,-31,-14,12;

MA /M: SY='T'; M=-9,-30,-24,-35,-27,3,-35,-27,37,-29,29,19,-25,-25,-21,-25,-22,-9,26,-21,-1,-27;

Fig. 1a

MA /M: SY='S'; M=3,8,-17,11,0,-25,10,-10,-27,-10,-30,-22,10,-12,-3,-12,26,8,-17,-37,-22,-1;

MA /M: SY='E'; M=-2,4,-24,10,31,-27,-2,-6,-28,0,-24,-20,3,-6,8,-6,13,-2,-24,-32,-21,20;

MA /M: SY='N'; M=-3,7,-23,5,10,-27,7,-3,-26,-3,-26,-17,14,-12,10,-5,12,-2,-25,-31,-20,10;

MA /M: SY='L'; M=-8,-30,-20,-32,-24,6,-32,-24,28,-28,37,18,-28,-28,-22,-22,-24,-8,21,-22,-2,-24;

MA /M: SY='D'; M=-3,17,-21,24,4,-29,9,-8,-31,-8,-30,-24,12,-12,-4,-12,18,2,-21,-36,-22,0;

MA /M: SY='S'; M=1,-9,-16,-9,-8,-18,0,-13,-15,-6,-21,-13,-1,-17,-6,2,16,5,-3,-32,-18,-8;

MA /M: SY='E'; M=-4,9,-22,10,26,-27,-11,0,-24,2,-24,-16,11,-8,19,-1,14,1,-24,-33,-18,22;

MA /M: SY='D'; M=-15,35,-28,44,29,-33,-11,2,-33,3,-27,-25,22,-9,6,-5,2,-8,-30,-37,-20,17;

MA /M: SY='L'; M=-8,-30,-18,-30,-22,8,-30,-22,22,-28,43,18,-30,-30,-22,-20,-26,-8,17,-22,-2,-22;

MA /M: SY='K'; M=-1,-4,-23,-7,8,-20,-17,-8,-18,13,-14,-3,-4,-11,5,13,-2,-1,-12,-23,-12,5;

MA /M: SY='D'; M=-5,20,-21,23,20,-27,-7,-2,-27,-1,-27,-22,19,-9,5,-6,16,2,-23,-37,-20,12;

MA /M: SY='L'; M=-12,-28,-20,-32,-22,21,-28,-16,16,-26,37,24,-26,-28,-20,-18,-26,-10,8,-14,6,-20;

MA /M: SY='K'; M=-10,-6,-30,-8,2,-24,-24,-14,-14,34,-20,-4,-4,-12,4,18,-12,-10,-10,-20,-8,2;

MA /M: SY='F'; M=-20,-30,-20,-40,-30,80,-30,-20,0,-30,10,0,-20,-30,-40,-20,-20,-10,0,10,30,-30;

MA /M: SY='L'; M=-10,-30,-20,-30,-20,10,-30,-20,20,-30,50,20,-30,-30,-20,-20,-30,-10,10,-20,0,-20;

MA /M: SY='C'; M=-7,-21,44,-25,-21,-8,-25,-23,-9,-27,5,-5,-19,-31,-21,-23,-9,-5,-2,-37,-17,-21;

MA /M: SY='L'; M=-11,-16,-27,-18,-6,-13,-27,-12,2,1,6,6,-13,-19,5,6,-16,-10,-4,-20,-5,-2;

MA /M: SY='D'; M=-11,18,-30,29,20,-34,12,-6,-37,-3,-27,-24,7,-10,0,-10,0,-13,-30,-31,-23,10;

MA /M: SY='R'; M=-10,-10,-24,-8,4,-8,-19,-2,-14,0,-6,-7,-7,-18,1,11,-3,-4,-13,-18,3,0;

MA /M: SY=T; M=-6,-30,-19,-34,-28,2,-34,-28,35,-26,22,16,-26,-26,-24,-24,-18,-6,35,-24,-4,-28;

MA /M: SY='P'; M=-2,-12,-29,-7,-3,-27,-2,-17,-23,-11,-30,-20,-8,44,-8,-17,7,-2,-24,-32,-27,-8;

MA /M: SY='K'; M=-10,7,-28,4,10,-30,-16,-3,-26,34,-28,-10,11,-12,16,21,-5,-8,-24,-24,-12,13;

MA /I: I=-5; MI=-25; MD=-25; IM=-25;

MA /M: SY='R'; M=-12,-8,4,-10,-4,-18,-18,-7,-24,17,-18,-10,-4,-18,1,30,-8,-8,-14,-22,-12,-4; D=-5;

MA /I: I=-5; MI=-25; MD=-25; IM=-25; DM=-25;

MA /M: SY='K'; M=-8,0,-24,0,8,-24,-16,-8,-24,40,-24,-8,0,-8,8,24,-8,-8,-16,-16,-8,8; D=-5;

MA /I: I=-5; MI=-25; MD=-25; IM=-25; DM=-25;

MA /M: SY='L'; M=-8,-19,-18,-19,-9,0,-22,-11,9,-17,28,13,-19,-21,-3,-11,-19,-8,1,-16,-2,-6; D=-5;

MA /I: I=-5; MI=-25; MD=-25; IM=-25; DM=-25;

MA /M: SY='E'; M=-10,15,-24,25,41,-26,-14,0,-26,6,-18,-18,4,-2,12,-2,0,-8,-24,-26,-16,26; D=-5;

MA /I: I=-5; MI=-25; IM=-25; DM=-25;

MA /M: SY='R'; M=-12,3,-28,7,4,-25,-18,-9,-26,13,-23,-15,0,4,1,17,-2,2,-19,-27,-15,0;

MA /M: SY='V'; M=-6,-2,-21,3,3,-17,-20,-15,-1,-10,-9,-7,-6,-15,-7,-14,2,-2,5,-32,-14,-3;

MA /M: SY='M'; M=-6,-8,-22,-12,-1,-19,-16,-1,-6,5,-8,18,-6,-14,17,2,-2,-4,-9,-24,-8,8;

MA /M: SY='S'; M=-1,16,-15,13,1,-21,-6,-7,-21,-6,-26,-20,19,-12,-2,-8,24,17,-15,-38,-18,0;

MA /M: SY='A'; M=18,-12,-17,-15,-12,-17,13,-18,-14,-16,-8,-9,-7,-16,-12,-18,6,-3,-8,-24,-19,-12;

MA /M: SY='L'; M=-6,-24,-18,-24,-16,4,-24,-18,13,-26,35,13,-23,-26,-16,-18,-17,-4,6,-24,-4,-16;

MA /M: SY='D'; M=-12,28,-24,39,9,-29,-10,-2,-26,-4,-22,-13,11,-12,0,-10,5,-4,-19,-37,-17,5;

MA /M: SY='L'; M=-10,-30,-20,-34,-26,20,-32,-24,23,-28,29,14,-26,-28,-26,-22,-22,-8,19,-16,4,-26;

MA /M: SY='F'; M=-18,-30,-20,-38,-28,66,-30,-20,4,-30,18,4,-22,-30,-36,-20,-22,-10,2,4,24,-28;

MA /M: SY='Q'; M=6,-6,-21,-9,6,-20,-17,-12,-6,-7,-11,-6,-4,-10,7,-11,6,4,-6,-26,-13,5;

MA /M: SY='H'; M=-14,-12,-24,-14,-1,3,-23,11,-10,-5,-5,4,-9,-20,-5,4,-11,-11,-7,-19,3,-4;

MA /M: SY='L'; M=-10,-28,-20,-30,-20,8,-28,-16,20,-26,44,27,-28,-28,-16,-18,-28,-10,10,-20,0,-18;

MA /M: SY='E'; M=-10,-10,-26,-6,18,-14,-24,-7,-6,-8,11,1,-13,-15,9,-7,-13,-10,-12,-24,-9,13;

MA /M: SY='E'; M=1,2,-26,5,28,-28,-16,-8,-26,22,-22,-14,-2,-6,11,8,-2,-8,-21,-24,-16,19;

MA /M: SY='Q'; M=-12,0,-30,2,22,-33,-20,3,-25,20,-22,-7,0,-10,35,23,-4,-10,-26,-22,-12,28;

MA /M: SY='N'; M=-10,11,-24,9,-6,-22,3,-7,-20,-3,-21,-15,14,-20,-8,2,-1,-8,-13,-31,-18,-8;

MA /M: SY='M'; M=-13,-1,-26,0,-5,-15,-23,-11,0,-3,2,8,-7,-17,-5,-8,-15,-10,-3,-25,-7,-6;

MA /M: SY='L'; M=-10,-30,-22,-32,-22,8,-32,-22,26,-30,44,20,-28,-28,-20,-22,-28,-10,14,-20,0,-22;

MA /M: SY='E'; M=-6,12,-26,21,26,-30,2,-6,-32,-1,-26,-22,6,-8,5,-8,7,-6,-26,-32,-22,15;

MA /M: SY='E'; M=-11,3,-31,12,44,-29,-20,-3,-29,10,-21,-19,-3,9,15,7,-3,-10,-29,-29,-20,28;

MA /M: SY='G'; M=-4,7,-27,13,6,-30,26,-11,-35,-9,-28,-22,6,-13,-5,-13,7,-9,-27,-29,-24,1;

MA /M: SY='H'; M=-14,24,-26,20,6,-26,-11,28,-28,7,-27,-15,28,-16,4,4,-1,-9,-28,-34,-8,4;

MA /M: SY='L'; M=-7,-20,-21,-21,-14,-3,-26,-20,5,-21,19,5,-20,-2,-16,-17,-13,6,0,-24,-8,-16;

MA /M: SY='S'; M=-7,4,-21,5,1,-6,-5,-12,-22,-11,-17,-16,2,-14,-9,-12,7,6,-15,-25,-10,-3;

MA /M: SY='F'; M=-14,-28,6,-35,-27,27,-32,-24,8,-30,16,5,-23,-30,-29,-24,-21,-10,6,-15,5,-27;

MA /M: SY='L'; M=-10,-30,-20,-30,-20,10,-30,-20,20,-30,50,20,-30,-30,-20,-20,-30,-10,10,-20,0,-20;

MA /M: SY='K'; M=-1,-2,-26,-2,14,-26,-16,-7,-26,26,-22,-12,-2,-11,8,25,-4,-8,-18,-22,-14,10;

MA /M: SY='E'; M=-14,14,-30,24,41,-30,-18,0,-32,12,-22,-20,4,-6,14,11,-2,-10,-28,-30,-18,27;

MA /M: SY='L'; M=-8,-30,-18,-30,-22,8,-30,-22,22,-28,43,18,-30,-30,-22,-20,-26,-8,17,-22,-2,-22;

MA /M: SY='C'; M=-10,-26,34,-30,-24,-2,-30,-24,1,-30,23,5,-26,-34,-24,-24,-22,-10,2,-32,-12,-24;

MA /M: SY='A'; M=13,-13,-19,-19,-11,4,-15,-11,-11,-1,-9,-6,-12,-17,-11,-8,-5,-6,-6,-6,9,-11;

MA /M: SY='T'; M=-4,-7,-21,-11,-4,-18,-19,-10,-7,-3,-13,-5,-1,-14,7,3,9,10,-6,-27,-11,0;

MA /M: SY='T'; M=-8,-30,-23,-35,-27,3,-35,-27,38,-28,26,18,-25,-25,-22,-25,-21,-8,29,-22,-2,-27;

MA /M: SY='R'; M=-12,4,-22,-3,-6,-16,-15,-3,-14,9,-17,-9,15,-22,-2,27,-3,-4,-9,-29,-14,-6;

MA /M: SY='R'; M=-14,-8,-32,-6,6,-28,-20,-4,-26,22,-24,-10,-4,6,15,33,-8,-10,-24,-22,-14,7;

MA /I: I=-6; MI=-32; MD=-32; IM=-32;

MA /M: SY='H'; M=-9,-5,-17,-5,-1,-9,-14,21,-10,2,-3,1,-2,-12,1,2,-9,-9,-10,-15,3,-1; D=-6;

MA /I: I=-6; MI=-32; IM=-32; DM=-32;

MA /M: SY='D'; M=-11,21,-26,30,12,-32,-12,-6,-32,18,-30,-20,10,-10,4,6,4,-4,-22,-32,-16,8;

MA /M: SY='L'; M=-10,-30,-20,-30,-20,10,-30,-20,20,-30,50,20,-30,-30,-20,-20,-30,-10,10,-20,0,-20;

MA /M: SY='L'; M=-10,-30,-20,-30,-20,10,-30,-20,20,-30,50,20,-30,-30,-20,-20,-30,-10,10,-20,0,-20;

MA /M: SY='R'; M=-13,-8,-30,-10,3,-25,-23,-4,-14,17,-15,-2,-3,-15,21,27,-8,-10,-15,-20,-8,10;

MA /M: SY='H'; M=-13,0,-25,-8,-7,-15,-20,12,-10,-1,-13,-5,10,-18,-1,11,-3,0,-11,-28,-5,-7;

MA /M: SY='T'; M=-10,-28,-24,-32,-25,8,-34,-19,30,-25,25,15,-24,-26,-20,-22,-22,-9,20,-13,12,-25;

MA /M: SY='B'; M=-13,25,-25,25,14,-22,-12,0,-21,-3,-15,-16,23,-15,1,-6,-1,-6,-23,-35,-17,7;

MA /M: SY='D'; M=-14,25,0,29,5,-31,-14,-6,-33,4,-28,-22,15,-17,-4,-4,-2,-8,-24,-38,-20,1;

MA /I: E1=0; IE=-105; DE=-105;

//

ID  MATRIX.

AC ZZ99999;

DE Based on FADD, FLICE, Mch4 and viral inhibitors.

MA /GENERAL_SPEC: ALPHABET='ABCDEFGHIKLMNPQRSTVWYZ'; LENGTH=78;

MA /DISJOINT: DEFINITION=PROTECT; N1=6; N2=73;

MA /NORMALIZATION: MODE=1; FUNCTION=LINEAR; R1=.8310; R2=.02233434; TEXT='NScore';

MA /CUT_OFF: LEVEL=0; SCORE=343; N_SCORE=8.5; MODE=1;

MA /CUT_OFF: LEVEL=-1; SCORE=253; N_SCORE=6.5; MODE=1;

MA /DEFAULT: D=-20; I=-20; B1=-50; E1=-50; MI=-105; MD=-105; IM=-105; DM=-105; MM=1; M0=-1;

MA /I: B1=0; BI=-105; BD=-105;

MA /M: SY='P'; M=-3,-7,-26,-4,1,-21,-18,-13,-11,-7,-13,-7,-9,15,-3,-10,-3,-3,-12,-28,-17,-3;

MA /M: SY='Y'; M=-14,-18,-23,-21,-17,27,-25,3,-5,-17,-1,-3,-14,-20,-16,-13,-9,-1,-7,2,33,-18;

MA /M: SY='R'; M=-13,-12,-26,-13,-6,-11,-15,-1,-18,9,-10,-4,-6,-21,1,24,-10,-9,-12,-18,-3,-5;

MA /M: SY='T'; M=-8,-7,-14,-7,-3,-12,-22,-11,-8,-7,-4,-5,-7,-14,-5,-6,-4,1,-5,-26,-7,-4;

MA /M: SY='L'; M=-7,-16,-18,-20,-14,3,-24,-16,5,-16,14,8,-14,-22,-12,-12,-9,4,5,-22,-3,-13;

MA /M: SY='L'; M=-9,-28,-19,-31,-23,9,-29,-19,23,-25,35,24,-27,-28,-19,-19,-24,-8,18,-21,-1,-21;

MA /M: SY='Y'; M=-6,-20,-15,-22,-16,2,-25,-4,2,-14,7,4,-17,-25,-10,-8,-14,-8,1,-14,9,-14;

MA /M: SY='H'; M=-4,9,-16,9,6,-23,-13,10,-20,-7,-18,-12,8,-15,3,-9,3,-6,-18,-32,-11,3;

MA /M: SY='T'; M=-6,-28,-20,-33,-26,2,-33,-27,33,-26,23,15,-24,-25,-22,-23,-17,-3,29,-24,-4,-26;

MA /M: SY='N'; M=-2,6,-20,4,-3,-17,2,-8,-19,-10,-17,-14,10,-16,-6,-11,8,4,-16,-28,-12,-5;

Fig. 16

MA /M: SY='E'; M=-7,12,-24,12,14,-21,-5,-3,-25,-1,-22,-17,13,-12,4,-2,5,-3,-23,-29,-16,9;

MA /M: SY='N'; M=-7,9,-23,6,13,-18,-7,3,-21,0,-22,-12,15,-12,6,-3,7,-3,-22,-30,-14,9;

MA /M: SY='L'; M=-9,-30,-21,-33,-24,11,-32,-23,26,-29,37,18,-27,-28,-22,-22,-25,-9,18,-19,1,-24;

MA /M: SY='D'; M=-6,17,-14,21,0,-27,2,-10,-29,-9,-26,-22,11,-14,-7,-12,11,6,-19,-35,-20,-3;

MA /M: SY='S'; M=4,3,-18,5,2,-22,-7,-10,-18,-6,-16,-14,1,-13,-1,-7,11,3,-10,-31,-17,0;

MA /M: SY='E'; M=-8,12,-25,16,25,-28,-13,3,-28,9,-25,-18,10,-8,12,5,6,-4,-24,-32,-16,18;

MA /I: MD=-29;

MA /M: SY='D'; M=-15,31,-29,44,37,-34,-14,1,-34,5,-25,-25,13,-6,9,-5,1,-9,-30,-35,-20,23;

MA /I: I=-8; MI=-10; MD=-29; IM=-10; DM=-29;

MA /M: SY='L'; M=-9,-18,-20,-18,-12,-2,-26,-12,9,-18,21,11,-18,-23,-12,-12,-16,-3,6,-24,-4,-13;

MA /I: DM=-29;

MA /M: SY='E'; M=-10,-5,-27,-4,15,-14,-21,-5,-18,10,-14,-8,-4,-12,5,14,-5,-6,-16,-20,-5,9;

MA /M: SY='S'; M=-1,-4,-9,-4,0,-18,-16,-12,-10,-7,-13,-9,-3,-17,-3,-6,6,3,-2,-32,-16,-2;

MA /M: SY='L'; M=-11,-27,-21,-31,-22,12,-28,-14,20,-24,34,26,-26,-27,-17,-18,-25,-9,11,-16,6,-20;

MA /M: SY='K'; M=-12,-15,-10,-17,-10,-10,-27,-13,-7,6,-2,0,-13,-22,-7,5,-16,-10,-6,-19,0,-10;

MA /M: SY='F'; M=-17,-27,-20,-35,-26,62,-28,-17,1,-27,11,1,-19,-29,-33,-19,-17,-8,0,4,26,-26;

MA /M: SY='L'; M=-6,-28,-12,-31,-22,9,-29,-22,17,-29,36,14,-26,-28,-21,-22,-24,-9,10,-20,-2,-22;

MA /M: SY='C'; M=0,-19,37,-25,-20,-10,-23,-23,-7,-25,2,-5,-17,-28,-20,-23,-6,-4,-1,-35,-17,-20;

MA /M: SY='R'; M=-9,2,-19,-1,-2,-21,-13,-2,-18,6,-14,-5,4,-18,2,7,-7,-9,-16,-27,-12,-1;

MA /M: SY='D'; M=-11,16,-29,23,14,-31,-2,-5,-29,0,-26,-20,9,-2,2,-7,-1,-10,-25,-31,-21,7;

EP 1 258 529 A2

Forts. Fig. 16

MA /M: SY='Y'; M=-10,-13,-13,-14,-10,5,-23,-9,-7,-12,3,-3,-13,-23,-12,-5,-10,-4,-5,-15,7,-12;

MA /M: SY='T'; M=-4,-24,-22,-32,-24,4,-30,-24,29,-25,20,13,-18,-23,-20,-23,-17,-7,20,-20,-2,-24;

MA /M: SY='P'; M=-7,-14,-35,-7,1,-29,-5,-17,-24,-7,-29,-19,-12,54,-8,-15,-3,-9,-27,-29,-27,-7;

MA /I: MD=-18;

MA /M: SY='K'; M=-3,1,-22,-1,3,-24,-7,-6,-23,13,-25,-13,7,-7,7,12,6,-2,-19,-26,-15,4; D=-4;

MA /I: MD=-18;

MA /M: SY='R'; M=-7,-2,-2,-4,1,-15,-13,-1,-18,5,-14,-9,2,-14,0,9,-1,-4,-13,-23,-10,0; D=-4;

MA /I: I=-5; MI=0; MD=-18; IM=0; DM=-18;

MA /M: SY='L'; M=0,-10,-12,-9,-4,-5,-10,-8,0,-9,5,0,-9,1,-4,-8,-5,-2,-3,-13,-6,-5; D=-4;

MA /I: DM=-18;

MA /M: SY='E'; M=1,3,-8,5,11,-18,1,-7,-19,-4,-17,-14,1,-7,0,-7,7,-2,-14,-22,-15,5; D=-4;

MA /I: DM=-18;

MA /M: SY='E'; M=-6,2,-19,5,12,-18,-13,-7,-18,6,-16,-12,0,4,3,3,3,4,-15,-22,-12,6; D=-4;

MA /I: DM=-18;

MA /M: SY='S'; M=-4,-8,-19,-7,-4,-10,-16,-14,-2,-9,-11,-7,-5,-1,-7,-12,4,0,-2,-24,-11,-7; D=-4;

MA /I: DM=-18;

MA /M: SY='K'; M=-7,-7,-14,-10,-1,-22,-19,-9,-15,9,-16,-1,-5,-10,8,6,-1,1,-11,-26,-12,3;

MA /M: SY='T'; M=-4,5,-1,-2,-5,-17,-15,-12,-16,-8,-18,-14,10,-16,-6,-7,12,16,-10,-35,-16,-6;

MA /M: SY='F'; M=3,-19,-11,-23,-19,6,-8,-18,-5,-21,4,-2,-15,-23,-20,-19,-7,-4,-1,-16,-2,-19;

MA /M: SY='L'; M=-3,-18,-9,-19,-10,-5,-23,-18,3,-17,12,2,-17,-23,-13,-12,-8,-1,7,-27,-10,-12;

MA /M: SY='D'; M=-9,22,-25,29,11,-29,-12,3,-25,0,-21,-15,11,-13,5,-4,1,-7,-20,-33,-15,8;

MA /M: SY='L'; M=-4,-29,-25,-33,-24,15,-25,-24,9,-25,16,4,-26,-26,-22,-21,-22,-12,4,16,7,-22;

MA /M: SY='F'; M=-6,-25,-17,-30,-23,27,-25,-22,10,-24,12,4,-20,-26,-26,-19,-11,-4,13,-14,5,-23;

MA /M: SY='S'; M=-4,-2,-16,-3,-2,-18,-11,-9,-16,-3,-17,-12,1,-11,-1,-2,4,-1,-14,-27,-11,-3;

MA /M: SY='A'; M=5,-16,-10,-21,-12,5,-18,-11,-5,-15,1,2,-13,-20,-13,-13,-5,-3,-1,-19,-4,-12;

MA /M: SY='L'; M=-10,-29,-21,-31,-21,8,-29,-18,22,-28,45,25,-28,-28,-18,-19,-28,-10,11,-20,0,-19;

MA /M: SY='E'; M=-8,-2,-18,2,27,-22,-21,-6,-17,-1,-10,-11,-5,-10,11,-3,-2,-7,-18,-29,-16,18;

MA /M: SY='E'; M=-5,16,-25,17,19,-28,-12,-2,-27,11,-25,-18,12,-10,9,5,3,-5,-23,-30,-17,14;

MA /M: SY='M'; M=-7,-13,-23,-14,-4,-11,-23,0,-4,-4,2,5,-12,-19,4,1,-10,-5,-4,-20,-1,-1;

MA /M: SY='N'; M=-4,3,-24,2,-2,-22,0,3,-22,-2,-17,-11,7,-18,-4,2,-3,-10,-17,-27,-13,-4;

MA /M: SY='M'; M=-4,-11,-13,-12,-7,-12,-21,-14,-2,-6,3,4,-12,-19,-8,-9,-9,-5,0,-27,-10,-8;

MA /M: SY='L'; M=-9,-25,-22,-25,-15,1,-29,-20,16,-18,29,13,-24,-25,-16,-14,-23,-9,11,-22,-3,-16;

MA /M: SY='S'; M=-2,5,-23,8,6,-26,12,-10,-29,-6,-26,-20,5,-12,-3,-7,13,1,-21,-31,-21,2;

MA /M: SY='P'; M=-6,-9,-20,-6,8,-23,-16,-13,-21,1,-17,-13,-10,13,-2,-2,-6,-6,-19,-27,-19,1;

MA /M: SY='D'; M=-1,5,-23,8,2,-24,-3,-10,-18,-9,-14,-12,1,-14,1,-12,3,-3,-15,-29,-16,1;

MA /M: SY='N'; M=-8,14,-15,9,-2,-21,-11,0,-21,-2,-24,-16,17,-17,-3,-5,2,-3,-20,-23,-12,-3;

MA /M: SY='L'; M=-9,-18,-21,-18,-13,0,-26,-14,7,-17,16,9,-19,-19,-9,-14,-15,-4,5,-20,-1,-11;

MA /I: I=-5; MI=-27; MD=-27; IM=-27;

MA /M: SY='S'; M=-4,3,-18,6,0,-14,-8,-10,-14,-8,-16,-13,1,1,-5,-11,8,5,-11,-25,-12,-3; D=-5;

MA /I: I=-5; MI=-27; MD=-27; IM=-27; DM=-27;

EP 1 258 529 A2

Forts. Fig. 16

MA /M: SY='L'; M=-5,-21,1,-26,-20,11,-24,-18,10,-21,12,5,-18,-22,-20,-18,-14,-6,10,-15,1,-20; D=-5;

MA /I: I=-5; MI=-27; MD=-27; IM=-27; DM=-27;

MA /M: SY='L'; M=-9,-26,-18,-27,-18,12,-26,-18,18,-26,38,16,-24,-25,-18,-18,-24,-9,9,-15,2,-18; D=-5;

MA /I: I=-5; MI=-27; IM=-27; DM=-27;

MA /M: SY='K'; M=2,-9,-22,-12,3,-17,-18,-10,-7,6,-8,4,-10,-14,1,4,-6,-7,-3,-23,-12,1;

MA /M: SY='E'; M=-11,9,-27,14,28,-27,-18,10,-28,7,-21,-15,4,-8,13,4,2,-3,-24,-30,-12,19;

MA /M: SY='L'; M=-9,-27,4,-31,-23,2,-29,-20,14,-26,28,18,-26,-30,-19,-21,-23,-9,10,-26,-6,-22;

MA /M: SY='L'; M=-11,-28,-4,-31,-23,15,-29,-20,11,-29,35,15,-27,-31,-23,-21,-26,-10,6,-20,0,-22;

MA /M: SY='Y'; M=-7,-11,-18,-14,-6,4,-21,0,-16,2,-11,-7,-9,-19,-6,3,-9,-6,-12,-8,14,-7;

MA /M: SY='T'; M=0,-11,-20,-15,-7,-16,-20,-13,-1,-5,-8,-2,-7,-15,3,0,3,5,1,-25,-11,-4;

MA /M: SY=T'; M=-4,-27,-20,-31,-23,1,-29,-24,27,-26,24,14,-23,-24,-20,-23,-16,-6,21,-23,-4,-23;

MA /M: SY='R'; M=-10,-4,-18,-7,-6,-23,2,0,-26,3,-22,-12,5,-20,2,18,-2,-10,-20,-26,-15,-4;

MA /M: SY='R'; M=-18,-9,-31,-8,2,-23,-20,-2,-29,29,-22,-10,-1,-12,11,57,-10,-10,-21,-21,-11,3;

MA /I: I=-6; MI=-32; MD=-32; IM=-32;

MA /M: SY='M'; M=-7,-17,-20,-19,-13,3,-20,-4,2,-11,4,7,-13,-12,-10,-8,-11,-7,0,-15,3,-12; D=-6;

MA /I: I=-6; MI=-32; IM=-32; DM=-32;

MA /M: SY='D'; M=-16,38,-29,53,15,-37,-5,-3,-38,3,-30,-27,16,-11,0,-6,1,-9,-28,-37,-20,7;

MA /M: SY='L'; M=-10,-30,-20,-31,-21,9,-31,-21,22,-30,46,20,-29,-29,-20,-21,-29,-10,13,-20,0,-21;

MA /M: SY='L'; M=-4,-25,-11,-27,-18,0,-27,-20,14,-24,28,12,-24,-27,-16,-19,-18,-7,11,-24,-6,-17;

MA /M: SY='R'; M=-12,-8,-27,-9,1,-21,-22,-8,-17,22,-15,-4,-4,-16,10,28,-8,-5,-12,-21,-9,4;

EP 1 258 529 A2

*Forts. Fig. 16*

MA /M: SY='N'; M=-5,2,-21,-4,-4,-17,-15,0,-9,-2,-12,0,7,-16,4,2,2,1,-9,-28,-10,-1;

MA /M: SY='T'; M=-9,-28,-22,-31,-23,3,-32,-22,25,-22,24,14,-24,-26,-19,-14,-20,-8,21,-20,0,-23;

MA /M: SY='F'; M=-11,-7,-21,-10,-7,9,-21,-11,-6,-10,-4,-4,-4,-21,-9,-9,-7,-6,-5,-19,-1,-6;

MA /M: SY='D'; M=-6,9,-17,10,1,-25,-2,-3,-24,-5,-19,-14,7,-16,-2,-8,-1,-6,-18,-30,-16,-1;

MA /I: E1=0; IE=-105; DE=-105;

//

EP 1 258 529 A2

Forts. Fig. 16

DED I

| EHV-2 (E8) | 1 | .MSHYSMIDTYFSLDEDE.TETYLK |
| HHV-8 (71) | 1 | MATYEVLCEVARKLGTDD.REVMLF |
| HVS (71) | 1 | MDLKTTVLHITDSFTDEE.MYCLLF |
| BHV-4 (ORF) | 1 | MVTRDVLLAIETHLNQNEKTFVVYF |
| MCV (159L) | 7 | MPSLPFLRHILEELDSHE.DSLLLF |
| MCV (160L) | 5 | PIPFSFLRNILAELDASE.HEVLRF |
| FLICE | 1 | MDFSRNLYDIGEQLDSEI.LASLKF |
| Mch4 | 18 | MSFREKLLLIIDSNLGVQ.VENLKF |

DED I

| EHV-2 (E8) | 24 | LCRDLIKNK...GEFQCTRDAFKFL |
| HHV-8 (71) | 25 | LLNVFIPQ.......PTLAQLIGAL |
| HVS (71) | 25 | LINGCIPR......SCNAVNISDLI |
| BHV-4 (ORF) | 26 | LLDPYLP........KECEDFLPTL |
| MCV (159L) | 25 | LCHDAAPG......CTTVTQALCSL |
| MCV (160L) | 25 | LCRDVAPA......SKTAEDALRAL |
| FLICE | 25 | LSLDYIPQRK.QEPIKDALMLFQRL |
| Mch4 | 43 | LCIGLVPNKK.LEKSSSASDVFEHL |

Fig. 2

DED I

| | | |
|---|---|---|
| EHV-2 (E8) | 46 | SDYACLGAAN...QMELLRVGRLD |
| HHV-8 (71) | 43 | RALKEEFRLTFPLLAECLRAGRRD |
| HVS (71) | 44 | IETLSKSTQWDICLMCCLVIRKIG |
| BHV-4 (ORF) | 43 | ENLSKRKIIYPILIELIIQRFD |
| MCV (159L) | 44 | SQQKLTLAA...LVEMLVIQRMD |
| MCV (160L) | 44 | QRRPLLILSS...MAELLCARRFD |
| FLICE | 49 | QEKMLEESNLSFLKELLRINRLD |
| Mch4 | 67 | LAEDLLEEDPFFLAELLIIRQKK |

DED I

| | | |
|---|---|---|
| EHV-2 (E8) | 68 | LLRIFGQTWTPDSCPRYYMPI...C |
| HHV-8 (71) | 68 | LLRDLLHIDPRFLERHIAGTMS..YF |
| HVS (71) | 69 | LLLNLFQITK.EAVKQSFFTQP..Q |
| BHV-4 (ORF) | 68 | LLFSIFLIDIRFVKDQITSIHW.NY |
| MCV (159L) | 66 | LLKSRFGLSR.EGAEQILGTSF... |
| MCV (160L) | 66 | VLKVRFGITR.ECAGRLLGHGF... |
| FLICE | 74 | LLIT.KLNTRKEEMERELQTPGRAQ |
| Mch4 | 92 | LLQH.LNCTK.EEVERILPTRQ..R |

Forts. Fig. 2

## DED II

```
EHV-2 (E8)    91    S P E R C L M A L V N D F L C D K E . V E E M Y F
HHV-8 (71)    92    S P Y Q T L H V D G E L C A R D . I R S I F
HVS (71)      92    E T H V L T V N V N N N L A K D . E K R C F
BHV-4 (ORF)   93    S P Y Q L F S I G Q N I D D E D . L I S K F
MCV (159L)    88    I R Y R K L M V C V G E E L D S S L R A L R L F
MCV (160L)    88    S Q Y R I Q V A A I N N M V G S E D . . L R M C
FLICE         99    S A Y R L M Y Q I S E E V R S . L R S F K F
Mch4          114   S L R N L Y E I S E G I D S E N . L K D I F
FADD          1     M D P L L L H S V S S S L I S S . L T E K F
PEA-15        1     M V E Y G T L F Q D L T N N L L E D . L E Q K S
```

## DED II

```
EHV-2 (E8)    115   L C A P R E S H L E P G S K K S F L R L A S L L
HHV-8 (71)    116   L S K D T G S R . . . S T P Q F L H W M Y C M
HVS (71)      116   L D Q F F P N . V A A P S V I L C V F S N M L
BHV-4 (ORF)   117   I S M N Y I G K S . . P S K I K N Y L D W M R A L
MCV (159L)    113   A C N L N P S L S T A L S E S S R F M L L A L L
MCV (160L)    111   L C A G K I L P P . . S C T P R C L I L M S A L
FLICE         123   L L Q E E S C . K L D D D M N L L I F I E M
Mch4          138   L L K D S P . . . . . T E M S L S F I A F L
FADD          26    L C L G R G R . K L E R V Q M G L L F S M L
PEA-15        26    A C K E D P S E . K S E E I T I G S A W F S F L
```

Forts. Fig. 2

## DED II

```
EHV-2  (E8)    140  ED E LGGDK L  FLRH L LT    GRA D
HHV-8  (71)    138  EN D LGPTD  DALMS L      SRV D
HVS    (71)    140  CE H  LE . . C L C  LKKC L  Q  GRS D
BHV-4  (ORF)   140  EK AM  GPDN L D FETLF  Q  HRM D
MCV    (159L)  138  EN GL  SPSS   LAD L    RRL D
MCV    (160L)  134  ED AGA SPQD   LVTL L  A  CRY D
FLICE          147  EKRV L GEGK L D LKR CA  N . S
Mch4           158  EKQGK DEDN L  CLED L C  VP . K
FADD            50  LEQND L EPGHTE  LRE LLA    RRH D
PEA-15          50  ESHNK L DKDN L   EH FEISR RP D
```

## DED II

```
EHV-2  (E8)    165  L   N  QV
HHV-8  (71)    163  LQ  Q  QT
HVS    (71)    163  LA T  . .
BHV-4  (ORF)   165  L M  KN
MCV    (159L)  163  LCQQ VE    ⇒
MCV    (160L)  159  LSVA SA    ⇒
FLICE          171  L  I  ND
Mch4           182  L  N EK
FADD            75  L  R DD
PEA-15          75  L TM VD
```

Forts. Fig. 2

EP 1 258 529 A2

DED I

| | | |
|---|---|---|
| FLIPL_HS | 1 | ...........................MSAEVIH |
| FLIPL_MM | 1 | ......................MAOSPVSAEVIH |
| vFLIP_EHV2 | 1 | .......................MSHYSMI |
| FLICE_HS | 1 | ......................MDFSRNLY |
| Mch4__HS | 1 | MKSQGQHWYSSSDKNCKVSFREKEL |

DED I

| | | |
|---|---|---|
| FLIPL_HS | 8 | OVEEALDTDEKEMLLFLCRDVAIDV |
| FLIPL_MM | 13 | OVEECLDEDEKEMMLFLCRDVTENL |
| vFLIP_EHV2 | 8 | DTYFSLDEDETETYLVLCRDLIKNK |
| FLICE_HS | 9 | DIGEOLDSEDLASLKFISLDYIPOR |
| Mch4__HS | 26 | ILDSNLGVQDVENLKFLCIGAVPNK |

DED I

| | | |
|---|---|---|
| FLIPL_HS | 33 | ...VPPNVRDLLDILRERCKLSVGD |
| FLIPL_MM | 38 | ...AAPNVRDLLDSLSEROLSFAT |
| vFLIP_EHV2 | 33 | ..GEFOCTRDAFKFLSDYACLSAAN |
| FLICE_HS | 34 | KOEPIKDALMLFORLOEKRMLEESN |
| Mch4__HS | 51 | KLEKSSSASDVFFHLLAEDLLSEED |

Fig. 3

DED I

```
FLIPL_HS    55   ...LAELLYRVRRFDLLKRILKMDF
FLIPL_MM    60   ...LAELLYRVRRFDLLKRILKTDK
vFLIP_EHV2  56   ...QMELLHRVGRLDLIRRIFGQ.T
FLICE_HS    59   LSFLKELLHRINRLDLIITYLNT.R
Mch4_HS     76   PFFLAELLYIHRQKKLLQHHNCT..
```

DED II

```
FLIPL_HS    77   KAVETHLL..RNPHLVSDYRVLMAE
FLIPL_MM    82   ATVEDHIR..RNPHLVSDYRVLLME
vFLIP_EHV2  77   WTPDSCPR..YYMPLCSPHRCIMAL
FLICE_HS    83   KEEMERELQTPGRAQISAYRVMLYQ
Mch4_HS     99   KEEVERLL..PTRQRVSLHRNLLYE
```

DED II

```
FLIPL_HS    100  IGEDLDKSPVSSLIFLMKDYMGR.G
FLIPL_MM    105  IGESLDQNDVSSLVFLTRDYTGR.G
vFLIP_EHV2  100  VNDFLSDKHVEEMYFLCAPRLESHLC
FLICE_HS    108  ISEEVSRSELRSFKFLLQEEISK.C
Mch4_HS     122  LSEGIDSENLKDMIFLLKDSLPK..
```

EP 1 258 529 A2

DED II

```
FLIPL_HS     124    KISKEKSFLDLVVELEKLNLVAPDQ
FLIPL_MM     129    KIAKDKSFLDLVIELEKLNLLASDQ
vFLIP_EHV2   125    EPGSKKSFLRLASLLEDLELLGGDK
FLICE_HS     132    KIDDDMNLLDIFIEMEKRVILGEGK
Mch4__HS     145    ...THMTSLSFLAFLEKQGKIDEDN
```

DED II

```
FLIPL_HS     149    LDLLEKCLKNIHRIDLKTKIQKYKQ
FLIPL_MM     154    LNLLEKCLKNIHRIDLNTKIQKYTQ
vFLIP_EHV2   150    LTFLRHLLTTIGRADLVKNLQV...
FLICE_HS     157    LDILKRVCAQINK.SLLKIINDYEE
Mch4__HS     167    LTCLEDLCKTVVP.KLLRNIEKYK.
```

```
FLIPL_HS     174    SVQGAGTSYRNVLQAAIQK.SLKDP
FLIPL_MM     179    SSQGA.RSNMNTLQASLPKLSIK..
FLICE_HS     181    FS.......................
Mch4__HS     190    .........................
```

Forts. Fig. 3

FLIPS → MITPYAHCPDLKILGNCSM

```
FLIPL _HS    198   SNNFRLHNGRSKEQRLKEQLGAQQE
FLIPL _MM    201   .YNSRLONGRSKEPRFVEYRDSORT
FLICE_HS     183   .KERSSSLEGSPDEFSNGEELCGVM
Mch4 __HS    190   .REKAIQIVTPPVDKEAESYQGEEE
```

→ FLIPc - - - - Caspase

```
FLIPL _HS    223   PVKKSIOESEAFLPOSIPEERYKMK
FLIPL _MM    225   LVKTSIOESGAFLPPHIRHETYRMO
FLICE_HS     207   TISDSPREODSESO..TLLKVYOMK
Mch4 __HS    214   LVSQIDVKIFLEAL..PRAAVYRMN
```

Caspase

```
FLIPL _HS    248   SKPLGICLIIDCIGNE..........
FLIPL _MM    250   SKPLGICLIIDCIGND..........
FLICE_HS     230   SKPRGYCLIINNHNFAKAREKVPKL
Mch4 __HS    237   RNHRGLCMIMNNHSFT..........
```

Forts. Fig.3

```
                         O        Caspase
FLIPL _HS   264   .IELLRDTFTSLGYEMQKFLH....
FLIPL _MM   266   .TKYLQETFTSLGYHIQLFLF....
FLICE_HS    255   HSIRDRNGTHLDAGALTTTFEELHF
Mch4__HS    253   .GLKDRQGTHKDAEIISHVFQWLGF

                              Caspase
FLIPL _HS   284   ........LSMHGISQIIGQFACMPE
FLIPL _MM   286   ........PKSHDITQIIRRMASMAQ
FLICE_HS    280   EIKPHDDCTVEQIYEILKIYQL.MD
Mch4__HS    277   TVHIHNNVTKVEMEMMIQKQKCNPA

                    Caspase         ★★
FLIPL _HS   302   HRDYDSFVCVLVSRGGSQSVYGVDQ
FLIPL _MM   304   HQDYDSFACVLVSLGGSQSMMGRDQ
FLICE_HS    304   HSNMDCFTCCLISHGDKGIIYGTDG
Mch4__HS    302   HADGDCFMFCTETHGRFGAVYSSDE
```

Forts. Fig. 3

EP 1 258 529 A2

Caspase

| | | |
|---|---|---|
| FLIPL _HS | 327 | THSGLPIHHIRRMFMGDSCPYLAGK |
| FLIPL _MM | 329 | VHSGFSIDHVKNMFTGDTCPSIRGK |
| FLICE_HS | 329 | QEP..PIYELTSOFTGLKCPSLAGK |
| Mch4__HS | 327 | ALI..PIREIMSHFTALQCPRLAEK |

○—☆  Caspase  ← p17

| | | |
|---|---|---|
| FLIPL _HS | 352 | PKMFFICNYVVSEGOIEDSSLIEVD |
| FLIPL _MM | 354 | PKIFFICNYESLGSOIEDSS.IEVD |
| FLICE_HS | 352 | PKVFFICACQGDN..YOKGIPVETD |
| Mch4__HS | 350 | PKIFFICACQGEE..IQPSVSIEAD |

p12 →  Caspase

| | | |
|---|---|---|
| FLIPL _HS | 377 | GPAMKNVEFKAOKRGLCTVHREADF |
| FLIPL _MM | 378 | GPSIKNVDSKPLOPRHCTTHPEADI |
| FLICE_HS | 375 | SEEOPYLEMDLSSPOTRYIPDEADF |
| Mch4__HS | 373 | ALNPEQAPTSLQDS....IPAEADF |

Forts. Fig. 3

Forts. Fig. 3

Caspase

| | | |
|---|---|---|
| FLIP_L — HS | 402 | FWSLCTADMSLLEQSHSSPSLYHQC |
| FLIP_L — MM | 403 | FWSLCTADVSHLEKPSSSSVYHQK |
| FLICE — HS | 400 | LLGMATVNNCVSYIRNPAEGIWHQS |
| Mch4 — HS | 394 | LLGLATVPGYVSFRHVEEGSWHQS |

Caspase

| | | |
|---|---|---|
| FLIP_L — HS | 427 | LSQKLQERKRPLLDIHIELNGYMY |
| FLIP_L — MM | 428 | LSQLKQGRRRPLVDIHVELMDKVY |
| FLICE — HS | 425 | LWQSLRERCPR.GDDHLTLTEVNY |
| Mch4 — HS | 419 | LCNHLKKLVPR.HEDILSILTAVND |

Caspase

| | | |
|---|---|---|
| FLIP_L — HS | 452 | DWNSRVSAK..EKYYVWLQHTLRKK |
| FLIP_L — MM | 453 | AWNSGVSSK..EKYSLSLQHTLRKK |
| FLICE — HS | 449 | EVSNKDDKKNMGKQMPQPTFTLRKK |
| Mch4 — HS | 443 | DVSRRVDKQGTKKQMPQPAFTLRKK |

Forts. Fig.3

Caspase

| | | |
|---|---|---|
| FLIPL_HS | 475 | LHLSYT..... |
| FLIPL_MM | 476 | LHLAPT..... |
| FLICE_HS | 474 | LMFPSD..... |
| Mch4__HS | 468 | LAFPVPLDALSI |

Human c-FLIP$_S$ Nucleotides: 1 to 1190 *Fig. 4a*

```
          10          20          30          40
 *         *   *         *   *         *   *         *
AAGCTCGAAA TTAACCCTCA CTAAAGGGAA CAAAAGCTGG


          50          60          70          80
 *         *   *         *   *         *   *         *
AGCTCCACCG CGGTGGCGGC CGCTCTAGAA CTAGTGGATC


          90         100         110         120
 *         *   *         *   *         *   *         *
CCCCGGGCTG CAGGAATTCG GCACGAGCGG CACGAGTAGA


         130         140         150         160
 *         *   *         *   *         *   *         *
CTTCTATAGA TCCCTTTCTA TAGAACTTAA TCTACTTAAG


         170         180         190         200
 *         *   *         *   *         *   *         *
TCAGGGAGAC CACCCAGAAG GAAAGAGCCC ATACTTTCAA


         210         220         230         240
 *         *   *         *   *         *   *         *
TCTTAGGCAT AAGTTAGCTT GATAAGATTT TCAGAAAAAT


         250         260         270         280
 *         *   *         *   *         *   *         *
TCCCTTTTAA CCACAGAACT CCCCCACTGG AAAGGATTCT
```

Forts. Fig. 4a

```
        290           300           310           320
      *       *     *       *     *       *     *       *
GAAAGAAATG AAGTCAGCCC TCAGAAATGA AGTTGACTGC


        330           340           350           360
      *       *     *       *     *       *     *       *
CTGCTGGCTT TCTGTTGACT GGCCCGGAGC TGTACTGCAA


        370           380           390           400
      *       *     *       *     *       *     *       *
GACCCTTGTG AGCTTCCCTA GTCTAAGAGT AGGATGTCTG
                                            M   S


        410           420           430           440
      *       *     *       *     *       *     *       *
CTGAAGTCAT CCATCAGGTT GAAGAAGCAC TTGATACAGA
A   E   V   I     H   Q   V     E   E   A     L   D   T   D


        450           460           470           480
      *       *     *       *     *       *     *       *
TGAGAAGGAG ATGCTGCTCT TTTTGTGCCG GGATGTTGCT
  E   K   E     M   L   L     F   L   C   R     D   V   A


        490           500           510           520
      *       *     *       *     *       *     *       *
ATAGATGTGG TTCCACCTAA TGTCAGGGAC CTTCTGGATA
  I   D   V     V   P   P   N     V   R   D     L   L   D
```

Forts. Fig. 4a

```
        530              540              550              560
     *        *        *        *        *        *        *        *
TTTTACGGGA  AAGAGGTAAG  CTGTCTGTCG  GGGACTTGGC
 I  L  R  E   R  G  K    L  S  V     G  D  L  A
```

```
        570              580              590              600
     *        *        *        *        *        *        *        *
TGAACTGCTC  TACAGAGTGA  GGCGATTTGA  CCTGCTCAAA
    E  L  L   Y  R  V     R  R  F  D    L  L  K
```

```
        610              620              630              640
     *        *        *        *        *        *        *        *
CGTATCTTGA  AGATGGACAG  AAAAGCTGTG  GAGACCCACC
    R  I  L   K  M  D  R   K  A  V     E  T  H
```

```
        650              660              670              680
     *        *        *        *        *        *        *        *
TGCTCAGGAA  CCCTCACCTT  GTTTCGGACT  ATAGAGTGCT
 L  L  R  N   P  H  L     V  S  D     Y  R  V  L
```

```
        690              700              710              720
     *        *        *        *        *        *        *        *
GATGGCAGAG  ATTGGTGAGG  ATTTGGATAA  ATCTGATGTG
    M  A  E   I  G  E     D  L  D  K   S  D  V
```

```
        730              740              750              760
     *        *        *        *        *        *        *        *
TCCTCATTAA  TTTTCCTCAT  GAAGGATTAC  ATGGGCCGAG
    S  S  L   I  F  L  M   K  D  Y     M  G  R
```

Forts. Fig. 4a

```
        770            780            790            800
     *         *     *         *     *         *     *         *
GCAAGATAAG CAAGGAGAAG AGTTTCTTGG ACCTTGTGGT
 G   K   I   S   K   E   K   S   F   L   D   L   V   V


        810            820            830            840
     *         *     *         *     *         *     *         *
TGAGTTGGAG AAACTAAATC TGGTTGCCCC AGATCAACTG
   E   L   E   K   L   N   L   V   A   P   D   Q   L


        850            860            870            880
     *         *     *         *     *         *     *         *
GATTTATTAG AAAAATGCCT AAAGAACATC CACAGAATAG
 D   L   L   E   K   C   L   K   N   I   H   R   I


        890            900            910            920
     *         *     *         *     *         *     *         *
ACCTGAAGAC AAAAATCCAG AAGTACAAGC AGTCTGTTCA
 D   L   K   T   K   I   Q   K   Y   K   Q   S   V   Q


        930            940            950            960
     *         *     *         *     *         *     *         *
AGGAGCAGGG ACAAGTTACA GGAATGTTCT CCAAGCAGCA
 G   A   G   T   S   Y   R   N   V   L   Q   A   A


        970            980            990           1000
     *         *     *         *     *         *     *         *
ATCCAAAAGA GTCTCAAGGA TCCTTCAAAT AACTTCAGGA
 I   Q   K   S   L   K   D   P   S   N   N   F   R
```

Forts. Fig. 4a

```
        1010              1020              1030              1040
   *         *        *         *        *         *        *         *
TGATAACMCC   CTATGCCCAT   TGCCCTGATC   TGAAAATTCT
 M    I    T    P     Y    A    H     C    P    D     L    K    I    L


        1050              1060              1070              1080
   *         *        *         *        *         *        *         *
TGGAAATTGT   TCCATGTGAT   TAACATGGAA   CBGCCTCTAC
      G    N    C     S    M▷


        1090              1100              1110              1120
   *         *        *         *        *         *        *         *
TTAATCATTC   TGAATGATTA   AATCGTTTCA   TTTTCTAAAT


        1130              1140              1150              1160
   *         *        *         *        *         *        *         *
GTGTAAAAAA   AAAAAAAAAA   AAAAAAACTC   GAGGGGGGGC


        1170              1180              1190
   *         *        *         *        *         *
   CCGTACCCAA   TTCGCCCTAT   AGTGAGTCGT
```

Fig. 46

# Human c-FLIP~L~ nucleotides: 1 to 2143

```
         10          20          30          40
    *         *    *         *    *         *    *         *
TAGGGGTGGG GACTCGGCCT CACACAGTGA GTGCCGGCTA


         50          60          70          80
    *         *    *         *    *         *    *         *
TTGGACTTTT GTCCAGTGAC AGCTGAGACA ACAAGGACCA


         90         100         110         120
    *         *    *         *    *         *    *         *
CGGGAGGAGG TGTAGGAGAG AAGCGCCGCG AACAGCGATC


        130         140         150         160
    *         *    *         *    *         *    *         *
GCCCAGCACC AAGTCCGCTT CCAGGCTTTC GGTTTCTTTG


        170         180         190         200
    *         *    *         *    *         *    *         *
CCTCCATCTT GGGTGCGCCT TCCCGGCGTC TAGGGGAGCG


        210         220         230         240
    *         *    *         *    *         *    *         *
AAGGCTGAGG TGGCAGCGGC AGGAGAGTCC GGCCGCGACA


        250         260         270         280
    *         *    *         *    *         *    *         *
GGACGAACTC CCCCACTGGA AAGGATTCTG AAAGAAATGA
```

Forts. Fig. 46

```
          290            300            310            320
     *         *      *         *      *         *      *         *
   AGTCAGCCCT CAGAAATGAA GTTGACTGCC TGCTGGCTTT


          330            340            350            360
     *         *      *         *      *         *      *         *
   CTGTTGACTG GCCCGGAGCT GTACTGCAAG ACCCTTGTGA


          370            380            390            400
     *         *      *         *      *          *     *         *
   GCTTCCCTAG TCTAAGAGTA GGATGTCTGC TGAAGTCATC
                                  M    S    A    E    V    I


          410            420            430            440
     *         *      *         *      *         *      *         *
   CATCAGGTTG AAGAAGCACT TGATACAGAT GAGAAGGAGA
    H    Q    V    E    E    A    L    D    T    D    E    K    E


          450            460            470            480
     *         *      *         *      *         *      *         *
   TGCTGCTCTT TTTGTGCCGG GATGTTGCTA TAGATGTGGT
    M    L    L    F    L    C    R    D    V    A    I    D    V    V


          490            500            510            520
     *         *      *         *      *         *      *         *
   TCCACCTAAT GTCAGGGACC TTCTGGATAT TTTACGGGAA
    P    P    N    V    R    D    L    L    D    I    L    R    E
```

57

Forts. Fig. 4b

```
          530              540              550              560
    *           *      *          *     *          *      *          *
AGAGGTAAGC  TGTCTGTCGG  GGACTTGGCT  GAACTGCTCT
  R    G    K    L    S    V    G    D    L    A    E    L    L


          570              580              590              600
    *           *      *          *     *          *      *          *
ACAGAGTGAG  GCGATTTGAC  CTGCTCAAAC  GTATCTTGAA
  Y    R    V    R    R    F    D    L    L    K    R    I    L    K


          610              620              630              640
    *           *      *          *     *          *      *          *
GATGGACAGA  AAAGCTGTGG  AGACCCACCT  GCTCAGGAAC
  M    D    R    K    A    V    E    T    H    L    L    R    N


          650              660              670              680
    *           *      *          *     *          *      *          *
CCTCACCTTG  TTTCGGACTA  TAGAGTGCTG  ATGGCAGAGA
  P    H    L    V    S    D    Y    R    V    L    M    A    E


          690              700              710              720
    *           *      *          *     *          *      *          *
TTGGTGAGGA  TTTGGATAAA  TCTGATGTGT  CCTCATTAAT
  I    G    E    D    L    D    K    S    D    V    S    S    L    I


          730              740              750              760
    *           *      *          *     *          *      *          *
TTTCCTCATG  AAGGATTACA  TGGGCCGAGG  CAAGATAAGC
  F    L    M    K    D    Y    M    G    R    G    K    I    S
```

Forts. Fig. 4b

```
        770              780              790              800
    *        *       *        *       *        *       *        *
AAGGAGAAGA  GTTTCTTGGA  CCTTGTGGTT  GAGTTGGAGA
 K   E   K   S   F   L   D   L   V   V   E   L   E


        810              820              830              840
    *        *       *        *       *        *       *        *
AACTAAATCT  GGTTGCCCCA  GATCAACTGG  ATTTATTAGA
 K   L   N   L   V   A   P   D   Q   L   D   L   L   E


        850              860              870              880
    *        *       *        *       *        *       *        *
AAAATGCCTA  AAGAACATCC  ACAGAATAGA  CCTGAAGACA
 K   C   L   K   N   I   H   R   I   D   L   K   T


        890              900              910              920
    *        *       *        *       *        *       *        *
AAAATCCAGA  AGTACAAGCA  GTCTGTTCAA  GGAGCAGGGA
 K   I   Q   K   Y   K   Q   S   V   Q.  G   A   G


        930              940              950              960
    *        *       *        *       *        *       *        *
CAAGTTACAG  GAATGTTCTC  CAAGCAGCAA  TCCAAAAGAG
 T   S   Y   R   N   V   L   Q   A   A   I   Q   K   S


        970              980              990             1000
    *        *       *        *       *        *       *        *
TCTCAAGGAT  CCTTCAAATA  ACTTCAGGCT  CCATAATGGG
 L   K   D   P   S   N   N   F   R   L   H   N   G
```

Forts. Fig. 46

```
        1010            1020            1030            1040
      *       *       *       *       *       *       *       *
  AGAAGTAAAG   AACAAAGACT   TAAGGAACAG   CTTGGCGCTC
   R   S   K    E   Q   R   L    K   E   Q    L   G   A


        1050            1060            1070            1080
      *       *       *       *       *       *       *       *
  AACAAGAACC   AGTGAAGAAA   TCCATTCAGG   AATCAGAAGC
   Q   Q   E   P    V   K   K    S   I   Q    E   S   E   A


        1090            1100            1110            1120
      *       *       *       *       *       *       *       *
  TTTTTTGCCT   CAGAGCATAC   CTGAAGAGAG   ATACAAGATG
    F   L   P    Q   S   I    P   E   E   R    Y   K   M


        1130            1140            1150            1160
      *       *       *       *       *       *·      *       *
  AAGAGCAAGC   CCCTAGGAAT   CTGCCTGATA   ATCGATTGCA
   K   S   K    P   L   G   I    C   L   I    I   D   C


        1170            1180            1190            1200
      *       *       *       *       *       *       *       *
  TTGGCAATGA   GACAGAGCTT   CTTCGAGACA   CCTTCACTTC
   I   G   N   E    T   E   L    L   R   D    T   F   T   S


        1210            1220            1230            1240
      *       *       *       *       *       *       *       *
  CCTGGGCTAT   GAAGTCCAGA   AATTCTTGCA   TCTCAGTATG
    L   G   Y    E   V   Q    K   F   L   H    L   S   M
```

Forts. Fig. 46

```
       1250            1260            1270            1280
    *         *      *         *      *         *      *         *
CATGGTATAT  CCCAGATTCT  TGGCCAATTT  GCCTGTATGC
  H   G   I    S   Q   I   L    G   Q   F    A   C   M


       1290            1300            1310            1320
    *         *      *         *      *         *      *         *
CCGAGCACCG  AGACTACGAC  AGCTTTGTGT  GTGTCCTGGT
  P   E   H   R    D   Y   D    S   F   V    C   V   L   V


       1330            1340            1350            1360
    *         *      *         *      *         *      *         *
GAGCCGAGGA  GGCTCCCAGA  GTGTGTATGG  TGTGGATCAG
    S   R   G    G   S   Q    S   V   Y   G    V   D   Q


       1370            1380            1390            1400
    *         *      *         *      *         *      *         *
ACTCACTCAG  GGCTCCCCCT  GCATCACATC  AGGAGGATGT
  T   H   S    G   L   P   L    H   H   I    R   R   M


       1410            1420            1430            1440
    *         *      *         *      *         *      *         *
TCATGGGAGA  TTCATGCCCT  TATCTAGCAG  GGAAGCCAAA
  F   M   G   D    S   C   P    Y   L   A    G   K   P   K


       1450            1460            1470            1480
    *         *      *         *      *         *      *         *
GATGTTTTTT  ATTCAGAACT  ATGTGGTGTC  AGAGGGCCAG
    M   F   F    I   Q   N    Y   V   V   S    E   G   Q
```

Fork. Fig.4b

```
         1490            1500            1510            1520
     *         *       *         *       *         *       *         *
CTGGAGGACA GCAGCCTCTT GGAGGTGGAT GGGCCAGCGA
 L   E   D   S   S   L   L   E   V   D   G   P   A


          1530            1540            1550            1560
     *         *       *         *       *         *       *         *
TGAAGAATGT GGAATTCAAG GCTCAGAAGC GAGGGCTGTG
 M   K   N   V   E   F   K   A   Q   K   R   G   L   C


         1570            1580            1590            1600
     *         *       *         *       *         *       *         *
CACAGTTCAC CGAGAAGCTG ACTTCTTCTG GAGCCTGTGT
   T   V   H   R   E   A   D   F   F   W   S   L   C


          1610            1620            1630            1640
     *         *       *         *       *         *       *         *
ACTGCGGACA TGTCCCTGCT GGAGCAGTCT CACAGCTCAC
   T   A   D   M   S   L   L   E   Q   S   H   S   S


          1650            1660            1670            1680
     *         *       *         *       *         *       *         *
CATCCCTGTA CCTGCAGTGC CTCTCCCAGA AACTGAGACA
 P   S   L   Y   L   Q   C   L   S   Q   K   L   R   Q


          1690            1700            1710            1720
     *         *       *         *       *         *       *         *
AGAAAGAAAA CGCCCACTCC TGGATCTTCA CATTGAACTC
   E   R   K   R   P   L   L   D   L   H   I   E   L
```

Fpits. Fig. 4b

```
        1730            1740            1750            1760
    *        *       *        *       *        *       *        *
AATGGCTACA  TGTATGATTG  GAACAGCAGA  GTTTCTGCCA
   N    G    Y    M    Y    D    W    N    S    R    V    S    A


        1770            1780            1790            1800
    *        *       *        *       *        *       *        *
AGGAGAAATA  TTATGTCTGG  CTGCAGCACA  CTCTGAGAAA
   K    E    K    Y    Y    V    W    L    Q    H    T    L    R    K


        1810            1820            1830            1840
    *        *       *        *       *        *       *        *
GAAACTTATC  CTCTCCTACA  CATAAGAAAC  CAAAAGGCTG
   K    L    I    L    S    Y    T


        1850            1860            1870            1880
    *        *       *        *       *        *       *        *
GGCGTAGTGG  CTCACACCTG  TAATCCCAGC  ACTTTGGGAG


        1890            1900            1910            1920
    *        *       *        *       *        *       *        *
GCCAAGGNGG  GCAGATCACT  TCAGGTCAGG  AGTTCGAGAC


        1930            1940            1950            1960
    *        *       *        *       *        *       *        *
CAGCCTGGCC  AACATGGTAA  ACGCTGTCCC  TAGTAAAAAT


        1970            1980            1990            2000
    *        *       *        *       *        *       *        *
ACAAAAATTA  GCTGGGTGTG  GGTGTGGGTA  CCTGTATTCC
```

```
            2010            2020          2030  Forts. Fig. 4b 2040
          *       *       *       *       *       *       *       *
        CAGTTACTTG  GGAGGCTGAG  GTGGGAGGAT  CTTTTGAACC


            2050            2060          2070            2080
          *       *       *       *       *       *       *       *
        CAGGAGTTCA  GGGTCATAGC  ATGCTGTGAT  TGTGCCTACG


            2090            2100          2110            2120
          *       *       *       *       *       *       *       *
        AATAGCCACT  GCATACCAAC  CTGGGCAATA  TAGCAAGATC


            2130            2140
          *       *       *       *
        CCATCTCTTT  AAAAAAAAAA  AAA
```

Fig. 4c

## Mouse c-FLIP_L nucleotides: 1 to 2452

```
           10          20          30          40
    *        *    *        *    *        *    *        *
 GAATTCCGAG CCTCTCAAGC GGCCACTTAG GGCCGGACAG


           50          60          70          80
    *        *    *        *    *        *    *        *
 AGTGTCTCTA TTGCAAGAAC TCTGAGAGAA ATGAAGAGAG


           90         100         110         120
    *        *    *        *    *        *    *        *
 TCCTCAGCAA TGATGTTGGC TTCTCGTGGT TCCCAGAGCC


          130         140         150         160
    *        *    *        *    *        *    *        *
 CTGCTTAATG GATGGAGACT GGACGAGAAC CTGGCTGCTG


          170         180         190         200
    *        *    *        *    *        *    *        *
 TGGTTCTGAA CATGGCCCAG AGCCCTGTGT CTGCCGAGGT
                   M    A    Q    S    P   V    S    A    E   V


          210         220         230         240
    *        *    *        *    *        *    *        *
 CATTCACCAG GTGGAAGAGT GTCTTGATGA AGACGAGAAG
    I    H    Q    V    E    E    C    L   D    E    D    E    K
```

Forts. Fig. 4c

```
        250               260               270               280
    *         *       *         *       *         *       *         *
GAGATGATGC  TCTTCCTGTG  TAGAGATGTG  ACTGAGAACC
 E   M   M    L   F   L   C    R   D   V    T   E   N


        290               300               310               320
    *         *       *         *       *         *       *         *
TGGCTGCACC  TAACGTCAGG  GACCTCCTGG  ATAGCTTAAG
 L   A   A   P    N   V   R    D   L   L    D   S   L   S


        330               340               350               360
    *         *       *         *       *         *       *         *
TGAGAGAGGC  CAGCTCTCTT  TTGCTACCTT  GGCTGAATTG
  E   R   G    Q   L   S    F   A   T   L    A   E   L


        370               380               390               400
    *         *       *         *       *         *       *         *
CTCTACAGAG  TGAGGCGGTT  TGACCTTCTC  AAGAGGATCT
  L   Y   R    V   R   R   F    D   L   L    K   R   I


        410               420               430               440
    *         *       *         *       *         *       *         *
TGAAGACAGA  CAAAGCAACC  GTGGAGGACC  ACCTGCGCAG
 L   K   T   D    K   A   T    V   E   D    H   L   R   R


        450               460               470               480
    *         *       *         *       *         *       *         *
AAACCCTCAC  CTGGTTTCTG  ATTATAGGGT  CCTGCTGATG
  N   P   H    L   V   S    D   Y   R   V    L   L   M
```

Foris. Fig. 4c

```
          490              500              510              520
      *         *      *         *      *         *      *         *
GAGATTGGTG AGAGCTTAGA TCAGAACGAT GTATCCTCCT
  E    I    G    E    S    L    D    Q    N    D    V    S    S


          530              540              550              560
      *         *      *         *      *         *      *         *
TAGTTTTCCT TACAAGGGAT TACACAGGCA GAGGCAAGAT
  L    V    F    L    T    R    D    Y    T    G    R    G    K    I


          570              580              590              600
      *         *      *         *      *         *      *         *
AGCCAAGGAC AAGAGTTTCT TGGATCTGGT GATTGAATTG
  A    K    D    K    S    F    L    D    L    V    I    E    L


          610              620              630              640
      *         *      *         *      *         *      *         *
GAGAAACTGA ATCTAATTGC TTCAGACCAA TTGAATTTGT
  E    K    L    N    L    I    A    S    D    Q    L    N    L


          650              660              670              680
      *         *      *         *      *         *      *         *
TAGAAAAATG CCTGAAGAAC ATCCACAGAA TAGACTTGAA
  L    E    K    C    L    K    N    I    H    R    I    D    L    N


          690              700              710              720
      *         *      *         *      *         *      *         *
CACAAAGATC CAGAAGTACA CCCAGTCCAG CCAAGGAGCA
  T    K    I    Q    K    Y    T    Q    S    S    Q    G    A
```

67

Forts. Fig. 4c

```
         730             740             750             760
    *         *       *         *     *         *     *         *
AGATCAAATA TGAATACTCT CCAGGCTTCG CTCCCAAAAT
 R   S   N   M   N   T   L   Q   A   S   L   P   K


         770             780             790             800
    *         *       *         *     *         *     *         *
TGAGTATCAA GTATAACTCA AGGCTCCAGA ATGGGCGAAG
 L   S   I   K   Y   N   S   R   L   Q   N   G   R   S


         810             820             830             840
    *         *       *         *     *         *     *         *
TAAAGAGCCA AGATTTGTGG AATACCGTGA CAGTCAAAGA
   K   E   P   R   F   V   E   Y   R   D   S · Q   R


         850             860             870             880
    *         *       *         *     *         *     *         *
ACACTGGTGA AGACATCCAT CCAGGAATCA GGAGCTTTTT
 T   L   V   K   T   S   I   Q   E   S   G   A   F


         890             900             910             920
    *         *       *         *     *         *     *         *
TACCTCCGCA CATCCGTGAA GAGACTTACA GGATGCAGAG
 L   P   P   H   I   R   E   E   T   Y   R   M   Q   S


         930             940             950             960
    *         *       *         *     *         *     *         *
CAAGCCCCTA GGAATCTGCT TGATCATTGA TTGTATTGGC
   K   P   L   G   I   C   L   I   I   D   C   I   G
```

Fonts. Fig. 4c

```
         970              980              990          1000
     *        *        *        *        *        *        *        *
AACGACACAA AATATCTTCA AGAGACCTTC ACTTCCCTGG
 N    D    T    K    Y    L    Q    E    T    F    T    S    L


        1010             1020             1030             1040
     *        *        *        *        *        *        *        *
GCTATCATAT CCAGCTTTTC TTGTTTCCCA AGTCACATGA
 G    Y    H    I    Q    L    F    L    F    P    K    S    H    D


        1050             1060             1070             1080
     *        *        *        *        *        *        *        *
CATAACCCAG ATTGTTCGCC GATATGCAAG TATGGCCCAA
  I    T    Q    I    V    R    R    Y    A    S    M    A    Q


        1090             1100             1110             1120
     *        *        *        *        *        *        *        *
CATCAAGACT ATGACAGCTT TGCATGTGTT CTGGTGAGCC
 H    Q    D    Y    D    S    F    A    C    V    L    V    S


        1130             1140             1150             1160
     *        *        *        *        *        *        *        *
TAGGAGGCTC CCAAAGCATG ATGGGCAGAG ATCAAGTTCA
 L    G    G    S    Q    S    M    M    G    R    D    Q    V    H


        1170             1180             1190             1200
     *        *        *        *        *        *        *        *
CTCAGGGTTC TCCTTGGATC ATGTCAAGAA CATGTTCACG
  S    G    F    S    L    D    H    V    K    N    M    F    T
```

Forts. Fig. 4c

```
        1210            1220            1230            1240
    *       *       *       *       *       *       *       *
GGGGACACGT GCCCTTCTCT CAGAGGGAAG CCAAAGCTCT
 G   D   T   C   P   S   L   R   G   K   P   K   L


        1250            1260            1270            1280
    *       *       *       *       *       *       *       *
TTTTTATTCA GAACTATGAG TCGTTAGGTA GCCAGTTGGA
 F   F   I   Q   N   Y   E   S   L   G   S   Q   L   E


        1290            1300            1310            1320
    *       *       *       *       *       *       *       *
AGATAGCAGC CTGGAGGTAG ATGGGCCATC AATAAAAAAT
 D   S   S   L   E   V   D   G   P   S   I   K   N


        1330            1340            1350            1360
    *       *       *       *       *       *       *       *
GTGGACTCTA AGCCCCTGCA ACCCAGACAC TGCACAACTC
 V   D   S   K   P   L   Q   P   R   H   C   T   T


        1370            1380            1390            1400
    *       *       *       *       *       *       *       *
ACCCAGAAGC TGATATCTTT TGGAGCCTGT GCACAGCAGA
 H   P   E   A   D   I   F   W   S   L   C   T   A   D


        1410            1420            1430            1440
    *       *       *       *       *       *       *       *
CGTATCTCAC TTGGAGAAGC CCTCCAGCTC ATCCTCTGTG
 V   S   H   L   E   K   P   S   S   S   S   S   V
```

Forts. Fig. 4c

```
        1450            1460            1470            1480
     *       *       *       *       *       *       *       *
TATCTGCAGA AGCTCTCCCA GCAGCTGAAG CAAGGCAGGA
  Y   L   Q   K   L   S   Q   Q   L   K   Q   G   R


        1490            1500            1510            1520
     *       *       *       *       *       *       *       *
GACGCCCACT CGTGGACCTC CACGTTGAAC TCATGGACAA
  R   R   P   L   V   D   L   H   V   E   L   M   D   K


        1530            1540            1550            1560
     *       *       *       *       *       *       *       *
AGTGTATGCG TGGAACAGTG GTGTTTCGTC TAAGGAGAAA
   V   Y   A   W   N   S   G   V   S   S   K   E   K


        1570            1580            1590            1600
     *       *       *       *       *       *       *       *
TACAGCCTCA GCCTGCAGCA CACTCTGAGG AAGAAACTCA
  Y   S   L   S   L   Q   H   T   L   R   K   K   L


        1610            1620            1630            1640
     *       *       *       *       *       *       *       *
TCCTGGCTCC TACGTGAGAA CCCCAGACCG TTGGTGTTCT
  I   L   A   P   T>


        1650            1660            1670            1680
     *       *       *       *       *       *       *       *
TGGTATATCA TCCAGGGTGG CGGCTTGGAG CAGAGCTTGG
```

Forts. Fig. 4 c

```
        1690           1700           1710           1720
    *         *     *         *     *         *     *         *
CGGTTACGGC  TGCTTCTGGC  TGCTTCTGGC  TCTGCCGTGA


        1730           1740           1750           1760
    *         *     *         *     *         *     *         *
GTCCTGGCCT  AGGGTTCTCC  TGTGCACAGG  CATGAGCCGT


        1770           1780           1790           1800
    *         *     *         *     *         *     *         *
AACCCTGTGC  CTGGGAAACG  TCTCACTCCC  GCCGCCGTGC


        1810           1820           1830           1840
    *         *     *         *     *         *     *         *
CTTTACCTCT  CTAAACTTCC  CTACTTACAT  TCCTTAGTCG


        1850           1860           1870           1880
    *         *     *         *     *         *     *         *
GATGTTTTGC  CAGAGTGTGG  AGAACAGTAA  GACATAAACC


        1890           1900           1910           1920
    *         *     *         *     *         *     *         *
TATTGTTTGT  TTGTTTTTTT  GGGGGGGAGG  TTATCTACCA


        1930           1940           1950           1960
    *         *     *         *     *         *     *         *
AGTTATACCA  AGTTATTGTA  TGGGTGTATA  GTGTATAGTG
```

Foris. Fig. 4e

```
        1970            1980            1990            2000
     *       *       *       *       *       *       *       *
   GTTCAAGATT  TGACACTGAA  TGTAACTTGA  GACTTACCTG


        2010            2020            2030            2040
     *       *       *       *       *       *       *       *
   AGTTTGTCAT  GCGACTGGGT  AAATTGTTTC  TATGGCACAT


        2050            2060            2070            2080
     *       *       *       *       *       *       *       *
   CTAATCATTT  AATAAGTAAT  TACCTCATTA  AGTACCCATT


        2090            2100            2110            2120
     *       *       *       *       *       *       *       *
   GCTTCAGGAC  TTTCACATTG  GCCACCAATT  TCTGTGACCC


        2130            2140            2150            2160
     *       *       *       *       *       *       *       *
   AGCTCCACAT  TTATATTCTC  TTTCTGCAAA  ACCAAATTTC


        2170            2180            2190            2200
     *       *       *       *       *       *       *       *
   ATTATGTCTG  TTTAATATCT  ACAGTCTAAT  GCTTTGTAAG


        2210            2220            2230            2240
     *       *       *       *       *       *       *       *
   ACATCTAGAT  AGAAAAATAG  TTACCCATGA  GCACAGAAGG
```

Fords. Fig. 4c

```
        2250          2260          2270          2280
    *         *     *         *     *         *     *         *
GCTGGCCTGA CCCTCACCAG CTGTGCAGTG GCTTCGGTGA


        2290          2300          2310          2320
    *         *     *         *     *         *     *         *
AGGAGAATGA GCCCTACTCC TTGAAGGTTG TAGTGCTTGG


        2330          2340          2350          2360
    *         *     *         *     *         *     *         *
GAGAGCAGTC TGTACCTTGC CTGGGCAGCA CAGTAGAGCC


        2370          2380          2390          2400
    *         *     *         *     *         *     *         *
AGCCCCAAGA ACACAACAGT GAGTGGGGGA GCTTGCCCTG


        2410          2420          2430          2440
    *         *     *         *     *         *     *         *
GTTGGCTCAG GATCAGGAAC AGGAGGGATG ACGGAATTCC


        2450
    *         *
TGCAGCCCGG GG
```

a

| Flag-E8 | - | - | + | + | + |
| FADD | - | + | - | + | + |
| myc-CD95 | + | + | + | + | - |

IP
α-Flag-E8    29–
α-myc                      →IgG
          19–              ◄CD95

          33–
          29–              ◄FADD
a-FADD

Cell
extracts    29–
α-Flag                     ◄E8
          19–

          33–
          29–              ◄FADD
α-FADD

          29–              ◄CD95
α-myc
          19–

b

| Flag-159L | - | + | + | + | + |
| FADD | - | - | + | - | + |
| myc-CD95 | - | - | - | + | + |

IP
α-Flag-159L  29–
α-myc                      →IgG
          19–              ◄CD95

          33–
          29–              ◄FADD
a-FADD                     →IgG

Cell
extracts    29–            ◄159L
α-Flag
          19–

          33–
          29–              ◄FADD
α-FADD

          29–              ◄CD95
α-myc
          19–

Fig. 5

Fig. 6

**a**

IEF → RCo/3          RE8/19

SDS-PAGE ↓

CD95

CAP

OH-       H+      OH-       H+

KD
- 67
- 46
- 30

**b**

RCo/3   RE8/19

- + - + KD

- FLICE
- p43
- p26
- p17
- p12
- p9

*Fig. 7*

EP 1 258 529 A2

Fig. 8

a                                                                    Fig. 9

b

c

Forts. Fig. 9

Fig. 10

*Fig. 11*

Fig 12

Fig. 13

Flag-FLIP_L    + - - - + - -
Flag-FLIP_S    - + - - - + -
Flag-FLIP_p    - - + - - - +
HA-FLICE       - - - + + + +

IP Ab:              α-Flag-FLIP

                47 —                    FLICE
Blot Ab: 32 —                          IgG
HA

Blot Ab: 47 —                          FLIPL
FLAG    32 —                           IgG
                                       FLIP_p
                                       FLIP_S

                Cell extracts

Blot Ab:
HA                                     FLICE
        47 —
        32 —

Fig.14a

*a*

Fig. 14b

Fig.14c

Fig. 15

**ORF71 aus Human Herpesvirus 8 (HHV8)**

```
MATYEVLCEV ARKLGTDDRE VVLFLLNVFI PQPTLAQLIG ALRALKEEGR LTFPLLAECL
FRAGRRDLLR DLLHLDPRFL ERHLAGTMSY FSPYQLTVLH VDGELCARDI RSLIFLSKDT
IGSRSTPQTF LHWVYCMENL DLLGPTDVDA LMSMLRSLSR VDLQRQVQTL MGLHLSGPSH
SQHYRHTP
```

EP 1 258 529 A2

Fig. 16

**ORF aus Bovine Herpesvirus 4 (BHV 4)**

```
MVTRDVLLAI ETHLNQNEKT FVMYFLLDPY IPKECKDFLP TLENLHSKRK IRYPILIELM
YILQRFDLLR SIFLLDHRFV KDQITSSHWK YISPYKQLIF SIGQNIDDED LISIKFISMN
YIGKSPSKIK NYLDWVRALE KVDMVGPDNL DLFETFFKQI HRMDIVKMIK NYRTRETLQI
TL
```

EP 1 258 529 A2

*Fig. 17*